(19) Europäisches Patentamt
European Patent Office
Office européen des brevets

(11) Publication number: 0 342 541 A2

(12) **EUROPEAN PATENT APPLICATION**

(21) Application number: 89108590.4

(22) Date of filing: 12.05.89

(51) Int. Cl.[4]: C07K 5/02 , C07K 5/04 , A61K 37/64 , C07C 103/49 , C07D 307/22 , C07C 125/065 , C07D 295/20 , C07D 263/38 , C07D 307/68

(30) Priority: 13.05.88 US 194678

(43) Date of publication of application: 23.11.89 Bulletin 89/47

(84) Designated Contracting States: ES GR

(71) Applicant: ABBOTT LABORATORIES
One Abbott Park Road
Abbott Park, IL 60064-3500(US)

(72) Inventor: Kempf, Dale J.
205 Chelsea Circle
Lake Villa Illinois 60046(US)
Inventor: Plattner, Jacob J.
1301 St. William Drive
Libertyville Illinois 60048(US)
Inventor: Norbeck, Daniel W.
307 Hazelwood
Lindenhurst Illinois 60046(US)
Inventor: Baker, William R.
1408 Juliet Lane
Libertyville Illinois 60048(US)
Inventor: Fung, Anthony K. L.
4239 Continental Drive
Waukegan Illinois 60087(US)
Inventor: Boyd, Steven A.
36039 N. Grand Oak Court Apt. 203
Gurnee Illinois 60031(US)
Inventor: Erickson, John W.
725 Seventh Street Summit
Barrington Illinois 60010(US)
Inventor: Crowley, Steven R.
1109 Marlowe Place
Vernon Hills Illinois 60061(US)

(74) Representative: Modiano, Guido et al
MODIANO, JOSIF, PISANTY & STAUB
Modiano & Associati Via Meravigli, 16
I-20123 Milan(IT)

(54) **Retroviral protease inhibitors.**

(57) A retroviral protease inhibiting compound of the formula:

EP 0 342 541 A2

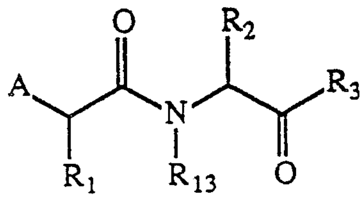

(I)

or a pharmaceutically acceptable salt, prodrug or ester thereof.

# RETROVIRAL PROTEASE INHIBITORS

## Technical Field

This application is a continuation-in-part of U.S. patent application Serial No. 194,678, filed May 13, 1988, which is a continuation-in-part of U.S. patent application Serial No. 132,356, filed December 18, 1987, which is a continuation-in-part of U.S. patent application Serial No. 943,567, filed December 31, 1986, which is a continuation-in-part of U.S. patent application Serial No. 895,009, filed August 7, 1986, now abandoned, which is a continuation-in-part of U.S. patent application Serial No. 818,734, filed January 16, 1986, now abandoned, which is a continuation-in-part of U.S. patent application Serial No. 693,951, filed January 23, 1985, now abandoned. U.S. patent application Serial No. 194,678, filed May 13, 1988, is also a continuation-in-part of U.S. patent application Serial No. 943,566, filed December 31, 1986, which is a continuation-in-part of U.S. patent application Serial No. 818,715, filed January 16, 1986, now abandoned, which is a continuation-in-part of U.S. patent application Serial No. 693,951, filed January 23, 1985, now abandoned. U.S. patent application Serial No. 194,678, filed May 13, 1988, is also a continuation-in-part of U.S. patent application Serial No. 946,883, filed January 9, 1987, which is a continuation-in-part of U.S. patent application Serial No. 850,802, filed April 11, 1986, now abandoned, which is a continuation-in-part of U.S patent application Serial No. 820,274, filed January 16, 1986, now abandoned. U.S. patent application Serial No. 194,678, filed May 13, 1988, is also a continuation-in-part of U.S. patent application Serial No. 946,881, filed January 9, 1987, which is a continuation-in-part of U.S. patent application Serial No. 735,491, file May 17, 1985, now U.S. Patent No. 4,645,759, issued February 24, 1987, which is a continuation-in-part of U.S. patent application Serial No. 623,807, filed June 22, 1984, now abandoned. U.S. patent application Serial No. 194,678, filed May 13, 1988, is also a continuation-in-part of U.S. patent application Serial No. 946,884, filed January 9, 1987, which is a continuation-in-part of U.S. patent application Serial No. 820,060, filed January 16, 1986. U.S. patent application Serial No. 194,678, filed May 13, 1988, is also a continuation-in-part of U.S. patent application Serial No. 097,553, filed September 16, 1987.

The present invention relates to novel compounds and a composition and method for inhibiting retroviral proteases and in particular for inhibiting human immunodeficiency virus (HIV) protease, a composition and method for treating a retroviral infection and in particular an HIV infection, processes for making such compounds and synthetic intermediates employed in these processes.

## Background Art

Retroviruses are those viruses which utilize a ribonucleic acid (RNA) intermediate and a RNA-dependent deoxyribonucleic acid (DNA) polymerase, reverse transcriptase, during their life cycle. Retroviruses include, but are not limited to, the RNA viruses of the Retroviridae family, and also the DNA viruses of the Hepadnavirus and Caulimovirus families. Retroviruses cause a variety of disease states in man, animals and plants. Some of the more important retroviruses from a pathological standpoint include human immunodeficiency viruses (HIV-1 and HIV-2), which cause acquired immune deficiency syndrome (AIDS) in man, hepatitis B virus, which causes hepatitis and hepatic carcinomas in man, human T-cell lymphotrophic viruses I, II, IV and V, which cause human acute cell leukemia, and bovine and feline leukemia viruses which cause leukemia in domestic animals.

Proteases are enzymes which cleave proteins at specific peptide bonds. Many biological functions are controlled or mediated by proteases and their complementary protease inhibitors. For example, the protease renin cleaves the peptide angiotensinogen to produce the peptide angiotensin I. Angiotensin I is further cleaved by the protease angiotensin converting enzyme (ACE) to form the hypotensive peptide angiotensin II. Inhibitors of renin and ACE are known to reduce high blood pressure in vivo. An inhibitor of a retroviral protease should provide a therapeutic agent for diseases caused by the retrovirus.

The genomes of retroviruses encode a protease that is responsible for the proteolytic processing of one or more polyprotein precursors such as the pol and gag gene products. See Wellink, Arch. Virol. 98 1 (1988). Retroviral proteases most commonly process the gag precursor into core proteins, and also process the pol precursor into reverse transciptase and retroviral protease. In addition, retroviral proteases are sequence specific. See Pearl, Nature 328 482 (1987).

The correct processing of the precursor polyproteins by the retroviral protease is necessary for the assembly of infectious virions. It has been shown that in vitro mutagenesis that produces protease-defective virus leads to the production of immature core forms which lack infectivity. See Crawford, J. Virol. 53 899

(1985); Katoh, et al., Virology 145 280 (1985). Therefore, retroviral protease inhibition provides an attractive target for antiviral therapy. See Mitsuya, Nature 325 775 (1987).

Current treatments for viral diseases usually involve administration of compounds that inhibit viral DNA synthesis. Current treatments for AIDS (Dagani, Chem. Eng. News, November 23, 1987 pp. 41-49) involve administration of compounds such as 2′,3′-dideoxycytidine, trisodium phosphonoformate, ammonium 21-tungsto-9-antimoniate, 1-beta-D-ribofuranosyl-1,2,4-triazole-3-carboxamide, 3′-azido-3′-deoxythymidine, and adriamycin that inhibit viral DNA synthesis; compounds such as AL-721 and polymannoacetate which may prevent HIV from penetrating the host cell; and compounds which treat the opportunistic infections caused by the immunosuppression resulting from HIV infection. None of the current AIDS treatments have proven to be totally effective in treating and/or reversing the disease. In addition, many of the compounds currently used to treat AIDS cause adverse side effects including low platelet count, renal toxicity and bone marrow cytopenia.

Moore, Biochem. Biophys. Res. Commun., 159 420 (1989), discloses peptidyl inhibitors of HIV protease.

U.S. Patent No. 4,652,552 discloses methyl ketone derivatives of tetrapeptides as inhibitors of viral proteases. U.S. Patent No. 4,644,055 discloses halomethylketone derivatives of peptides as inhibitors of viral proteases. European Patent Application No. EP0246630 discloses antiviral peptides for treating herpes. PCT Patent Application No. WO87/07836 discloses L-glutamic acid gamma-monohydroxamate as an antiviral agent.

The following patents/published patent applications disclose renin inhibiting peptides which in the present application are disclosed to be retroviral protease inhibitors:

European Patent Application No. EP0229667;
European Patent Application No. EP0230266;
PCT Patent Application No. WO87/04349;
U.S. Patent No. 4,725,584;
U.S. Patent No. 4,645,759;
European Patent Application No. EP0231919.

The ability to inhibit retroviral proteases, and in particular HIV protease, provides a method for blocking viral replication and therefore a treatment for retroviral diseases, and AIDS in particular, that has fewer or no side effects when compared to current treatments.

## Disclosure of the Invention

In accordance with the present invention, there are retroviral protease inhibiting compounds of the formula:

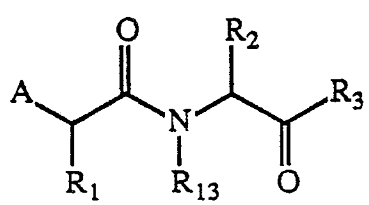

(I)

or a pharmaceutically acceptable salt, prodrug or ester thereof.

A is

(1) hydrogen,
(2) loweralkyl,
(3) functionalized alkyl,
(4) amino,
(5) functionalized amino,

(6) functionalized carbonyl,

(7) functionalized sulfonyl or

(8) $-OR_{14}$ or $-SR_{14}$ wherein $R_{14}$ is hydrogen, loweralkyl or aminoalkyl;

Functionalized alkyl groups include:

(1) (phenyl)alkyl,

(2) (naphthyl)alkyl,

(3) (substituted phenyl)alkyl wherein the phenyl ring is substituted with one, two or three substituents independently selected from loweralkyl, alkoxy, thioalkoxy, hydroxy, halogen, mercapto, nitro, amino, loweralkylamino, dialkylamino, carboxaldehyde, carboxy, alkoxycarbonyl and carboxamide; and

(4) (substituted naphthyl)alkyl wherein the naphthyl ring is substituted with one, two or three substituents independently selected from loweralkyl, alkoxy, thioalkoxy, hydroxy, halogen, mercapto, nitro, amino, loweralkylamino, dialkylamino, carboxaldehyde, carboxy, alkoxycarbonyl and carboxamide.

Functionalized amino includes:

$-NR_{15}R_{16}$ wherein $R_{15}$ and $R_{16}$ are independently selected from hydrogen, loweralkyl, aminoalkyl, alkylaminoalkyl, dialkylaminoalkyl, (N-protected)aminoalkyl, (N-protected)alkylaminoalkyl, cyanoalkyl, hydroxyalkyl, carboxyalkyl, alkoxycarbonylalkyl, (amino)carboxyalkyl, ((N-protected)amino)carboxyalkyl, (alkylamino)carboxyalkyl, ((N-protected)alkylamino)carboxyalkyl, (dialkylamino)carboxyalkyl, (amino)-alkoxycarbonylalkyl, ((N-protected)amino)alkoxycarbonylalkyl, (alkylamino)alkoxycarbonylalkyl, ((N-protected)alkylamino)alkoxycarbonylalkyl and (dialkylamino)alkoxycarbonylalkyl.

Functionalized carbonyl and functionalized sulfonyl include $R_{17}C(O)B$- and $R_{17}S(O)_2B$-, respectively, wherein B is NH, -N(alkyl)-, S, O, $CH_2$ or CHOH, and

$R_{17}$ is

(i) hydrogen,

(ii) loweralkyl,

(iii) cycloalkyl,

(iv) phenyl,

(v) substituted phenyl wherein the phenyl ring is substituted with one, two or three substituents independently selected from loweralkyl, alkoxy, thioalkoxy, hydroxy, halogen, mercapto, nitro, amino, loweralkylamino, carboxaldehyde, carboxy, alkoxycarbonyl and carboxamide,

(vi) naphthyl,

(vii) substituted naphthyl wherein the naphthyl ring is substituted with one, two or three substituents independently selected from loweralkyl, alkoxy, thioalkoxy, hydroxy, halogen, mercapto, nitro, amino, loweralkylamino, dialkylamino, carboxaldehyde, carboxy, alkoxycarbonyl and carboxamide,

(viii) (phenyl)alkyl,

(ix) (substituted phenyl)alkyl as defined above,

(x) (naphthyl)alkyl,

(xi) (substituted naphthyl)alkyl as defined above,

(xii) alkoxy,

(xiii) alkenyloxy,

(xiv) hydroxyalkoxy,

(xv) dihydroxyalkoxy,

(xvi) (phenyl)alkoxy,

(xvii) (substituted phenyl)alkoxy wherein substituted phenyl is as defined above,

(xviii) (naphthyl)alkoxy,

(xix) (substituted naphthyl)alkoxy wherein substituted naphthyl is as defined above,

(xx) (phenyl)alkoxyalkyl,

(xxi) (substituted phenyl)alkoxyalkyl wherein substituted phenyl is as defined above,

(xxii) (naphthyl)alkoxyalkyl,

(xxiii) (substituted naphthyl)alkoxyalkyl wherein substituted naphthyl is as defined above,

(xxiv) amino,

(xxv) alkylamino,

(xxvi) dialkylamino,

(xxvii) (hydroxyalkyl)(alkyl)amino,

(xxviii) (dihydroxyalkyl)(alkyl)amino,

(xxix) aminoalkyl,

(xxx) N-protected-aminoalkyl,

(xxxi) alkylaminoalkyl,
(xxxii) (N-protected)(alkyl)aminoalkyl,
(xxxiii) dialkylaminoalkyl,
(xxxiv) carboxyalkoxyalkyl,
(xxxv) (alkoxycarbonyl)alkoxyalkyl,
(xxxvi) carboxyalkyl,
(xxxvii) carboxyalkylamino,
(xxxviii) alkoxycarbonylalkyl,
(xxxix) alkoxycarbonylalkylamino,
(xl) (amino)carboxyalkyl,
(xli) (amino)carboxyalkylamino,
(xlii) ((N-protected)amino)carboxyalkyl,
(xliii) ((N-protected)amino)carboxyalkylamino,
(xliv) (alkylamino)carboxyalkyl,
(xlv) (alkylamino)carboxyalkylamino,
(xlvi) ((N-protected)alkylamino)carboxyalkyl,
(xlvii) ((N-protected)alkylamino)carboxyalkylamino,
(xlviii) (dialkylamino)carboxyalkyl,
(xlix) (dialkylamino)carboxyalkylamino,
(l)(amino)alkoxycarbonylalkyl,
(li) (amino)alkoxycarbonylalkylamino,
(lii)((N-protected)amino)alkoxycarbonylalkyl,
(liii) ((N-protected)amino)alkoxycarbonylalkylamino,
(liv) (alkylamino)alkoxycarbonylalkyl,
(lv) (alkylamino)alkoxycarbonylalkylamino,
(lvi) ((N-protected)alkylamino)alkoxycarbonylalkyl,
(lvii) ((N-protected)alkylamino)alkoxycarbonylalkylamino,
(lviii) (dialkylamino)alkoxycarbonylalkyl,
(lix) (dialkylamino)alkoxycarbonylalkylamino,
(lx) aminocycloalkyl,
(lxi) aminoalkylamino,
(lxii) dialkylaminoalkyl(alkyl)amino,
(lxiii) (phenyl)alkylamino,
(lxiv) (substituted phenyl)alkylamino wherein substituted phenyl is as defined above,
(lxv) (naphthyl)alkylamino,
(lxvi) (substituted naphthyl)alkylamino wherein substituted naphthyl is as defined above,
(lxvii) (phenyl)alkyl(alkyl)amino,
(lxviii) (substituted phenyl)alkyl(alkyl)amino wherein substituted phenyl is as defined above,
(lxix) (naphthyl)alkyl(alkyl)amino,
(lxx) (substituted naphthyl)alkyl(alkyl)amino wherein substituted naphthyl is as defined above,
(lxxi) alkoxyalkyl(alkyl)amino,
(lxxii) (polyalkoxy)alkyl(alkyl)amino,
(lxxiii) di(alkoxyalkyl)amino,
(lxxiv) di(hydroxyalkyl)amino,
(lxxv) di((polyalkoxy)alkyl)amino,
(lxxvi)polyalkoxy,
(lxxvii) (polyalkoxy)alkyl,
(lxxviii) substituted or unsubstituted heterocyclic wherein saturated heterocyclics are unsubstituted, monosubstituted or disubstituted with hydroxy, oxo, amino, alkylamino, dialkylamino, alkoxy, polyalkoxy, haloalkyl or loweralkyl; unsaturated heterocyclics are unsubstituted or monosubstituted with hydroxy, amino, alkylamino, dialkylamino, alkoxy, polyalkoxy, haloalkyl or loweralkyl,
(lxxix) (heterocyclic)alkyl,
(lxxx) (substituted heterocyclic)alkyl wherein substituted heterocyclic is as defined above,
(lxxxi) heterocyclic carbonyloxy;
(lxxxii) (substituted heterocyclic) carbonyloxy,
(lxxxxiii) heterocyclic carbonylamino,
(lxxxxiv) (substituted heterocyclic) carbonylamino,
(lxxxxv) (N-protected)amino, or

(lxxxxvi) (N-protected)(alkyl)amino.

$R_1$ is

(1) hydrogen,
(2) loweralkyl,
(3) hydroxyalkyl,
(4) cycloalkylalkyl,
(5) $-(CH_2)_yC(O)OH$ wherein y is 1 to 3,
(6) $-(CH_2)_yC(O)NH_2$ wherein y is 1 to 3,
(7) $-(CH_2)_yC(O)NHOH$ wherein y is 1 to 3,
(8) phenylalkyl,
(9) (substituted phenyl)alkyl wherein substituted phenyl is independently as defined above,
(10) naphthylalkyl or
(11) (substituted naphthyl)alkyl wherein substituted naphthyl is independently as defined above.

$R_2$ is

(1) loweralkyl,
(2) $-(CH_2)_zC(O)OH$ wherein z is 1 to 3,
(3) $-(CH_2)_zC(O)NH_2$ wherein z is 1 to 3,
(4) $-(CH_2)_zC(O)NHOH$ wherein z is 1 to 3,
(5) heterocyclic alkyl or
(6) (substituted heterocyclic)alkyl wherein substituted heterocyclic is independently as defined above.

$R_3$ is (substituted alkyl)amino.
(Substituted alkyl)amino includes:

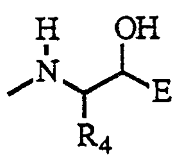 , 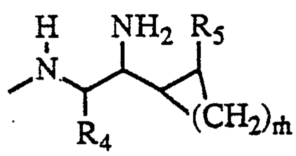 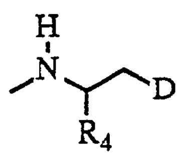 or 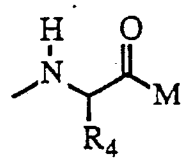

wherein m is 1 to 5;
$R_4$ is

(i) loweralkyl,
(ii) cycloalkyl,
(iii) cycloalkylalkyl,
(iv) (naphthyl)methyl,
(v) (substituted naphthyl)methyl wherein substituted naphthyl is independently as defined above,
(vi) benzyl or
(vii) substituted benzyl wherein the phenyl ring is substituted with a substituent selected from loweralkyl, alkoxy, thioalkoxy, hydroxy, halogen, mercapto, nitro, amino, loweralkylamino, dialkylamino, carboxaldehyde, carboxy, alkoxycarbonyl, and carboxamide;

$R_5$ is

(i) hydrogen,
(ii) loweralkyl,
(iii) $-C(O)R_6$ or
(iv) $-C(O)-R_{75}-R_6$ wherein $R_6$ is hydroxy, alkoxy or $-NR_{15}R_{16}$ wherein $R_{15}$ and $R_{16}$ are independently as defined above and $R_{75}$ is $-NHCH(R'')C(O)-$, $-NHCH(R'')C(O)NHCH(R''')C(O)-$, $-NHCH(R'')C(O)NHCH(R''')C(O)-R_{220}-$ or $-NHCH(R'')C(O)NHCH(R''')C(O)-R_{220}-R_{221}-$wherein $R''$ is hydrogen, loweralkyl, cycloalkylalkyl, (phenyl)alkyl, (substituted phenyl)alkyl wherein substituted phenyl is independently as defined above, (naphthyl)alkyl or (substituted naphthyl)alkyl wherein substituted naphthyl is independently as defined above, $R'''$ is hydrogen, loweralkyl, hydroxyalkyl, aminoalkyl, carboxyalkyl, carboxamidoalkyl, N-hydroxycarboxamidoalkyl or guanidinoalkyl, and R220 and R221 are amino acyl residues independently selected from the twenty naturally occuring alpha amino acids;

M is

(i) hydrogen,
(ii) loweralkyl or
(iii) haloalkyl;

E is

(i) $-CH(R_7)(R_8)$ wherein $R_7$ is hydrogen, amino or hydroxy and $R_8$ is loweralkyl, hydroxy, hydroxyalkyl, alkoxy, alkoxyalkyl, azido, azidoalkyl, amino, aminoalkyl, alkylamino alkylaminoalkyl, dialkylamino, dialkylaminoalkyl, (N-protected)amino, (N-protected)aminoalkyl, (N-protected)(alkyl)amino, (N-protected)(alkyl)aminoalkyl, thioalkoxy, thioalkoxyalkyl, alkylsulfonyl, alkylsulfonylalkyl, thioaryloxy, thioaryloxyalkyl, phenyl, substituted phenyl independently as defined above, naphthyl, substituted naphthyl independently, as defined above, heterocyclic, substituted heterocyclic independently as defined above, phenylalkyl, (substituted phenyl)alkyl independently as defined above, naphthylalkyl, (substituted naphthyl)alkyl independently as defined above, heterocyclic alkyl, (substituted heterocyclic) alkyl independently as defined above, (phenyl)sulfonyl, (substituted phenyl)sulfonyl wherein substituted phenyl is independently as defined above, (naphthyl)sulfonyl, (substituted naphthyl)sulfonyl wherein substituted naphthyl is independently as defined above, (phenyl)sulfonylalkyl, (substituted phenyl)sulfonylalkyl wherein substituted phenyl is independently as defined above, (naphthyl)sulfonylalkyl, (substituted naphthyl)sulfonylalkyl wherein substituted naphthyl is independently as defined above, hydrogen, $-C(=CH_2)C(O)NHR_9$, $-C(OH)(R_{10})C(O)NHR_9$ or $-CH(R_{10})C(O)NHR_9$ wherein $R_9$ is loweralkyl, hydroxyalkyl, alkoxyalkyl, aminoalkyl, alkylaminoalkyl or dialkylaminoalkyl and $R_{10}$ is hydrogen, loweralkyl, hydroxyalkyl, haloalkyl or azidoalkyl, such that when $R_7$ is hydroxy or amino then $R_8$ is not hydroxy, alkoxy, azido, amino, alkylamino, dialkylamino, (N-protected)-amino, (N-protected)(alkyl)amino, thioalkoxy, alkylsulfonyl, (phenyl)sulfonyl, (substituted phenyl)sulfonyl wherein substituted phenyl is independently as defined above, (naphthyl)sulfonyl or (substituted naphthyl)-sulfonyl wherein substituted naphthyl is independently as defined above;

or E is

(ii) $-R_{200}-R_{201}-R_{202}-R_{203}$ wherein $R_{200}$ is an alkylene group, $R_{201}$ is S, S(O), $S(O)_2$, O, NH or -N(alkyl)-, $R_{202}$ is an alkylene group and $R_{203}$ is phenyl, substituted phenyl independently as defined above, naphthyl or substituted naphthyl independently as defined above;

or E is

(iii)

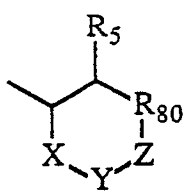 , 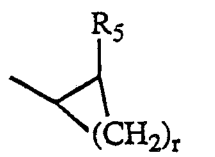 , 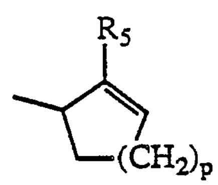 ,

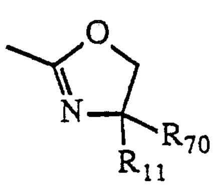 , 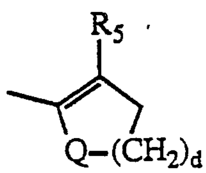 , 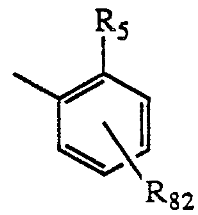 ,

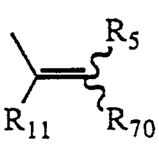 , 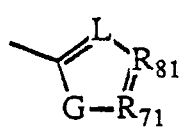 , 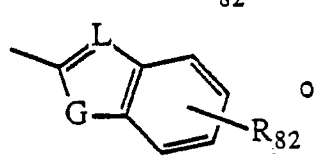 or

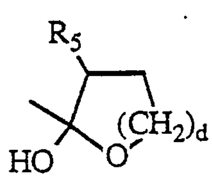

wherein $R_5$ is independently as defined above,

G is O, S, -N($R_{11}$)- or -C($R_{11}$)($R_{70}$)-wherein $R_{11}$ is hydrogen or loweralkyl and $R_{70}$ is hydrogen or loweralkyl,
L is N or -C($R_5$)- wherein $R_5$ is independently as defined above,
$R_{81}$ is N or -C($R_{11}$)- wherein $R_{11}$ is independently as defined above,
$R_{71}$ is N or -C($R_{11}$)- wherein $R_{11}$ is independently as defined above,
$R_{80}$ is absent, -C($R_{11}$)($R_{70}$)- or -N($R_{11}$)-wherein $R_{11}$ and $R_{70}$ are independently as defined above,
$R_{82}$ is absent, or one or two substituents independently selected from loweralkyl, alkoxy, thioalkoxy, hydroxy, halogen, mercapto, nitro, amino, loweralkylamino, dialkylamino, carboxaldehyde, carboxy, alkoxycarbonyl and carboxamide,
Q is O or -C($R_{11}$)($R_{70}$)- wherein $R_{11}$ and $R_{70}$ are independently as defined above,
X is O, S, -N($R_{11}$)-, -C($R_{11}$)($R_{70}$)-, -C(O)- or -C(=$CH_2$)- wherein $R_{11}$ and $R_{70}$ are independently as defined above,
Y is -C(O)-, -C(S)-, -C(=$CH_2$)- or -C($R_{11}$)($R_{70}$)- wherein $R_{11}$ and $R_{70}$ are independently as defined above,
Z is O, S, -C(=$CH_2$)-, -C($R_{11}$)($R_{70}$)-wherein $R_{11}$ and $R_{70}$ are independently as defined above, or -N($R_{12}$)- wherein $R_{12}$ is hydrogen, loweralkyl, alkoxy, hydroxyalkyl or alkylamino,
d is 1 to 3, p is 0 to 3, and
r is 1, 2 or 5;
with the proviso that when $R_{80}$ is -N($R_{11}$)- then Z is O, S, -N($R_{12}$)- wherein $R_{12}$ is independently as defined above or -C($R_{11}$)($R_{70}$)- and with the proviso that when Y is -C(=$CH_2$)- then X is -C(O)-, -C(=$CH_2$)- or -C($R_{11}$)($R_{70}$)- and Z is -C(=$CH_2$)- or -C($R_{11}$)($R_{70}$)-; and

D is

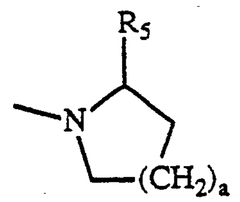

wherein a is 0 to 3 and $R_5$ is independently as defined above.

$R_{13}$ is hydrogen or loweralkyl.

The compounds of the invention wherein $R_1$ is loweralkyl, benzyl, 4-methoxybenzyl, halobenzyl, (1-naphthyl)methyl or (2-naphthyl)methyl and $R_2$ is loweralkyl, heterocyclic alkyl or (substituted heterocyclic) alkyl and A is as defined above and $R_3$ is

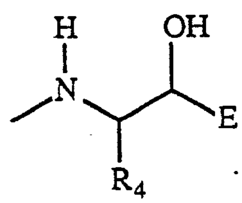

wherein $R_4$ is loweralkyl, cycloalkylmethyl or benzyl and E is -CH($R_7$)($R_8$) wherein $R_7$ is hydroxy or amino and $R_8$ is loweralkyl, phenylalkyl, (substiututed phenyl)alkyl, naphthylalkyl or (substituted naphthyl)alkyl have been disclosed as renin inhibitors in copending U.S. patent applications, USSN 217,106, filed July 11, 1988 and USSN 132,356, filed December 18, 1987, both of which are hereby incorporated by reference.

The compounds of the invention wherein $R_1$ is loweralkyl, phenylalkyl, (substituted phenyl)alkyl, naphthylalkyl and (substituted naphthyl)alkyl and $R_2$ is loweralkyl, heterocyclic alkyl or (substituted heterocyclic) alkyl and A is hydrogen, loweralkyl, (phenyl)alkyl, (substituted phenyl)alkyl wherein substituted phenyl is as defined above, (naphthyl)alkyl, (substituted naphthyl)alkyl wherein substituted naphthyl is as defined above, -O$R_{14}$ wherein $R_{14}$ is hydrogen or loweralkyl, -N$R_{15}R_{16}$ wherein $R_{15}$ and $R_{16}$ are independently selected from hydrogen and loweralkyl or $R_{17}$C(O)B- wherein B is NH, O or $CH_2$ and $R_{17}$ is loweralkyl, alkoxy, (phenyl)alkoxy, (substituted phenyl)alkoxy wherein substituted phenyl is as defined above, (naphthyl)alkoxy, (substituted naphthyl)alkoxy wherein substituted naphthyl is as defined above, (phenyl)alkoxyalkyl, (substituted phenyl)alkoxyalkyl wherein substituted phenyl is as defined above,

(naphthyl)alkoxyalkyl, (substituted naphthyl)alkoxyalkyl wherein substituted naphthyl is as defined above, amino, alkylamino, dialkylamino, (dihydroxyalkyl)(alkyl)amino, aminoalkyl, heterocyclic, substituted heterocyclic, (heterocyclic)alkyl or (substituted heterocyclic)alkyl; and $R_3$ is

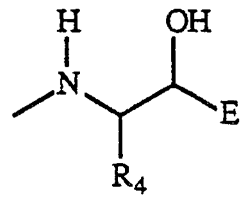

wherein $R_4$ is loweralkyl, cycloalkyl, cycloalkylalkyl, naphthylmethyl, benzyl or substituted benzyl and E is -$CH_2R_8$ wherein $R_8$ is loweralkyl, alkoxy, alkylamino, (N-protected)amino, thioalkoxy, alkylsulfonyl, thioaryloxy or (phenyl)sulfonyl, (substituted phenyl)sulfonyl wherein substituted phenyl is as defined above, (naphthyl)sulfonyl, or (substituted naphthyl)sulfonyl wherein substituted naphthyl is as defined above have been disclosed as renin inhibitors in copending U.S. patent application USSN 946,881, filed January 9, 1987, which is hereby incorporated by reference.

The compounds of the invention wherein $R_1$ is loweralkyl, benzyl, 4-methoxybenzyl, halobenzyl, (1-naphthyl)methyl or (2-naphthyl)methyl and $R_2$ is loweralkyl, heterocyclic alkyl or (substituted heterocyclic) alkyl and A is hydrogen, loweralkyl, (phenyl)alkyl, (substituted phenyl)alkyl wherein substituted phenyl is as defined above, (naphthyl)alkyl, (substituted naphthyl)alkyl wherein substituted naphthyl is as defined above, -$OR_{14}$ or -$SR_{14}$ wherein $R_{14}$ is hydrogen, loweralkyl or aminoalkyl, -$NR_{15}R_{16}$ wherein $R_{15}$ and $R_{16}$ are independently selected from hydrogen, loweralkyl, aminoalkyl, cyanoalkyl or hydroxyalkyl, $R_{17}C(O)B$- or $R_{17}S(O)_2B$- wherein B is NH, -N(alkyl)-, S, O, $CH_2$ or CHOH and $R_{17}$ is loweralkyl, cycloalkyl, phenyl, substituted phenyl as defined above, naphthyl, substituted naphthyl as defined above, (phenyl)alkyl, (substituted phenyl)alkyl wherein substituted phenyl is as defined above, (naphthyl)alkyl, (substituted naphthyl)alkyl wherein substituted naphthyl is as defined above, alkoxy, alkenyloxy, hydroxyalkoxy, dihydroxyalkoxy, (phenyl)alkoxy, (substituted phenyl)alkoxy wherein substituted phenyl is as defined above, (naphthyl)alkoxy, (substituted naphthyl)alkoxy wherein substituted naphthyl is as defined above, (phenyl)-alkoxyalkyl, (substituted phenyl)alkoxyalkyl wherein substituted phenyl is as defined above, (naphthyl)-alkoxyalkyl, (substituted naphthyl)alkoxyalkyl wherein substituted naphthyl is as defined above, amino, alkylamino, dialkylamino, (hydroxyalkyl)(alkyl)amino, (dihydroxyalkyl)(alkyl)amino, aminoalkyl, alkylaminoalkyl, (N-protected)(alkyl)aminoalkyl, dialkylaminoalkyl, carboxyalkyl, alkoxycarbonylalkyl, heterocyclic, substituted heterocyclic, (heterocyclic)alkyl or (substituted heterocyclic)alkyl; and $R_3$ is

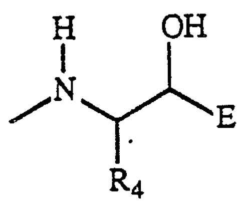

wherein $R_4$ is loweralkyl, cycloalkylmethyl or benzyl and E is -$CH(OH)R_8$ wherein $R_8$ is hydroxyalkyl, alkoxyalkyl, azidoalkyl, aminoalkyl, (N-protected)aminoalkyl, alkylaminoalkyl, (N-protected)(alkyl)aminoalkyl, thioalkoxyalkyl, alkylsulfonylalkyl, thioaryloxyalkyl or (phenyl)sulfonylalkyl, (substituted phenyl)sulfonylalkyl wherein substituted phenyl is as defined above, (naphthyl)sulfonylalkyl, or (substituted naphthyl)sulfonylalkyl wherein substituted naphthyl is as defined above; or E is -$CHR_7R_8$ wherein $R_7$ is hydrogen or hydroxy and $R_8$ is -$C(=CH_2)C(O)NHR_9$, -$C(OH)R_{10}C(O)NHR_9$ or -$CHR_{10}C(O)NHR_9$ wherein $R_9$ is loweralkyl, hydroxyalkyl, alkoxyalkyl, loweralkyl, alkylaminoalkyl or dialkylaminoalkyl and $R_{10}$ is hydrogen, loweralkyl, hydroxyalkyl, haloalkyl or azidoalkyl have been disclosed as renin inhibitors in copending U.S. patent applications USSN 191,714, filed May 9, 1988, USSN 946,883, filed January 9, 1987, and USSN 946,884, filed January 9, 1987, all of which are hereby incorporated by reference.

The compounds of the invention wherein $R_1$ is loweralkyl, benzyl, 4-methoxybenzyl, halobenzyl, 4-hydroxybenzyl, (1-naphthyl)methyl or (2-naphthyl)methyl and $R_2$ is loweralkyl, heterocyclic alkyl or (substituted heterocyclic) alkyl and A is hydrogen, loweralkyl, (phenyl)alkyl, (substituted phenyl)alkyl as defined above, (naphthyl)alkyl, (substituted naphthyl)alkyl as defined above, -$OR_{14}$ or -$SR_{14}$ wherein $R_{14}$ is

hydrogen, loweralkyl or aminoalkyl, $-NR_{15}R_{16}$ wherein $R_{15}$ and $R_{16}$ are independently selected from hydrogen, loweralkyl, aminoalkyl, cyanoalkyl and hydroxyalkyl, $R_{17}C(O)B-$ or $R_{17}S(O)_2B-$ wherein B is NH, alkylamino, S, O, $CH_2$ or CHOH and $R_{17}$ is hydrogen, loweralkyl, cycloalkyl, phenyl, substituted phenyl as defined above, naphthyl, substituted naphthyl as defined above, alkoxy, alkenyloxy, hydroxyalkoxy, dihydroxyalkoxy, (phenyl)alkoxy, (substituted phenyl)alkoxy wherein substituted phenyl is as defined above, (naphthyl)alkoxy, (substituted naphthyl)alkoxy wherein substituted naphthyl is as defined above, (phenyl)-alkoxyalkyl, (substituted phenyl)alkoxyalkyl wherein substituted phenyl is as defined above, (naphthyl)-alkoxyalkyl, (substituted naphthyl)alkoxyalkyl wherein substituted naphthyl is as defined above, amino, alkylamino, dialkylamino, (hydroxyalkyl)(alkyl)amino, (dihydroxyalkyl)(alkyl)amino, aminoalkyl, alkylaminoalkyl, (N-protected)(alkyl)aminoalkyl, dialkylaminoalkyl, carboxyalkyl, alkoxycarbonylalkyl, aminocycloalkyl, aminoalkylamino, dialkylaminoalkylamino, (phenyl)alkylamino, (substituted phenyl)alkylamino wherein substituted phenyl is as defined above, (naphthyl)alkylamino, (substituted naphthyl)alkylamino wherein substituted naphthyl is as defined above, (phenyl)alkyl(alkyl)amino, (substituted phenyl)alkyl(alkyl)amino wherein substituted phenyl is as defined above, (naphthyl)alkyl(alkyl)amino, (substituted naphthyl)alkyl(alkyl)amino wherein substituted naphthyl is as defined above, alkoxyalkyl(alkyl)amino, (polyalkoxy)alkyl(alkyl)amino, di-(alkoxyalkyl)amino, di-(hydroxyalkyl)amino, heterocyclic, substituted heterocyclic, (heterocyclic)alkyl or (substituted heterocyclic)alkyl and $R_3$ is

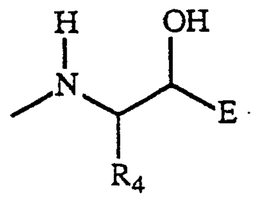

wherein E is

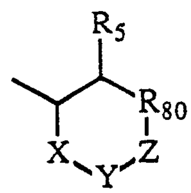

wherein $R_5$ is hydrogen, $R_{80}$ is absent, $-CH_2-$ or $-N(R_{11})-$wherein $R_{11}$ is as defined above, X is O, S or NH, Y is $-CH_2-$, $-C(=O)-$ or $-C(=S)-$, and Z is O, S, $-N(R_{12})-$ wherein $R_{12}$ is as defined above or $-C(R_{11})(R_{70})-$ wherein $R_{11}$ and $R_{70}$ are as defined above, with the proviso that when $R_{80}$ is -N(R11)- then $R_{12}$ is loweralkyl or hydroxyalkyl have been disclosed as renin inhibitors in copending U.S. patent application USSN 231,869, filed August 16, 1988, which is hereby incorporated by reference.

The compounds of the invention wherein

(1) E is

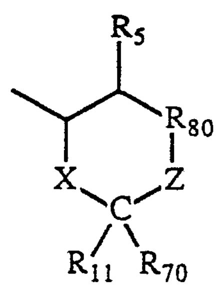

wherein $R_5$ is hydrogen or loweralkyl, $R_{11}$ is hydrogen or loweralkyl and $R_{70}$ is hydrogen or loweralkyl; and

(i) wherein X is $-N(R_{11})-$, Z is O, S, $-C(R_{11})(R_{70})-$ or $-N(R_{12})-$ wherein $R_{12}$ is hydrogen or loweralkyl and $R_{80}$ is absent, $-C(R_{11})(R_{70})-$ or $-N(R_{11})-$;

or

(ii) wherein Z is $-N(R_{12})-$ wherein $R_{12}$ is hydrogen or loweralkyl, X is O, S, $-C(R_{11})(R_{70})-$ or $-N(R_{11})-$, and $R_{80}$ is absent, $-C(R_{11})(R_{70})-$ or $-N(R_{11})-$;

or

(iii) wherein $R_{80}$ is $-N(R_{11})-$, X is O, S, $-C(R_{11})(R_{70})-$ or $-N(R_{11})-$ and Z is O, S, $-C(R_{11})(R_{70})-$ or $-N(R_{12})-$ wherein $R_{12}$ is hydrogen or loweralkyl;

or

(2) E is

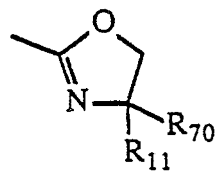

wherein $R_{11}$ is hydrogen or loweralkyl and $R_{70}$ is hydrogen or loweralkyl;

or

(3) E is

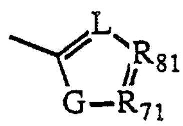

wherein $R_5$ is hydrogen or loweralkyl and $R_{11}$ is hydrogen or loweralkyl;

and

(i) wherein L is N;

or

(ii) wherein G is $-N(R_{11})-$ and L is -C(R5)-;

or

(iii) wherein $R_{71}$ is N and L is -C(R5);

or

(iv) wherein $R_{81}$ is N and L is $-C(R_5)$;

or

(4) E is

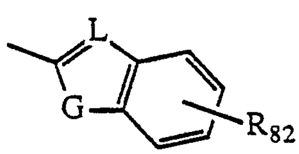

(i) wherein L is N and $R_{82}$ is hydrogen or loweralkyl;

or

(ii) wherein L is $-C(R_5)-$ wherein $R_5$ is hydrogen or loweralkyl, G is $-N(R_{11})-$ wherein $R_{11}$ is hydrogen or loweralkyl and $R_{82}$ is hydrogen or loweralkyl have been disclosed as renin inhibitors in European Patent Application No. EP0231919, published August 12, 1987.

The chiral centers of the compounds of the invention may have either the "R" or "S" configuration. The

terms "S" and "R" configuration are as defined by the IUPAC 1974 Recommendations for Section E, Fundamental Stereochemistry, Pure Appl. Chem. (1976) 45, 13 - 30.

The term "N-protecting group" or "N-protected" as used herein refers to those groups intended to protect nitrogen atoms against undesirable reactions during synthetic procedures or to prevent the attack of exopeptidases on the final compounds or to increase the solubility of the final compounds and includes but is not limited to acyl, acetyl, pivaloyl, t-butylacetyl, t-butyloxycarbonyl (Boc), benzyloxycarbonyl (Cbz) or benzoyl groups or an L- or D-aminoacyl residue, which may itself be N-protected similarly.

The term "loweralkyl" as used herein refers to straight or branched chain alkyl radicals containing from 1 to 6 carbon atoms including but not limited to methyl, ethyl, n-propyl, iso-propyl, n-butyl, iso-butyl, sec-butyl, n-pentyl, 1-methylbutyl, 2,2-dimethylbutyl, 2-methylpentyl, 2,2-dimethylpropyl, n-hexyl and the like.

The term "loweralkenyl" as used herein refers to a loweralkyl radical which contains at least one carbon-carbon double bond.

The term "alkylene" as used herein refers to straight or branched chain spacer radicals containing 2 to 6 carbon atoms such as $-CH_2-$, $-CH(CH_3)-$, $-CH_2CH_2-$, $-CH_2CH(CH_3)-$ and the like.

The term "substituted phenyl" as used herein refers to a phenyl ring substituted with one, two or three substituents independently selected from loweralkyl, alkoxy, thioalkoxy, hydroxy, halogen, mercapto, nitro, amino, loweralkylamino, dialkylamino, carboxaldehyde, carboxy, alkoxycarbonyl and carboxamide.

The term "substituted naphthyl" as used herein refers to a naphthyl ring substituted with one, two or three substituents independently selected from loweralkyl, alkoxy, thioalkoxy, hydroxy, halogen, mercapto, nitro, amino, loweralkylamino, dialkylamino, carboxaldehyde, carboxy, alkoxycarbonyl and carboxamide.

The term "(phenyl)alkyl" as used herein refers to a phenyl ring appended to a loweralkyl radical, including but not limited to benzyl and the like.

The term "(substituted phenyl)alkyl" as used herein refers to a substituted phenyl ring as defined above appended to a loweralkyl radical, including but not limited to 4-hydroxybenzyl, 4-methoxybenzyl and 4-chlorobenzyl.

The term "(naphthyl)alkyl" as used herein refers to a naphthyl ring appended to a loweralkyl radical, including but not limited to 1-naphthylmethyl and 2-naphthylmethyl.

The term "(substituted naphthyl)alkyl" as used herein refers to a substituted naphthyl ring as defined above appended to a loweralkyl radical.

The term "aminoalkyl" as used herein refers to $-NH_2$ appended to a loweralkyl radical.

The term "cyanoalkyl" as used herein refers to -CN appended to a loweralkyl radical.

The term "hydroxyalkyl" as used herein refers to -OH appended to a loweralkyl radical.

The term "alkylamino" as used herein refers to a loweralkyl radical appended to an NH radical.

The term "cycloalkyl" as used herein refers to an aliphatic ring having 3 to 7 carbon atoms.

The term "cycloalkylalkyl" as used herein refers to a cycloalkyl group appended to a loweralkyl radical, including but not limited to cyclohexylmethyl.

The terms "alkoxy" and "thioalkoxy" as used herein refer to $R_{18}O-$ and $R_{18}S-$, respectively, wherein $R_{18}$ is a loweralkyl group.

The term "alkenyloxy" as used herein refers to $R_{19}O$-wherein $R_{19}$ is a loweralkenyl group.

The term "hydroxyalkoxy" as used herein refers to -OH appended to an alkoxy radical.

The term "dihydroxyalkoxy" as used herein refers to an alkoxy radical which is disubstituted with -OH groups.

The term "(phenyl)alkoxy" as used herein refers $R_{90}O$-wherein $R_{90}$ is a (phenyl)alkyl group as defined above.

The term "(substituted phenyl)alkoxy" as used herein refers to $R_{91}O-$ wherein $R_{91}$ is a (substituted phenyl)alkyl group as defined above.

The term "(naphthyl)alkoxy" as used herein refers to $R_{92}O-$ wherein $R_{92}$ is a (naphthyl)alkyl group as defined above.

The term "(substituted naphthyl)alkoxy" as used herein refers to $R_{93}O-$ wherein $R_{93}$ is a (substituted naphthyl)alkyl group as defined above.

The term "(phenyl)alkoxyalkyl" as used herein refers to a (phenyl)alkoxy group as defined above appended to a loweralkyl radical.

The term "(substituted phenyl)alkoxyalkyl" as used herein refers to a (substituted phenyl)alkoxy group as defined above appended to a loweralkyl radical.

The term "(naphthyl)alkoxyalkyl" as used herein refers to a (naphthyl)alkoxy group as defined above appended to a loweralkyl radical.

The term "(substituted naphthyl)alkoxyalkyl" as used herein refers to a (substituted naphthyl)alkoxy group as defined above appended to a loweralkyl radical.

The term "(thioalkoxy)alkyl" as used herein refers to thioalkoxy appended to a loweralkyl radical.

The term "dialkylamino" as used herein refers to $-NR_{20}R_{21}$ wherein $R_{20}$ and $R_{21}$ are independently selected from loweralkyl groups.

The term "((alkoxy)alkoxy)alkyl" refers to an alkoxy group appended to an alkoxy group which is appended to a loweralkyl radical.

The term "(hydroxyalkyl)(alkyl)amino" as used herein refers to $-NR_{22}R_{23}$ wherein $R_{22}$ is hydroxyalkyl and $R_{23}$ is loweralkyl.

The term "N-protected aminoalkyl" as used herein refers to $-NHR_{24}$ appended to a loweralkyl group, wherein $R_{24}$ is an N-protecting group.

The term "alkylaminoalkyl" as used herein refers to $NHR_{25}$ appended to a loweralkyl radical, wherein $R_{25}$ is a loweralkyl group.

The term "(N-protected)(alkyl)aminoalkyl" as used herein refers to $-NR_{24}R_{25}$, which is appended to a loweralkyl radical, wherein $R_{24}$ and $R_{25}$ are as defined above.

The term "dialkylaminoalkyl" as used herein refers to $-NR_{26}R_{27}$ which is appended to a loweralkyl radical wherein $R_{26}$ and $R_{27}$ are independently selected from loweralkyl.

The term "carboxamidoalkyl" as used herein refers to a $-C(O)NH_2$ group appended to a loweralkyl radical.

The term "N-hydroxycarboxamidoalkyl" as used herein refers to a -C(O)NHOH group appended to a loweralkyl radical.

The term "guanidinoalkyl" as used herein refers to a $-NHC(=NH)NH_2$ group appended to a loweralkyl radical.

The term "azidoalkyl" as used herein refers to a $-N_3$ group appended to a loweralkyl radical.

The term "carboxyalkyl" as used herein refers to a carboxylic acid group (-COOH) appended to a loweralkyl radical.

The term "alkoxycarbonylalkyl" as used herein refers to $R_{28}C(O)R_{29}$- wherein $R_{28}$ is an alkoxy group and $R_{29}$ is a loweralkyl radical.

The term "carboxyalkoxyalkyl" as used herein refers to a carboxylic acid group (-COOH) appended to an alkoxy group which is appended to a loweralkyl radical.

The term "alkoxycarbonylalkoxyalkyl" as used herein refers to an alkoxycarbonyl group ($R_{30}C(O)$- wherein $R_{30}$ is an alkoxy group) appended to an alkoxy group which is appended to a loweralkyl radical.

The term "(amino)carboxyalkyl" as used herein refers to a loweralkyl radical to which is appended a carboxylic acid group (-COOH) and an amino group ($-NH_2$).

The term "((N-protected)amino)carboxyalkyl" as used herein refers to a loweralkyl radical to which is appended a carboxylic acid group (-COOH) and $-NHR_{31}$ wherein $R_{31}$ is an N-protecting group.

The term "(alkylamino)carboxyalkyl" as used herein refers to a loweralkyl radical to which is appended a carboxylic acid group (-COOH) and an alkylamino group.

The term "((N-protected)alkylamino)carboxyalkyl" as used herein refers to a loweralkyl radical to which is appended a carboxylic acid group (-COOH) and an $-NR_{31}R_{32}$ wherein $R_{31}$ is as defined above and $R_{32}$ is a loweralkyl group.

The term "(dialkylamino)carboxyalkyl" as used herein refers to a loweralkyl radical to which is appended a carboxylic acid group (-COOH) and $-NR_{32}R_{32}$ wherein $R_{32}$ is as defined above.

The term "(amino)alkoxycarbonylalkyl" as used herein refers to a loweralkyl radical to which is appended an alkoxycarbonyl group as defined above and an amino group ($-NH_2$).

The term "((N-protected)amino)alkoxycarbonylalkyl" as used herein refers to a loweralkyl radical to which is appended an alkoxycarbonyl group as defined above and $-NHR_{31}$ wherein $R_{31}$ is as defined above.

The term "(alkylamino)alkoxycarbonylalkyl" as used herein refers to a loweralkyl radical to which is appended an alkoxycarbonyl group as defined above and an alkylamino group as defined above.

The term "((N-protected)alkylamino)alkoxycarbonylalkyl" as used herein refers to a loweralkyl radical to which is appended an alkoxycarbonyl group as defined above and $-NR_{31}R_{32}$ wherein $R_{31}$ and $R_{32}$ are as defined above.

The term "(dialkylamino)alkoxycarbonylalkyl" as used herein refers to a loweralkyl radical to which is appended an alkoxycarbonyl group as defined above and $-NR_{32}R_{32}$ wherein $R_{32}$ is as defined above.

The term "carboxyalkylamino" as used herein refers to $-NHR_{33}$ wherein $R_{33}$ is a carboxyalkyl group.

The term "alkoxycarbonylalkylamino" as used herein refers to $-NHR_{34}$ wherein $R_{34}$ is an alkoxycarbonylakyl group.

The term "(amino)carboxyalkylamino" as used herein refers to $-NHR_{35}$ wherein $R_{35}$ is an (amino)-carboxyalkyl group.

The term "((N-protected)amino)carboxyalkylamino" as used herein refers to $-NHR_{36}$ wherein $R_{36}$ is an

[(N-protected)amino]carboxyalkyl group.

The term"(alkylamino)carboxyalkylamino" as used herein refers to $-NHR_{37}$ wherein $R_{37}$ is an (alkylamino)carboxyalkyl group.

The term "((N-protected)alkylamino)carboxyalkylamino" as used herein refers to $-NHR_{38}$ wherein $R_{38}$ is an ((N-protected)alkylamino)carboxyalkyl group.

The term "(dialkylamino)carboxyalkylamino" as used herein refers to $-NHR_{39}$ wherein $R_{39}$ is a (dialkylamino)carboxyalkyl group.

The term"(amino)alkoxycarbonylalkylamino" as used herein refers to $-NHR_{40}$ wherein $R_{40}$ is an (amino)alkoxycarbonylalkyl group.

The term "((N-protected)amino)alkoxycarbonylalkylamino" as used herein refers to $-NHR_{41}$ wherein $R_{41}$ is an ((N-protected)amino)alkoxycarbonylalkyl group.

The term "(alkylamino)alkoxycarbonylalkylamino" as used herein refers to $-NHR_{42}$ wherein $R_{42}$ is an (alkylamino)alkoxycarbonylalkyl group.

The term "((N-protected)alkylamino)alkoxycarbonylalkylamino" as used herein refers to $-NHR_{43}$ wherein $R_{43}$ is an ((N-protected)alkylamino)alkoxycarbonylalkyl group.

The term "(dialkylamino)alkoxycarbonylalkylamino" as used herein refers to $-NHR_{44}$ wherein $R_{44}$ is a (dialkylamino)alkoxycarbonylalkyl group.

The term "aminocycloalkyl" as used herein refers to an $NH_2$ appended to a cycloalkyl radical.

The term "((alkoxy)alkoxy)alkyl" as used herein refers to an alkoxy group appended to an alkoxy group which is appended to a loweralkyl radical.

The term "polyalkoxyalkyl" as used herein refers to a polyalkoxy residue appended to a loweralkyl radical.

The term "polyalkoxy" as used herein refers to $-OR_{45}$ wherein $R_{45}$ is a straight or branched chain containing 1-5, $C_n$-O-$C_{n'}$ linkages wherein n and n′ are independently selected from 1 to 3, including but not limited to methoxyethoxymethoxy, methoxymethoxy and the like.

The term "(phenyl)alkylamino" as used herein refers to $R_{100}NH$- wherein $R_{100}$ is a (phenyl)alkyl group as defined above.

The term "(substituted phenyl)alkylamino" as used herein refers to $R_{101}NH$- wherein $R_{101}$ is a (substituted phenyl)alkyl group as defined above.

The term "(naphthyl)alkylamino" as used herein refers to $R_{102}NH$- wherein $R_{102}$ is a (naphthyl)alkyl group as defined above.

The term "(substituted naphthyl)alkylamino" as used herein refers to $R_{103}NH$- wherein $R_{103}$ is a (substituted naphthyl)alkyl group as defined above.

The term "(phenyl)alkyl(alkyl)amino" as used herein refers to $R_{104}R_{105}N$- wherein $R_{104}$ is a (phenyl)-alkyl group as defined above and $R_{105}$ is a loweralkyl group.

The term "(substituted phenyl)alkyl(alkyl)amino" as used herein refers to $R_{106}R_{107}N$- wherein $R_{106}$ is a (substituted phenyl)alkyl group as defined above and $R_{107}$ is a loweralkyl group.

The term "(naphthyl)alkyl(alkyl)amino" as used herein refers to $R_{108}R_{109}N$- wherein $R_{108}$ is a (naphthyl)alkyl group as defined above and $R_{109}$ is a loweralkyl group.

The term "(substituted naphthyl)alkyl(alkyl)amino" as used herein refers to $R_{110}R_{111}N$- wherein $R_{110}$ is a (substituted naphthyl)alkyl group as defined above and $R_{111}$ is a loweralkyl group.

The term "dialkylaminoalkyl(alkyl)amino" as used herein refers to $-NR_{49}R_{50}$ wherein $R_{49}$ is a dial-kylamino residue appended to a loweralkyl residue and $R_{50}$ is a loweralkyl residue.

The term "alkylaminoalkylamino" as used herein refers to $-NHR_{50a}$ wherein $R_{50a}$ is an alkylaminoalkyl group as previously defined.

The term "dialkylaminoalkylamino" as used herein refers to $-NHR_{50b}$ wherein $R_{50b}$ is a dialkylaminoalkyl group as previously defined.

The term "aminoalkylamino" as used herein refers to $-NHR_{51}$ wherein $R_{51}$ is an aminoalkyl residue.

The term "(dihydroxyalkyl)(alkyl)amino" as used herein refers to a loweralkyl group which is disubstituted with -OH radicals, appended to an amino group, which amino group also has appended another loweralkyl group, including but not limited to N-(2,3-dihydroxypropyl)-N-(methyl)amine.

The term "di-(hydroxyalkyl)amino" as used herein refers to $-NR_{52}R_{53}$ wherein $R_{52}$ and $R_{53}$ are hydroxyalkyl residues.

The term "alkoxyalkyl(alkyl)amino" as used herein refers to $-NR_{54}R_{55}$ wherein $R_{54}$ is an alkoxyalkyl group and $R_{55}$ is a loweralkyl group.

The term "di-(alkoxyalkyl)amino" as used herein refers to $-NR_{56}R_{57}$ wherein $R_{56}$ and $R_{57}$ are alkoxyalkyl groups.

The term "di-(polyalkoxyalkyl)amino" as used herein refers to $-NR_{58}R_{59}$ wherein $R_{58}$ and $R_{59}$ are

polyalkoxy residues appended to loweralkyl residues.

The term ((polyalkoxy)alkyl)(alkyl)amino" as used herein refers to $-NR_{60}R_{61}$ wherein $R_{60}$ is a polyalkoxy residue appended to a loweralkyl residue and $R_{61}$ is a loweralkyl residue.

The term "halo" or "halogen" as used herein refers to -Cl, -Br, -I or -F.

The term "haloalkyl" as used herein refers to a loweralkyl radical in which one or more of the hydrogen atoms are replaced by halogen including, but not limited to, chloromethyl, trifluoromethyl, 1-chloro-2-fluoroethyl and the like.

The term "thioalkoxyalkyl" as used herein refers to a thioalkoxy group appended to a loweralkyl radical.

The term "alkylsulfonyl" as used herein refers to $R_{62}SO_2$- wherein $R_{62}$ is loweralkyl group.

The term "alkylsulfonylalkyl" as used herein refers to an alkylsulfonyl group appended to a loweralkyl radical.

The term "thioaryloxy" as used herein refers to $R_{63}S$-wherein $R_{63}$ is a substituted or unsubstituted phenyl or naphthyl group as defined above, including but not limited to phenyl, naphthyl, halophenyl and alkoxyphenyl.

The term "thioaryloxyalkyl" as used herein refers to a thioaryloxy group appended to a loweralkyl radical.

The term "arylsulfonyl" as used herein refers to $R_{63}SO_2$- wherein $R_{63}$ is as defined above.

The term "arylsulfonylalkyl" as used herein refers to an arylsulfonyl group appended to a loweralkyl radical.

The term "heterocyclic ring" or "heterocyclic" as used herein refers to any 3- or 4-membered ring containing a heteroatom selected from oxygen, nitrogen and sulfur, or a 5- or 6-membered ring containing from one to three heteroatoms selected from the group consisting of nitrogen, oxygen, and sulfur; wherein the 5-membered ring has 0-2 double bonds and the 6-membered ring has 0-3 double bonds; wherein the nitrogen and sulfur heteroatoms are optionally oxidized; wherein the nitrogen heteroatoms are optionally quaternized; and including any bicyclic group in which any of the above heterocyclic rings is fused to a benzene ring. Heterocyclics in which nitrogen is the heteroatom are preferred. Fully saturated heterocyclics are also preferred. Preferred heterocyclics are: pyrryl, pyrrolinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, pyrimidinyl, pyridazinyl, oxazolyl, oxazolidinyl, isoxazolyl, isoxazolidinyl, morpholinyl, thiazolyl, thiazolidinyl, isothiazolyl, isothiazolidinyl, indolyl, quinolinyl, isoquinolinyl, benzimidazolyl, benzothiazolyl, benzoxazolyl, furyl, thienyl and benzothienyl.

The most preferred heterocyclics are as follows:

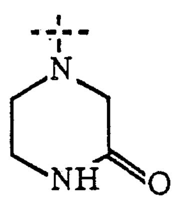 , 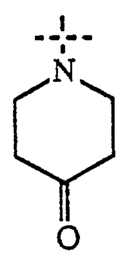 , 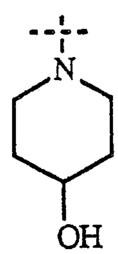 , 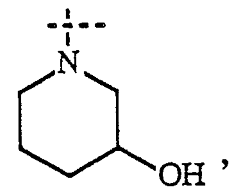 ,

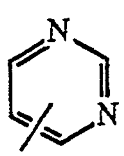 , 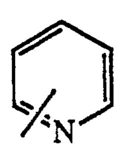 , 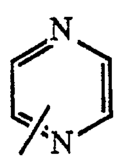 , 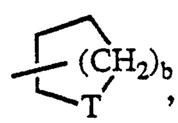 , 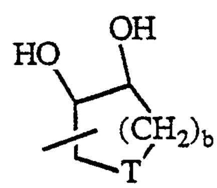

wherein b is 1 or 2 and T is N, NH, O, S, provided that T is the point of connection only when T is N,

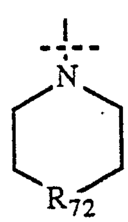

wherein $R_{72}$ is NH, N-loweralkyl, N-haloalkyl, O, S, or $SO_2$, or

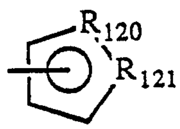 , 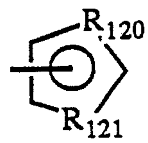 , 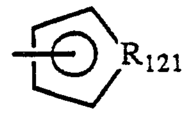

(i) (ii) (iii)

wherein the symbols (i), (ii) and (iii) represent 5-membered heterocycles containing one or more heteroatoms and containing 2 double bonds; wherein $R_{120}$ is N, O, or S and not the point of connection and $R_{121}$ is N when it is the point of connection and NH, O or S when it is not the point of connection.

The term "substituted heterocyclic" as used herein refers to saturated heterocyclics which are monosubstituted or disubstituted with hydroxy, oxo, amino, alkylamino, dialkylamino, alkoxy, polyalkoxy, haloalkyl or loweralkyl; or unsaturated heterocyclics which are monosubstituted with hydroxy, oxo, amino, alkylamino, dialkylamino, alkoxy, polyalkoxy, haloalkyl or loweralkyl.

The term "(heterocyclic)alkyl" as used herein refers to a heterocyclic group appended to a loweralkyl radical, including but not limited to imidazolylmethyl and thiazolylmethyl.

The term "(substituted heterocyclic)alkyl" as used herein refers to a substituted heterocyclic group as defined above appended to a loweralkyl radical.

The term "heterocyclic carbonyloxy" as used herein refers to $R_{205}C(O)O$- wherein $R_{205}$ is a heterocyclic group.

The term "(substituted heterocyclic) carbonyloxy" as used herein refers to $R_{206}C(O)O$- wherein $R_{206}$ is a substituted heterocyclic group.

The term "heterocyclic carbonylamino" as used herein refers to $R_{207}C(O)NH$- wherein $R_{207}$ is a heterocyclic group.

The term "(substituted heterocyclic) carbonylamino" as used herein refers to $R_{208}C(O)NH$- wherein $R_{208}$ is a substititued heterocyclic group.

The term "amino acyl residue" as used herein refers to a residue of the type -NHCH(R*)C(O)- wherein R* represents the side chain of one of the twenty naturally occuring alpha amino acids including, but not limited to alanyl, prolyl, leucyl, phenylalanyl, aspartyl and the like.

The terms "Ala", "Gln", "Leu", "Phe", "Tyr", "Asn", "Gly", "Ser", "Ile", "Sar" and "Pro" as used herein refer to alanine, glutamine, leucine, phenylalanine, tyrosine, asparagine, glycine, serine, isoleucine, sarcosine and proline, respectively.

The compounds of the invention can be prepared as shown in Scheme I. The syntheses of segments containing substituents $R_3$ and A-$CHR_1$-CO- are described in the Examples. The process shown in Scheme 1 discloses the carbodiimide coupling of acid (1) to substituent $R_3$ to give the peptide (2). Alternatively, carbodiimide coupling of protected amino acid (3) to substituent $R_3$ followed by hydrogenolytic deprotection provides the amine (4). Reaction of (4) with the mixed anhydride (5) gives the peptide (2).

## Scheme 1

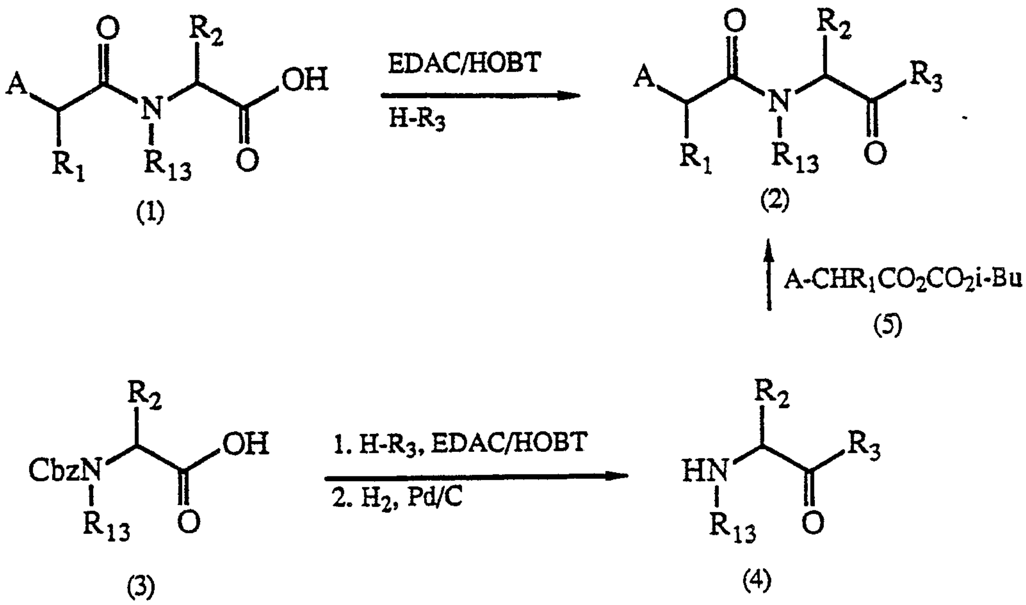


In addition to the use of carbodiimide based coupling, other coupling reagents known in the art can be used including, but not limited to, bis(2-oxo-3-oxazolidinyl)phosphinic chloride (BOP-Cl), dipenylphosphoryl azide (DPPA) and the like.

In addition to the use of the carboxylic acids or anhydrides shown in the scheme, acid halide and other activated ester derivatives of the carboxylic acid are useful for the coupling reactions with amines. Acid halide derivatives include the acid chloride. Activated ester derivatives include activated esters commonly used by those skilled in the art for activating carboxylic acid groups for coupling with an amine to form a peptide bond including, but not limited to, formic and acetic acid derived anhydrides, anhydrides derived from alkoxycarbonyl halides such as isobutyloxycarbonylchloride and the like, N-hydroxysuccinimide derived esters, N-hydroxyphthalimide derived esters, N-hydroxybenzotriazole derived esters, N-hydroxy-5-norbornene-2,3-dicarboxamide derived esters, 2,4,5-trichlorophenol derived esters and the like.

$H\text{-}R_3$ represents compounds of the formula:

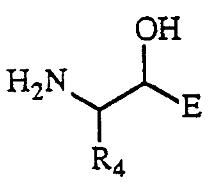 , 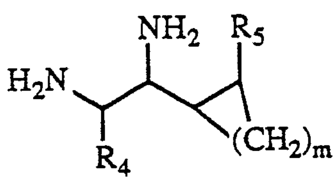 , 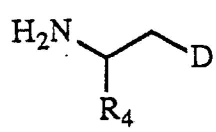 or 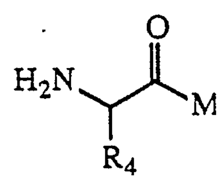

wherein m is 1 to 5;

$R_4$ is

(i) loweralkyl,

(ii) cycloalkyl,

(iii) cycloalkylalkyl,

(iv) (naphthyl)methyl,

(v) (substituted naphthyl)methyl wherein substituted naphthyl is independently as defined above,

(vi) benzyl or

(vii) substituted benzyl wherein the phenyl ring is substituted with a substituent selected from loweralkyl, alkoxy, thioalkoxy, hydroxy, halogen, mercapto, nitro, amino, loweralkylamino, dialkylamino, carboxaldehyde, carboxy, alkoxycarbonyl, and carboxamide;

$R_5$ is
(i) hydrogen,
(ii) loweralkyl,
(iii) -C(O)$R_6$ or
(iv) -C(O)-$R_{75}$-$R_6$ wherein $R_6$ is hydroxy, alkoxy or -N$R_{15}R_{16}$ wherein $R_{15}$ and $R_{16}$ are independently as defined above and $R_{75}$ is -NHCH($R''$)C(O)-, -NHCH($R''$)C(O)NHCH($R'''$)C(O)-, -NHCH($R''$)C(O)NHCH($R'''$)C(O)-$R_{220}$- or -NHCH($R''$)C(O)NHCH($R'''$)C(O)-$R_{220}$-$R_{221}$-wherein $R''$ is hydrogen, loweralkyl, cycloalkylalkyl, (phenyl)alkyl, (substituted phenyl)alkyl wherein substituted phenyl is independently as defined above, (naphthyl)alkyl or (substituted naphthyl)alkyl wherein substituted naphthyl is independently as defined above, $R'''$ is hydrogen, loweralkyl, hydroxyalkyl, aminoalkyl, carboxyalkyl, carboxamidoalkyl, N-hydroxycarboxamidoalkyl or guanidinoalkyl, and R220 and R221 are amino acyl residues independently selected from the twenty naturally occuring alpha amino acids;

M is
(i) hydrogen,
(ii) loweralkyl or
(iii) haloalkyl;

E is
(i) -CH($R_7$)($R_8$) wherein $R_7$ is hydrogen, amino or hydroxy and $R_8$ is loweralkyl, hydroxy, hydroxyalkyl, alkoxy, alkoxyalkyl, azido, azidoalkyl, amino, aminoalkyl, alkylamino, alkylaminoalkyl, dialkylamino, dialkylaminoalkyl, (N-protected)amino, (N-protected)aminoalkyl, (N-protected)(alkyl)amino, (N-protected)-(alkyl)aminoalkyl, thioalkoxy, thioalkoxyalkyl, alkylsulfonyl, alkylsulfonylalkyl, thioaryloxy, thioaryloxyalkyl, phenyl, substituted phenyl independently as defined above, naphthyl, substituted naphthyl independently as defined above, heterocyclic, substituted heterocyclic independently as defined above, phenylalkyl, (substituted phenyl)alkyl independently as defined above, naphthylalkyl, (substituted naphthyl)alkyl independently as defined above, heterocyclic alkyl, (substituted heterocyclic) alkyl independently as defined above, (phenyl)sulfonyl, (substituted phenyl)sulfonyl wherein substituted phenyl is independently as defined above, (naphthyl)sulfonyl, (substituted naphthyl)sulfonyl wherein substituted naphthyl is independently as defined above,
(phenyl)sulfonylalkyl, (substituted phenyl)sulfonylalkyl wherein substituted phenyl is independently as defined above,
(naphthyl)sulfonylalkyl, (substituted naphthyl)sulfonylalkyl wherein substituted naphthyl is independently as defined above, hydrogen, -C(=$CH_2$)C(O)NH$R_9$, -C(OH)($R_{10}$)C(O)NH$R_9$ or -CH($R_{10}$)C(O)NH$R_9$ wherein $R_9$ is loweralkyl, hydroxyalkyl, alkoxyalkyl, aminoalkyl, alkylaminoalkyl or dialkylaminoalkyl and $R_{10}$ is hydrogen, loweralkyl, hydroxyalkyl, haloalkyl or azidoalkyl, such that when $R_7$ is hydroxy or amino then $R_8$ is not hydroxy, alkoxy, azido, amino, alkylamino, dialkylamino, (N-protected)amino, (N-protected)(alkyl)amino, thioalkoxy, alkylsulfonyl, (phenyl)sulfonyl, (substituted phenyl)sulfonyl wherein substituted phenyl is independently as defined above, (naphthyl)sulfonyl or (substituted naphthyl)sulfonyl wherein substituted naphthyl is independently as defined above;
or E is
(ii) -$R_{200}$-$R_{201}$-$R_{202}$-$R_{203}$ wherein $R_{200}$ is an alkylene group, $R_{201}$ is S, S(O), $S(O)_2$, O, NH or -N(alkyl)-, $R_{202}$ is an alkylene group and $R_{203}$ is phenyl, substituted phenyl independently as defined above, naphthyl or substituted naphthyl independently as defined above;
or E is
(iii)

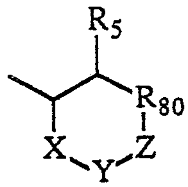 , 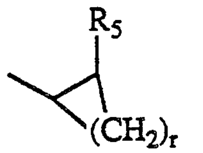 , 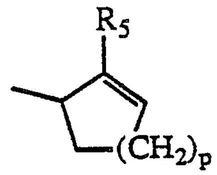 ,

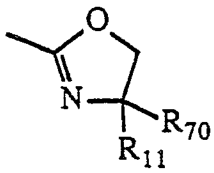 , 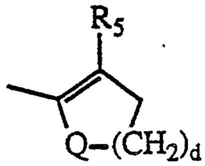 , 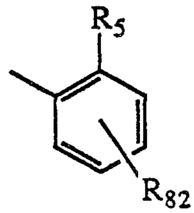 ,

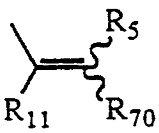 , 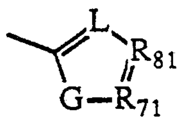 , 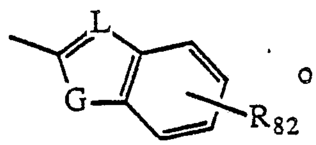 or

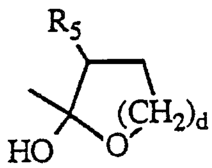

wherein $R_5$ is independently as defined above,

G is O, S, $-N(R_{11})-$ or $-C(R_{11})(R_{70})-$wherein $R_{11}$ is hydrogen or loweralkyl and $R_{70}$ is hydrogen or loweralkyl,

L is N or $-C(R_5)-$ wherein $R_5$ is independently as defined above,

$R_{81}$ is N or $-C(R_{11})-$ wherein $R_{11}$ is independently as defined above,

$R_{71}$ is N or $-C(R_{11})-$ wherein $R_{11}$ is independently as defined above,

$R_{80}$ is absent, $-C(R_{11})(R_{70})-$ or $-N(R_{11})-$wherein $R_{11}$ and $R_{70}$ are independently as defined above,

$R_{82}$ is absent, or one or two substituents independently selected from loweralkyl, alkoxy, thioalkoxy, hydroxy, halogen, mercapto, nitro, amino, loweralkylamino, dialkylamino, carboxaldehyde, carboxy, alkoxycarbonyl and carboxamide,

Q is O or $-C(R_{11})(R_{70})-$ wherein $R_{11}$ and $R_{70}$ are independently as defined above,

X is O, S, $-N(R_{11})-$, $-C(R_{11})(R_{70})-$, -C(O)- or $-C(=CH_2)-$ wherein $R_{11}$ and $R_{70}$ are independently as defined above,

Y is -C(O)-, -C(S)-, $-C(=CH_2)-$.or $-C(R_{11})(R_{70})-$ wherein $R_{11}$ and $R_{70}$ are independently as defined above,

Z is O, S, $-C(=CH_2)-$, -C(R11)(R70)-wherein $R_{11}$ and $R_{70}$ are independently as defined above, or $-N(R_{12})-$ wherein $R_{12}$ is hydrogen, loweralkyl, alkoxy, hydroxyalkyl or alkylamino,

d is 1 to 3, p is 0 to 3, and

r is 1, 2 or 5;

with the proviso that when $R_{80}$ is -N(R11)- then Z is O, S, $-N(R_{12})-$ wherein $R_{12}$ is independently as defined above or $-C(R_{11})(R_{70})-$ and with the proviso that when Y is $-C(=CH_2)-$ then X is -C(O)-, $-C(=CH_2)-$ or $-C(R_{11})(R_{70})-$ and Z is $-C(=CH_2)-$ or $-C(R_{11})(R_{70})-$; and

D is

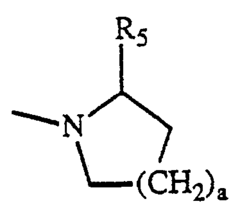

wherein a is 0 to 3 and $R_5$ is independently as defined above.

H-$R_3$ can optionally be obtained from the N-protected form of the compound.

The following examples will serve to further illustrate preparation of the novel compounds of the invention.

Example 1

N-Methyl-6-(2-(t-butyloxycarbonyl)amino-1-hydroxy-3-phenyl)propyl-1-cyclohexenecarboxamide.

A solution of 0.88 g (6.4 mmol) of N-methyl-1-cyclohexenecarboxamide in 30 ml of anhydrous tetrahydrofuran was cooled under nitrogen atmosphere to -78° C and treated with 5.2 ml (13.1 mmol) of n-butyllithium. The resulting solution was warmed to 0° C for 30 min, recooled to -78° C, and treated with 6.4 ml (6.4 mmol) of chlorotitanium triisopropoxide in hexane. After 10 min, a solution of 3.2 mmol of freshly prepared Boc-phenylalaninal (Chem. Pharm. Bull. **1982**, 30, 1921) in 10 ml of tetrahydrofuran was added. The resulting solution was stirred at 0° C for 30 min, treated with 10% citric acid and ether, and stirred until the salts had dissolved. The product was extracted into ether, washed with aqueous $NaHCO_3$ and saturated brine, dried over $MgSO_4$, and concentrated. Silica gel chromatography of the crude mixture using 4:1 ethyl acetate/chloroform gave 0.46 g (37%) of the desired compound as a mixture of diastereomers.

Example 2

N-Methyl-6-(2-amino-1-hydroxy-3-phenylpropyl)-1-cyclohexenecarboxamide Hydrochloride.

The resultant compound of Example 1 (0.23 g) was treated with 8 ml of 4 M HCl in dioxane. After 1 h, the solution was concentrated *in vacuo* to give the desired compound.

Example 3

Boc-Leu-Asn-OH

A solution of 10 g (40 mmol) of Boc-Leu-OH and 4.4 ml (40 mmol) of 4-methylmorpholine in 200 ml of anhydrous tetrahydrofuran was cooled under $N_2$ atmosphere to -10° C and treated with 5.2 ml (40 mmol) of isobutyl chloroformate. After 15 min, the solution was treated with a mixture of 5.30 g (40 mmol) of asparagine and 25 ml of 3 N NaOH in 50 ml of tetrahydrofuran and 200 ml of water. The resulting mixture was stirred at ambient temperature for 2.5 h and concentrated *in vacuo* to an aqueous residue, which was acidified with conc. HCl, extracted 3 times with chloroform, dried over $MgSO_4$, and reduced to a white foam. The foam was taken up in 50 ml of ethyl acetate, heated to boiling, and treated with portions of hexane until a precipitate appeared. The mixture was allowed to cool and filtered to give 5.79 g (42%) of the desired compound as a pure white crystalline solid. $^1H$ NMR ($d_6$-DMSO) d 0.84 (d,J=7Hz,3H), 0.86 (d,J=7Hz,3H), 1.37 (s,9H), 1.40 (t,J=7Hz,2H), 1.60 (m,1H), 3.95 (q,J=8Hz,1H), 4.46 (q,J=8Hz,1H), 6.80 (br s,1H), 6.82 (br d,1H), 7.37 (br s,1H), 7.91 (br d,J=8Hz,1H), 12.5 (br,1H). Mass spectrum: $(M+H)^+$ = 346.

Anal. Calcd. for $C_{15}H_{27}N_3O_6$. 0.25 $H_2O$: C, 51.49; H, 7.92; N, 12.01.

Found: C, 51.61; H, 7.87; N, 12.01.

Example 4

Boc-Leu-Asn Amide of N-Methyl-6-(2-amino-1-hydroxy-3-phenylpropyl)-1-cyclohexenecarboxamide.

A solution of 77 mg (0.22 mmol) of Boc-Leu-Asn-OH and 0.024 ml (0.22 mmol) of 4-methylmorpholine in 5 ml of dichloromethane was cooled under $N_2$ atmosphere to -10°C and treated with 0.029 ml (0.22 mmol) of isobutyl chloroformate. After 15 min, the solution was treated with a solution of 61 mg (0.21 mmol) of the resultant compound of Example 2 and 0.24 ml of 4-methylmorpholine in 2 ml of dichloromethane. The resulting solution was stirred at ambient temperature for 2 h, diluted with ethyl acetate, washed sequentially with 1 M HCl, $H_2O$, aqueous $NaHCO_3$, and saturated brine; dried over $MgSO_4$, and concentrated. Silica gel chromatography using methanol/chloroform gave 12.4 mg (10%) of one desired diastereomeric product and 25.6 mg (20%) of another desired diastereomeric product. (6S,1′R,2′S)-isomer: $^1H$ NMR ($CDCl_3$) δ 0.92 (d,J=7Hz,3H), 0.93 (d,J=7Hz,3H), 1.43 (s,9H), 1.4-1.9 (br envelope), 2.52 (dd,J=15,7Hz,1H), 2.67 (dd,J=15,5Hz,1H), 2.2-2.3 (m,1H), 2.83 (q,J=5Hz,3H), 3.04 dd,J=11,4Hz,1H), 3.72 (m,1H), 4.00 (m,1H), 4.36 (m,1H), 4.56 (m,1H), 4.95 (m,1H), 5.08 (m,1H), 5.45 (br,1H), 6.21 (br, 1H), 6.54 (br t,J=3Hz,1H), 6.72 (br,1H), 7.15-7.3 (m,5H), 7.61 (br d,1H). Mass spectrum: $(M+H)^+$ = 616.
Anal. Calcd. for $C_{32}H_{49}N_5O_7 \cdot 2.5\ H_2O$: C, 58.16; H, 8.24; N, 10.60. Found: C, 58.11; H, 7.52; N, 10.34.
(6R,1′S,2′S)-isomer: $^1H$ NMR ($CDCl_3/CD_3OD$) δ0.93 (d,J=7Hz,3H), 0.96 (d,J=7Hz,3H), 1.4-2.0 (br envelope), 1.49 (s,9H), 2.13 (m,2H), 2.41 (dd,J=15,6Hz,1H), 2.45 (dd,J=15,6Hz,1H), 2.7-2.8 (m,2H), 2.82 (s,3H), 3.31 (br d,J=15Hz,1H), 3.67 (dd,J=8,4Hz,1H), 3.93 (dd,J=10,6Hz,1H), 4.20 (m,1H), 4.51 (t,J=6Hz,1H), 6.57 (br t,1H), 7.1-7.25 (m,5H). Mass spectrum: $(M+H)^+$ = 616.

## Example 5

N-(3-Methylbutyl)-1-cyclopentenecarboxamide.

1-Cyclopentenecarboxylic acid (7.17 g, 64 mmol) was coupled to isoamylamine using the procedure of Example 4 to give, after silica gel chromatography using 7:3 hexane/ethyl acetate, 7.86 g (68%) of the desired compound as a white crystalline solid. $^1H$ NMR ($CDCl_3$) δ0.93 (d,J=7Hz,6H), 1.43 (q,J=7Hz,2H), 1.64 (heptet,J=7Hz,1H), 1.99 (pentet,J=7Hz,2H), 2.4-2.6 (m,4H), 3.34 (m,2H), 5.58 (br,1H), 6.51 (m,1H).

## Example 6

N-(3-Methylbutyl)-5-(2-(t-butyloxycarbonyl)amino-1-hydroxy-3-phenyl)propyl-1-cyclopentenecarboxamide.

Using the procedure of Example 1 but replacing N-methyl-1-cyclohexenecarboxamide with the resultant compound of Example 5 gives the desired compound as a mixture of diastereomers.

## Example 7

Boc-Leu-Asn Amide of N-(3-Methylbutyl)-5-(2-amino-1-hydroxy-3-phenyl)propyl-1-cyclopentenecarboxamide.

Using the procedures of Examples 2 and 4 with the resultant compound of Example 6 gives the desired compound.

Example 8

((4-Morpholinyl)carbonyl)-Leu Methyl Ester.

A suspension of L-leucine methyl ester hydrochloride (6 g) in toluene (125 ml) is heated to 100°C while phosgene gas is bubbled into the reaction mixture. After approximately 1.5-2 h, the mixture becomes homogeneous. The passage of phosgene is continued for an additional 15 min, keeping the temperature at 90-100°C. The toluene is then evaporated and the residue chased several times with benzene. A 31.7 mmol sample of a-isocyanato-L-leucine methyl ester is dissolved in 50 ml of methylene chloride and cooled to 0°C. Morpholine (2.76 ml, 31.7 mmol) dissolved in 5 ml of methylene chloride is added dropwise. After 10 min at 0-5°C, the reaction mixture is distributed between 0.5 N HCl and methylene chloride. The organic layer is washed with aqueous $NaHCO_3$ and dried over $MgSO_4$. Evaporation of the solvent gives the desired compound after trituration with hexane.

Example 9

((4-Morpholinyl)carbonyl)-Leu-OH.

To a 0°C solution of the product from Example 8 (3.63 mmol) in dioxane (15 ml) is added a solution of lithium hydroxide (0.174 g, 4.15 mmol) in water (7.5 ml). After stirring for 1 h at 0-5°C, the reaction mixture is diluted with cold water and extracted 2X with ether. The aqueous portion is acidified with 6N HCl and extracted with ether. The organic extract is washed with brine and evaporated to give the desired compound.

Example 10

3-Benzyloxycarbonylamino-3-methylbutanoic Acid.

A solution of 2,2-dimethyl-3-carbomethoxypropionic acid (LeMaul, Bull. Soc. Chim. Fr., 828 (1965), 20 g, 0.125 mol), diphenylphosphorylazide (34.3 g, 0.125 mol) and triethylamine is heated in toluene (150 ml) at 100°C for 2 h. After cooling to 5°C, the toluene solution is washed successively with 0.5 M HCl, aqueous $NaHCO_3$ and brine. Evaporation of the dried solution gave a residue which is chromatographed on silica gel eluting with 60/40 hexane-ether. There is obtained 13 g of methyl 3- isocyanato-3-methylbutanoate as a mobile liquid. A solution of this material in toluene (20 ml) is treated with benzyl alcohol (13 ml) and the resulting mixture heated at reflux for 40 h. Evaporation of the toluene left a residue which is dissolved in methanol (125 ml) and then treated with a solution of NaOH (6.6 g, 0.165 mol) in 22 ml of water. After 5 h, the reaction mixture is partially evaporated, washed with ether and acidified with 6N HCl. Extraction with methylene chloride and evaporation gave 21 g of the desired product. NMR (300 MHz, $CDCl_3$): 1.42 (s,6H), 2.78 (s,2H), 5.08 (s,2H).

Example 11

Cbz-((β,β-di-Me)-β-Ala)-Leu-$OCH_3$.

A 4.0 g sample of 3-benzyloxycarbonylamino-3-methylbutanoic acid is coupled to leucine methyl ester hydrochloride using the mixed anhydride procedure described in Example 4. Purification of the crude product by flash chromatography gives the desired compound.

## Example 12

### Cbz-((β,β-di-Me)-β-Ala)-Leu-OH.

To a 0°C solution of Cbz-((β,β-di-Me)-β-Ala)-Leu-OMe (3.63 mmol) in dioxane (15 ml) is added a solution of lithium hydroxide (0.174 g, 4.15 mmol) in water (7.5 ml). After stirring for 1 h at 0-5°C, the reaction mixture is diluted with cold water and extracted 2X with ether. The aqueous portion is acidified with 6N HCl and extracted with ether. The organic extract is washed with brine and evaporated to give the desired compound.

## Example 13

### (4R)-3-(4-Methylpentanoyl)-4-(2-propyl)oxazolidine-2-one.

To a stirred solution of 4-(2-propyl)-oxazolidine-2-one in anhydrous tetrahydrofuran (250 ml) under a nitrogen atmosphere at -78°C is added in a dropwise fashion a solution of n-butyllithium in hexane (50 ml, 77.4 mmol) over 5 to 10 min. After stirring an additional 20 min at -78°C, 4-methylpentanoyl chloride (85.2 mmol) is added neat. The reaction is warmed to room temperature and stirred 1 to 2 h at the temperature. The reaction is quenched by adding 100 ml of saturated aqueous ammonium chloride and the volatiles are removed by rotary evaporation. The resulting aqueous residue is extracted three times with ether and the combined organic phases are washed with brine, dried ($Na_2SO_4$), filtered and concentrated in vacuo Recrystallization from hexanes/ethyl acetate provides the desired compound.

## Example 14

### (4R)-3-((2-R)-2-(t-Butyloxycarbonyl)methyl-4-methylpentanoyl)-4-(2-propyl)oxazolidine-2-one.

To a stirred solution of the product resulting from Example 13 (8.72 mmol) in anhydrous tetrahydrofuran (30 ml) under a nitrogen atmosphere at -78°C is added a solution of sodium hexamethyldisilylamide (9.6 ml, 9.59 mmol) in tetrahydrofuran. After stirring for 30 min at -78°C, t-butyl bromoacetate (2.21 g, 11.34 mmol) is added in anhydrous tetrahydrofuran and the resulting solution stirred 1 h at -78°C. The reaction is quenched by adding 20 ml of saturated aqueous ammonium chloride and partitioned between water and ether. The aqueous layer is drawn off and extracted with ether. The combined organic phases are washed with 10% aqueous HCl, saturated aqueous $NaHCO_3$, and brine, dried ($Na_2SO_4$), filtered, and concentrated in vacuo. Recrystallization from acetone/hexanes provides the desired compound.

## Example 15

### Benzyl-(2R)-2-(t-Butyloxycarbonyl)methyl-4-methylpentanoate.

To a stirred solution of dry benzyl alcohol (0.55 ml, 5.33 mmol) in anhydrous tetrahydrofuran (18 ml) under a nitrogen atmosphere at 0° C is added a hexane solution of n-butyllithium (2.58 ml; 4.00 mmol). To this solution is added the product from Example 55 in anhydrous tetrahydrofuran (10 ml). After stirring 1 h at 0° C the reaction is quenched by adding excess saturated aqueous ammonium chloride. The volatiles are removed by rotary evaporation and the resulting aqueous residue is extracted two times with ether. The combined organic layers are washed with brine, dried ($Na_2SO_4$), filtered, and concentrated in vacuo to provide an oil which is purified by chromatography on $SiO_2$ to give the desired compound.

## Example 16

### Benzyl-(2R)-2-(Carboxymethyl)-4-methylpentanoate.

The resultant compound from Example 15 (1.47 mmol) is dissolved in a 1:1 (v:v) solution (6 ml) of trifluoroacetic acid and dichloromethane and stirred at room temperature for 1 h. The volatiles are removed in vacuo to provide the desired compound. The unpurified material is of sufficient purity to employ in subsequent steps.

## Example 17

### Benzyl(2R)-2-Isobutyl-3-morpholinocarbonylpropionate.

The resultant compound of Example 16 is coupled to morpholine using the mixed anhydride procedure as described in Example 4 to give the desired compound.

## Example 18

### (3,4-cis-Dihydroxypyrrolidinylcarbonyl)-Leu-Methyl Ester.

A suspension of L-leucine methyl ester hydrochloride (10 g) in toluene (f200 ml) is heated to 100° C while phosgene gas was bubbled into the reaction mixture. After approximately 2 h the mixture becomes homogeneous. The bubbling of phosgene is continued for 15 more minutes keeping the temperature at 100° C. The toluene is then evaporated and the residue chased with benzene several times. The isocyanate from L-Leu-$OCH_3$ is then dissolved in 100 ml of methylene chloride and 1.1 equivalent of 3-pyrroline (75% pure) is added dropwise at 0° C. After 15 min, the reaction mixture is washed with 0.5 N HCl and methylene chloride. The organic layer is washed with aqueous $NaHCO_3$ and dried over $MgSO_4$. Evaporation of the solvent gives 3-pyrrolinylcarbonyl-Leu-methyl ester which is cis-hydroxylated under the following conditions: 2.5 g of the 3-pyrrolinylcarbonyl-Leu-methyl ester is dissolved in 50 ml of THF and 1 ml of a 2.5% solution of $OsO_4$ in t-butanol is added, followed by 1.15 g of N-methylmorpholine-N-oxide. After 1 h, the solvent is evaporated and the residue dissolved in 150 ml of ethyl acetate, washed with dilute $Na_2SO_3$ solution and satd. $NaHCO_3$ solution, and then dried over $MgSO_4$. Evaporation of the solvent gives a gummy solid which is purified by $SiO_2$ column chromatography to give the desired compound.

## Example 19

((4-Thiomorpholinyl)carbonyl)-Leu Methyl Ester.

A suspension of L-leucine methyl ester hydrochloride (6 g) in toluene (125 ml) is heated to 100 °C and phosgene gas is bubbled into the reaction mixture. After approximately 1.5 h, the mixture becomes homogeneous. The bubbling of phosgene is continued for 10 more min. The solvent is then evaporated and the residue chased with benzene several times. The residue is then dissolved in 100 ml of methylene chloride and cooled to 0 °C, and 1.1 equivalent of thiomorpholine is added dropwise. After 10 min the solution is washed with 1N HCl and the organic layer was dried with $MgSO_4$. Evaporation of solvent gives the desired compound.

Example 20

((4-Sulphonylmorpholinyl)carbonyl)-Leu Methyl Ester.

To 2 g of the resultant compound of Example 19 in 100 ml of methylene chloride is added 2.94 g of a metachloroperbenzoic acid at 0 °C. After 30 min the solvent is evaporated and the ether solution is washed with 10% sodium sulfite solution and then with satd.sodium bicarbonate several times. The organic layer is dried with $MgSO_4$ and evaporation of the solvent gives the crude product which is purified by silica gel column chromatography to give the desired compound.

Example 21

Boc-6-aminohexanoic acid.

A mixture of 3.0 g (0.02 mol) of 6-aminohexanoic acid, 5.04 g (0.02 mol) of di-t-butyldicarbonate and 3.84 g (0.05 mol) of $NaHCO_3$ in 160 ml of 1:1 $H_2O$/tetrahydrofuran is stirred vigorously for 24 h. After concentration of the solvent, the mixture is acidified with HCl, saturated with NaCl, extracted with ethyl acetate, dried over $MgSO_4$ and concentrated in vacuo to give the desired product ($R_f$ 0.48, 9:1 chloroform/methanol).

Example 22

Boc-6-aminohexanoyl-Leu benzyl ester.

A solution of 1.50 g (6.5 mmol) of the resultant compound of Example 21, 6.5 mmol of L-leucine benzyl ester p-toluenesulfonate salt, 0.87 g (6.5 mmol) of 1-hydroxybenzotriazole hydrate, 1.19 ml (8.4 mmol) of triethylamine and 1.74 g (8.4 mmol) of dicyclohexylcarbodiimide in 150 ml of tetrahydrofuran is allowed to stir at ambient temperature for 18 h. After concentration in vacuo, the residue is taken up in 300 ml of ethyl acetate, filtered, washed consecutively with 1 M HCl, $H_2O$, saturated $NaHCO_3$, $H_2O$ and saturated NaCl; dried over $MgSO_4$ and concentrated. Purification by flash column chromatography gives the desired compound.

Example 23

Boc-6-aminohexanoyl-Leu-OH.

A mixture of 1.07 mmol of the resultant compound of Example 22 and 30 mg of 10% palladium on carbon in 50 ml of methanol is stirred under an $H_2$ atmosphere for 3.5 h. After filtration through Celite, concentration in vacuo gives the desired compound.

Example 24

N-Methyl-N-(2-(N,N-dimethylamino)ethyl)carbamoyl-leucine methyl ester.

A solution of 2.1 mmol of a-isocyanoto-leucine methyl ester in 50 ml of dichloromethane is cooled to 0°C and treated with 0.3 ml (2.3 mmol) of N,N,N′-trimethylethylenediamine. After being allowed to stir for 16 h, the solution is concentrated and the desired compound is isolated by flash column chromatography.

Example 25

N-Methyl-N-(2-(N,N-dimethylamino)ethyl)carbamoyl-leucine lithium salt.

A solution of the resultant compound of Example 24 in dioxane is cooled to 0°C, treated with 1.05 equiv. of aqueous lithium hydroxide (0.5 M) and stirred for 1.5 h. The resulting solution is concentrated in vacuo to give the desired compound as a white solid.

Example 26

(2R)-2-Isobutyl-3-morpholinocarbonylpropionic Acid.

The resultant compound of Example 17 is a hydrogenolyzed according to the procedure of Example 23 to provide the desired compound.

Example 27

(3,4-cis-Dihydroxypyrrolidinylcarbonyl)-Leu-OH.

The resultant compound of Example 18 is hydrolyzed according to the procedure of Example 9 to provide the desired compound.

Example 28

(4-Thiomorpholinylcarbonyl)-Leu-OH.

The resultant compound of Example 19 is hydrolyzed according to the procedure of Example 9 to provide the desired compound.

Example 29

(4-Sulphonylmorpholinylcarbonyl)-Leu-OH.

The resultant compound of Example 20 is hydrolyzed according to the procedure of Example 9 to provide the desired compound.

Example 30

(((4-Morpholinyl)carbonyl)oxy)-4-methylpentanoic Acid Methyl Ester.

To 2-hydroxy-4-methylpentanoic acid methyl ester is added 150 ml of 12.5% phosgene in toluene and 25 drops of dimethylformamide. After stirring for 16 h at room temperature, the solvent is evaporated and the residue chased several times with benzene. The resulting product is dissolved in methylene chloride (50 ml), cooled to 0° C and treated by dropwise addition with 3.86 g (0.044 mol) of morpholine. The reaction mixture is stirred for 2 h at 0-5° C and then distributed between 0.5 N HCl and methylene chloride. The organic phase is washed with aqueous $NaHCO_3$ and brine and evaporated to a residue. Flash chromatography on silica gel gives the desired compound.

Example 31

(((4-Morpholinyl)carbonyl)oxy)-4-methylpentanoic Acid.

The resultant compound of Example 30 is hydrolyzed according to the procedure of Example 9 to provide the desired compound.

Example 32

Benzyl-(2R)-2-isobutyl-3-(((4-morpholinyl)carbonyl)amino)-propionate.

The product from Example 16 (1.47 mmol), diphenylphosphoryl azide (1.47 mmol), and triethylamine (1.47 mmol) in dry benzene (6 ml) are refluxed for 3 to 5 h to provide a solution of the derived isocyanate

which is cooled to 0 °C and treated with morpholine (1.6 mmol). The cooling bath is removed and the reaction stirred for 1 h. The reaction mixture is poured into 10% aqueous HCl and extracted two times with ether. The combined organic layers are washed successively with saturated aqueous $NaHCO_3$ and brine, dried ($Na_2SO_4$), filtered and concentrated in vacuo to provide the unpurified product. The desired product is obtained in pure form after chromatography on $SiO_2$.

Example 33

Benzyl-(2R)-2-isobutyl-3-((ethoxycarbonyl)amino)-propionate.

Using the procedure of Example 32 but replacing morpholine with ethanol gives the desired compound.

Example 34

(2R)-2-Isobutyl-3-(((4-morpholinyl)carbonyl)amino)propionic Acid.

The resultant compound of Example 32 is hydrolyzed according to the procedures of Example 9 to give the desired compound.

Example 35

(2R)-2-Isobutyl-3-((ethoxycarbonyl)amino)propionic Acid.

The resultant compound of Example 33 is hydrolyzed according to the procedures of Example 9 to give the desired compound.

Example 36

1-Benzyloxycarbonylamino-2,3-propanediol.

1-Amino-2,3-propanediol (15.2 g, 167 mmol) and NaOH (8.1 g, 204 mmol) in water (70 ml) at -10 °C was treated dropwise with benzyl chloroformate (28.5 ml, 200 mmol) in ether (30 ml) over 20 min. The reaction was stirred at 0 °C for 30 min then at room temperature for 2 h. The mixture was acidified with 2 M HCl and extracted with ethyl acetate which was washed with 0.5 M $H_3PO_4$ and brine, then dried over $Na_2SO_4$ and evaporated. Recrystallization of the residue from benzene afforded 16.59 g (44%) of the desired product as a white powder. NMR (300 MHz, $CD_3OD$, ppm): 3.12 (dd,1H), 3.28 (dd,1H), 3.50 (m,2H), 3.68 (m,1H), 5.08 (s,2H), 7.35 (m,5H).

Example 37

1-Methylamino-2,3-propanediol.

Lithium aluminum hydride (7.20 g, 189 mmol) in tetrahydrofuran (THF, 300 ml) was heated to reflux and the resultant compound from Example 36 (17.0 g, 75.5 mmol) in THF (150 ml) was added dropwise over 10 min. The mixture was refluxed for 2 h, cooled, quenched sequentially with water (10 ml), 3 M NaOH (40 ml) and water (20 ml), then filtered and concentrated. The residue was dissolved in water which was washed with ether and evaporated. Bulb to bulb distillation of the residue afforded 2.0 g (25%) of the desired compound as an oil. NMR (300 MHz, $CDCl_3$, ppm): 2.45 (s,3H), 2.68 (dd,1H), 2.77 (dd,1H), 3.61 (dd,1H), 3.72 (dd,1H), 3.78 (m,1H).

Example 38

(N-Methyl-2,3-dihydroxypropylamino)carbonyl-leucine Methyl Ester.

Using the procedure of Example 19 but replacing thiomorpholine with the resultant compound of Example 37 gives the desired compound.

Example 39

(N-Methyl-2,3-dihydroxypropylamino)carbonyl-leucine.

The resultant compound of Example 38 is hydrolyzed according to the procedures of Example 9 to give the desired compound.

Example 40

5-(Methoxycarbonyl)pentanoyl-leucine Benzyl Ester.

Adipic acid monomethyl ester is coupled to leucine benzyl ester hydrochloride using the mixed anhydride procedure described in Example 4 to provide the desired compound.

Example 41

5-(Methoxycarbonyl)pentanoyl-leucine.

The resultant compound of Example 40 is hydrogenolyzed according to the procedure of Example 23 to provide the desired compound.

Example 42

Isobutyryl leucine.

To leucine (0.6 mmol) in dioxane (3 ml) is added NaOH (1.3 mmol) in water (3 ml). Isobutyric anhydride (0.6 mmol) is added and the mixture is stirred at 0° C for 2 h, poured into saturated $NaHCO_3$ solution and extracted with ether.

The aqueous phase is acidified with concentrated HCl then extracted with ethyl acetate which is dried over $Na_2SO_4$ and evaporated. Trituration with hot ethyl acetate affords the desired compound.

Example 43

N-Benzyloxycarbonyl-N-methyl-2-aminoethanol.

To N-methylethanolamine (149 mmol) in methylene chloride (100 ml) at 0° C was added benzyl chloroformate (70 mmol). The mixture was stirred at 0° C for 30 min, then at room temperature for 1 h, poured into ethyl acetate, washed with 2 M HCl, saturated $NaHCO_3$ solution, and brine, then dried over $Na_2SO_4$ and evaporated to provide the desired compound. $^1H$ NMR ($CDCl_3$,TMS) $\delta$ 7.36 (m,5H), 5.14 (s,2H), 3.78 (m,2H), 3.47 (m,2H), 3.01 (s,3H).

Example 44

1-Methoxyethoxymethoxy-2-(N-methyl-N-benzyloxycarbonylamino)ethane.

To the resultant compound from Example 43 (66 mmol) in methylene chloride (100 ml) was added diisopropylethylamine (138 mmol) and 2-methoxyethoxymethyl chloride (132 mmol). After 4 h the mixture was evaporated, dissolved in ethyl acetate, washed with 0.5 M $H_3PO_4$, saturated $NaHCO_3$ solution, and brine, then dried over $Na_2SO_4$, and evaporated to afford the desired product as an oil, b.p. 150-170° C (0.3 mm).

Example 45

1-Methylamino-2-methoxyethoxymethoxyethane.

The resultant compound from Example 44 (31 mmol) and 10% palladium on carbon (3 g) in methanol (60 ml) were stirred under a hydrogen atmosphere for 24 h. The mixture was filtered, evaporated and distilled to afford the desired product as an oil, b.p. 130-140° C (45 mm).

Example 46

Benzyl (2R)-2-Isobutyl-3-(N-methyl-N-(2-methoxyethoxymethoxethyl)aminocarbonyl)propionate.

The resultant compound of Example 16 is coupled to the resultant compound of Example 45 using the

mixed anhydride procedure of Example 4 to give the desired compound.

Example 47

(2R)-2-Isobutyl-3-(N-methyl-N-(2-methoxyethoxymethoxyethyl)aminocarbonyl)propionic Acid.

The resultant compound of Example 46 is hydrolyzed according to the procedure of Example 9 to give the desired compound.

Example 48

N-(2-Cyanoethyl)leucine Methyl Ester.

A solution of leucine methyl ester (0.590 mmol) in acrylonitrile (2 ml) is heated at reflux. Evaporation provides a residue which is chromatographed on silica gel to give desired compound.

Example 49

N-(3-Benzyloxycarbonylaminopropyl)leucine Methyl Ester.

The resultant compound of Example 48 (0.135 mmol) is hydrogenated (4 atmospheres $H_2$) over Raney Nickel (85 mg) in anhydrous methanol/ammonia (20 ml/5 ml) for 3 h. Filtration and evaporation provide the crude amine which is taken up in dichloromethane and treated with 0.14 mmol of N-(benzyloxycarbonyloxy)succinimide. After 2 h, the solution is washed with aqueous $NaHCO_3$, dried over $Na_2SO_4$, and concentrated. Silica gel chromatography gives the desired compound.

Example 50

N-(3-Benzyloxycarbonylaminopropyl)leucine.

The resultant compound of Example 49 is hydrolyzed according to the procedure of Example 9 to give the desired compound.

Example 51

Cbz-Sar-Leu Methyl Ester.

Cbz-sarcosine is coupled to leucine methyl ester using the mixed anhydride procedure of Example 4 to

give the desired compound.

Example 52

Cbz-Sar-Leu-OH.

The resultant compound of Example 51 is hydrolyzed according to the procedure of Example 9 to give the desired compound.

Example 53

(N,N-Dimethyl)-Gly-Leu-$OCH_3$.

The resultant product of Example 51 (0.243 mmol) is hydrogenated (1 atmosphere $H_2$) with 10% Pd/C (39 mg) in methanol/formalin (12 ml/5 ml) for 8 h. Filtering and evaporating (high vacuum) provides a residue which is chromatographed on silica gel to give the desired compound.

Example 54

(N,N-Dimethyl)-Gly-Leu-$O^{-}Li^{+}$

The resultant compound of Example 53 is hydrolyzed according to the procedure of Example 25 to give the desired compound.

Example 55

(N-(Benzyloxycarbonyl)pipiridin-4-yl)carbonyl-leucine Methyl Ester.

Cbz-isonipecotic acid is coupled to leucine methyl ester using the mixed anhydride procedure of Example 4 to give the desired compound.

Example 56

(N-(Benzyloxycarbonyl)pipiridin-4-yl)carbonyl-leucine.

The resultant compound of Example 55 is hydrolyzed according to the procedure of Example 9 to give the desired compound.

Example 57

N-(Allyloxycarbonyl)-leucine Methyl Ester.

A solution of leucine methyl ester (5 mmol) and triethylamine (10 mmol) in dichloromethane (50 ml) is cooled to 0° C and treated dropwise with allyl chloroformate. After addition, the solution is stirred at ambient temperature for 2 h, diluted with dichloromethane, washed successively with 1 N HCl and aqueous $NaHCO_3$, dried over $Na_2SO_4$, and concentrated to give the desired compound.

Example 58

N-(3-Hydroxypropyloxycarbonyl)-leucine Methyl Ester.

To a stirred 0° C solution of the resultant compound of Example 57 (2.13 mmol) in dry tetrahydrofuran (THF, 50 ml) is added 9-borabicyclo(3.3.1)nonane (9-BBN, 25.5 ml of a 0.5 M solution in THF). The mixture is warmed to room temperature for 12 h and then cooled to 0° C. Water (15 ml) and 3 M NaOH (4.5 ml) are added followed 2 min later by 30% $H_2O_2$ (5 ml). The mixture is partitioned between brine (20 ml) and ethyl acetate (100 ml). The organic phase is washed (brine), dried ($Na_2SO_4$), filtered, and evaporated. Silica gel chromatography provides the desired compound.

Example 59

N-(3-Hydroxypropyloxycarbonyl)-leucine.

The resultant compound of Example 58 is hydrolyzed according to the procedure of Example 9 to give the desired compound.

Example 60

N-(2,3-Dihydroxypropyloxycarbonyl)-leucine Methyl Ester.

To a solution of the resultant compound of Example 57 (27.5 mmol) in dry THF (150 ml) are added $OsO_4$ (2.8 ml of a 2.5% solution in t-butanol) and N-methylmorpholine N-oxide (68.7 mmol). After 4 days the mixture is partitioned between ether (200 ml) and brine (100 ml). The aqueous layer is back-extracted with ether (2x100 ml), and the combined organic phase is washed with 10% $Na_2SO_3$, 0.1 M $H_3PO_4$, and brine. Drying ($MgSO_4$) and evaporating provides a residue which is chromatographed on silica gel to give the desired compound.

Example 61

3-Benzyloxycarbonyl-4-(4-carboxybutyl)oxazolidin 5-one.

A solution of 3.0 g (17.1 mmol) of dl-α-aminopimelic acid in 30 ml of dioxane and 10 ml of water was treated simultaneously in a dropwise fashion with 4.8 g (18.8 mmol) of N-(benzyloxycarbonyloxy)-succinimide in 10 ml of dioxane and 17 ml (51 mmol) of 3 N NaOH. After the addition was complete, the reaction mixture was stirred for 1.5 h and concentrated in vacuo The residue was taken up in dilute NaOH, washed with chloroform, acidified to pH 1 with concentrated HCl, extracted with three 200 ml portions of chloroform, washed with saturated brine, dried over $MgSO_4$, and concentrated to give 3.21 g of a white solid. The solid was taken up in benzene along with 0.17 g (0.57 mmol) of p-toluenesulfonic acid monohydrate and 0.57 g of paraformaldehyde. The resulting suspension was heated at reflux with azeotropic removal of water for 7 h. The solution was washed with two 10 ml portions of water and extracted with 100 ml of 1 M $NaHCO_3$. The basic layer was cooled to 0 °C, acidified with concentrated HCl, extracted with three 200 ml portions of ethyl acetate, dried over $MgSO_4$, and concentrated. Purification by silica gel chromatography using 10% methanol in chloroform as eluent gave 2.61 g of the desired compound. Mass spectrum: $M^+$ = 321.

Example 62

N-(3-Benzyloxypropanoyl)-leucine Methyl Ester.

3-Benzyloxypropanoic acid is coupled to leucine methyl ester using the mixed anhydride procedure of Example 4 to give the desired compound.

Example 63

N-(3-Benzyloxypropanoyl)-leucine.

The resultant compound of Example 62 is hydrolyzed according to the procedure of Example 9 to give the desired compound.

Example 64

N-(2,3-Dihydroxypropyloxycarbonyl)-leucine.

The resultant compound of Example 60 is hydrolyzed according to the procedure of Example 9 to give the desired compound.

Example 65

(3S,5S,1′R,2′S)-5-(2-Amino-3-cyclohexyl-1-hydroxypropyl)-3-methyldihydrofuran-2(3H)-one Hydrochloride.

The resultant compound of Example 106 (1.13 g, 2.86 mmol) was dissolved in 10 ml of anhydrous ethanol. The resulting solution was treated with 75 ml of HCl in dioxane (4 M) and stirred at ambient temperature for 1 h. Concentration of the solution in vacuo gave the desired compound as an off-white foam.

## Example 66

### Boc-Leu-Asn Amide of (3S,5S,1′R,2′S)-5-(2-Amino-3-cyclohexyl-1-hydroxypropyl)-3-methyldihydrofuran-2(3H)-one.

A solution of 0.20 g (0.69 mmol) of the resultant compound of Example 65, 0.24 g (2.1 mmol) of 1-hydroxybenzotriazole and 0.15 ml (1.4 mmol) of 4-methylmorpholine in 5 ml of dimethylformamide was cooled under $N_2$ atmosphere to -23°C and treated with 0.13 g (0.69 mmol) of N-ethyl-N′-(dimethylaminopropyl)carbodiimide. The resulting solution was stirred for 16 h during which the temperature slowly warmed to ambient temperature. The solvent was removed in vacuo at 30°C, and the residue was partitioned between ethyl acetate and aqueous $NaHCO_3$. The organic layer was washed with saturated brine, dried over $MgSO_4$, and concentrated. Chromatography on silica gel using 5% methanol in chloroform provided 0.30 g (75%) of the desired compound. $^1H$ NMR ($CDCl_3$) δ0.7-1.9 (br envelope), 0.95 (d,J=7Hz,3H), 0.97 (d,J=7Hz,3H), 1.27 (d,J=7Hz,3H), 1.47 (s,9H), 2.51 (ddd,J=13,9,6Hz,1H), 2.6-2.7 (m,2H), 2.40 (dd,J=15,5Hz,1H), 3.55 (m,1H), 3.70 (m,1H), 3.83 (m,1H), 3.97 (m,1H), 4.19 (m,1H), 4.29 (ddd,J=10,7,6Hz,1H), 4.64 (m,1H), 5.17 (br d,1H), 5.65 (br,1H), 6.64 (br,1H), 7.12 (br d,1H), 8.17 (br d,1H). Mass spectrum: $(M+H)^+$ = 583.
Anal. Calcd. for $C_{29}H_{50}N_4O_8$•0.75 $H_2O$: C, 58.42; H, 8.71; N, 9.40.
Found: C, 58.26; H, 8.43; N, 9.62.

## Example 67

### Boc-Leu-Asn Amide of (4S,5S)-N-Methyl-5-amino-4-hydroxy-6-phenylhex-1-ene-2-carboxamide.

Using the procedures of Examples 65 and 66 but replacing the resultant compound of Example 106 with (4S,5S)-N-methyl-4-hydroxy-5-(t-butyloxycarbonylamino)-6-phenylhex-1-ene-2-carboxamide (J. Org. Chem. **1986**, 51, 3921) provided the desired compound in 43% yield after chromatography on silica gel using 8% methanol in chloroform as eluent. $^1H$ NMR ($d_6$-DMSO) δ 0.84 (d,J=7Hz,3H), 0.87 (d,J=7Hz,3H), 1.3-1.5 (m,2H), 1.38 (s,9H), 1.60 (m,1H), 2.23 (m,2H), 2.39 (dd,J=16,7Hz,1H), 2.56 (d,J=5Hz,3H), 2.61 (dd,J=14,7Hz,1H), 2.76 (dd,J=14,7Hz,1H), 3.49 (m,1H), 3.86 (br q,1H), 3.96 (m,1H), 4.51 (br q,1H), 5.08 (d,J=5Hz,1H), 5.27 (s,1H), 5.58 (s,1H), 6.93 (br,1H), 6.98 (br d,J=8Hz,1H), 7.1-7.4 (m,6H), 7.89 (br q,J=5Hz,1H), 8.06 (br d,J=8Hz,1H). Mass spectrum: $(M+H)^+$ = 576.

## Example 68

### Cbz-Leu-Asn-OH

Cbz-Leu-OH (19.8 g, 75 mmol) was coupled to asparagine (9.9 g, 75 mmol) using the mixed anhydride procedure described in Example 3. After extraction into chloroform and washing with water, the product formed a white precipitate in the organic layer. The mixture was filtered, and the residue was washed with chloroform and dried in vacuo at 50°C to provide 10.37 g (37%) of the desired compound. $^1H$ NMR ($d_6$-

DMSO) δ 0.84 (d,J=7Hz,3H), 0.87 (d,J=7Hz,3H), 1.43 (m,2H), 1.62 (heptet,J=7Hz,1H), 3.18 (m,2H), 4.06 (br q,1H), 4.49 (br q,1H), 5.02 (AA′,2H), 6.91 (br,1H), 7.3-7.4 (m,5H), 7.44 (br d,1H), 8.08 (br d,1H), 12.5 (br,1H). Mass spectrum: $(M+H)^+$ = 380.

Anal. Calcd. for $C_{18}H_{25}N_3O_6 \cdot 0.25\ H_2O$: C, 56.31; H, 6.69; N, 10.95.

Found: C, 56.26; N, 6.65; N, 11.05.

## Example 69

### Cbz-Leu-Asn Amide of N-(3-Methylbutyl)-2-(2(S)-amino-1(R)-hydroxy-3-phenylpropyl)furan-3-carboxamide.

The resultant 1′(R),2′(S)-diastereomer of Example 102 was deprotected according to the procedure of Example 2 and coupled to Cbz-Leu-Asn-OH according to the procedure of Example 66 to provide, after silica gel chromatography using 7.5% methanol in chloroform, the desired compound in 87% yield. $^1H$ NMR ($d_6$-DMSO) δ 0.8-0.93 (m,12H), 1.39 (m,4H), 1.60 (m,2H), 2.2-2.8 (br envelope), 3.2-3.5 (br envelope), 4.02 (m,1H), 4.23 (m,1H), 4.48 (m,1H), 5.0-5.1 (m,3H), 6.42 (br t,J=6Hz,1H), 6.35 (d,J=4Hz,1H), 6.94 (br,1H), 7.1-7.5 (br envelope), 7.57 (d,J=4Hz,1H), 7.79 (m,1H), 8.00 (m,1H), 8.22 (m,1H). Mass spectrum: $(M+H)^+$ = 692.

Anal. Calcd. for $C_{37}H_{49}N_5O_8 \cdot 0.5\ H_2O$: C, 63.41; H, 7.19; N, 9.99. Found: C, 63.13; H, 7.08; N, 10.01.

## Example 70

### Boc-Leu-Asn Amide of N-(1-Hydroxy-3-methylpent-2-yl)-2-(2-amino-1-hydroxy-3-phenylpropyl)thiophene-3-carboxamide.

The resultant compound of Example 104 is deprotected according to the procedure of Example 2 and coupled to Boc-Leu-Asn-OH according to the procedure of Example 66 to provide the desired compound.

## Example 71

### Boc-Leu-Asn Amide of (5S,1′R,2′S)-5-(2-Amino-3-cyclohexyl-1-hydroxypropyl)-3-methylenedihydrofuran-2(4H)-one.

The resultant compound of Example 105 is deprotected according to the procedure of Example 65 and coupled to Boc-Leu-Asn-OH according to the procedure of Example 66 to provide the desired compound.

## Example 72

### Boc-Leu-Asn Amide of (2S,3R,4S,6S)-2-Amino-1-cyclohexyl-6-methyl-3,4,7-trihydroxyheptane.

The resultant compound of Example 107 is deprotected according to the procedure of Example 65 and coupled to Boc-Leu-Asn-OH according to the procedure of Example 66 to provide the desired compound.

Example 73

Boc-Leu-Asn Amide of (5S,1'R,2'S)-5-(2-Amino-3-cyclohexyl-1-hydroxypropyl)-3,3-dimethyldihydrofuran-2(3H)-one.

The resultant compound of Example 169 is deprotected according to the procedure of Example 65 and coupled to Boc-Leu-Asn-OH according to the procedure of Example 66 to provide the desired compound.

Example 74

Boc-Leu-Asn Amide of (2S,3R,4S)-2-Amino-1-cyclohexyl-6,6-dimethyl-3,4,7-trihydroxyheptane.

The resultant compound of Example 170 is deprotected according to the procedure of Example 65 and coupled to Boc-Leu-Asn-OH according to the procedure of Example 66 to provide the desired compound.

Example 75

2N-Boc-Leu-Asn Amide of (2S,3R,4S,6S)-2-Amino-7-(benzylamino)-1-cyclohexyl-3,4-dihydroxy-6-methylheptane.

The resultant compound of Example 109B is deprotected according to the procedure of Example 65 and coupled to Boc-Leu-Asn-OH according to the procedure of Example 66 to provide the desired compound.

Example 76

2N-Boc-Leu-Asn Amide of (2S,3R,4S,6S)-1-Cyclohexyl-2,7-diamino-3,4-dihydroxy-6-methylheptane.

The resultant compound of Example 75 is hydrogenolyzed according to the procedure of Example 23 to provide the desired compound.

Example 77

Cbz-Asn Amide of (2S,4S,1'R,2'S)-2-(2-Amino-3-cyclohexyl-1-hydroxypropyl)-4-methyltetrahydrofuran.

The resultant compound of Example 108 is deprotected according to the procedure of Example 55 and coupled to Cbz-Asn-OH according to the procedure of Example 66 to provide the desired compound.

Example 78

Boc-(6-aminohexanoyl)Leu-Asn Amide of (2S,4S,1'R,2'S)-2-(2-Amino-3-cyclohexyl-1-hydroxypropyl-4-methyltetrahydrofuran.

The resultant compound of Example 77 is hydrogenolyzed according to the procedure of Example 23 and coupled to the resultant compound of Example 23 according to the procedure of Example 4 to provide the desired compound.

Example 79

(6-Aminohexanoyl)Leu-Asn Amide of (2S,4S,1'R,2'S)-2-(2-Amino-3-cyclohexyl-1-hydroxypropyl)-4-methyltetrahydrofuran.

The resultant compound of Example 78 is deprotected according to the procedure of Example 2. The crude product is partitioned between ethyl acetate and aqueous $NaHCO_3$, dried over $MgSO_4$, and concentrated. Chromatography on silica gel provides the desired compound.

Example 80

Cbz-Asn Amide of 2-Amino-1-cyclopentyl-1-hydroxy-3-phenylpropane.

The resultant compound of Example 110 is deprotected according to the procedure of Example 2 and coupled to Cbz-Asn-OH according to the procedure of Example 66 to provide the desired compound.

Example 81

(N-Methyl-N-(2-(N,N-dimethylamino)ethyl)carbamoyl)-Leu-Asn Amide of 2-Amino-1-cyclopentyl-1-hydroxy-3-phenylpropane

The resultant compound of Example 80 is hydrogenolyzed according to the procedure of Example 23 and coupled to the resultant compound of Example 25 according to the procedure of Example 4 to provide the desired compound.

Example 82

2-(((4-Morpholinyl) carbonyl)oxy)-4-methylpentanoyl-Asn Amide of 2-Amino-1-cyclopentyl-1-hydroxy-3-phenylpropane.

The resultant compound of Example 80 is hydrogenolyzed according to the procedure of Example 23

and coupled to the resultant compound of Example 31 according to the procedure of Example 4 to provide the desired compound.

Example 83

((2R)-2-Isobutyl-3-(((4-morpholinyl)carbonyl)amino)propanoyl)-Asn Amide of 2-Amino-1-cyclopentyl-1-hydroxy-3-phenylpropane.

The resultant compound of Example 80 is hydrogenolyzed according to the procedure of Example 23 and coupled to the resultant compound of Example 34 according to the procedure of Example 4 to provide the desired compound.

Example 84

((2R)-2-Isobutyl-3-((ethoxycarbonyl)amino)propanoyl)-Asn Amide of 2-Amino-1-cyclopentyl-1-hydroxy-3-phenylpropane.

The resultant compound of Example 80 is hydrogenolyzed according to the procedure of Example 23 and coupled to the resultant compound of Example 35 according to the procedure of Example 4 to provide the desired compound.

Example 85

Boc-Leu-Asn Amide of N-(3-Methylbutyl)-2-(2-amino-3-cyclohexyl-1-hydroxypropyl)cyclopentanecarboxamide.

The resultant compound of Example 111 is deprotected according to the procedure of Example 65 and coupled to Boc-Leu-Asn-OH according to the procedure of Example 66 to provide the desired compound.

Example 86

Boc-Leu-Asn Amide of (1′R,2′S,5S)-5-(2-Amino-3-cyclohexyl-1-hydroxypropyl)-3-ethyloxazolidin-2-one.

The resultant compound of Example 118 is deprotected according to the procedure of Example 65 and coupled to Boc-Leu-Asn-OH according to the procedure of Example 66 to provide the desired compound.

Example 87

Boc-Leu-Asn Amide of (1′R,2′S,5S)-5-(2-Amino-3-cyclohexyl-1-hydroxypropyl)-3-methoxyoxazolidin-2-one.

The resultant compound of Example 119 is deprotected according to the procedure of Example 65 and coupled to Boc-Leu-Asn-OH according to the procedure of Example 66 to provide the desired compound.

## Example 88

### Boc-Leu-Asn Amide of (1′R,2′S,5S)-5-(2-Amino-3-cyclohexyl-1-hydroxypropyl)-3-(N-benzyloxycarbonyl-N-methylamino)oxazolidin-2-one.

The resultant compound of Example 120 is deprotected according to the procedure of Example 65 and coupled to Boc-Leu-Asn-OH according to the procedure of Example 66 to provide the desired compound.

## Example 89

### Boc-Leu-Asn Amide of (1′R,2′S,5S)-5-(2-Amino-3-cyclohexyl-1-hydroxypropyl)-3-(methylamino)oxazolidin-2-one.

The resultant compound of Example 88 is hydrogenolyzed according to the procedure of Example 23 to provide the desired compound.

## Example 90

### Boc-Leu-Asn Amide of (1′R,2′S,4S)-4-(2-Amino-3-cyclohexyl-1-hydroxypropyl)dioxolan-2-one.

The resultant compound of Example 128 is deprotected according to the procedure of Example 65 and coupled to Boc-Leu-Asn-OH according to the procedure of Example 66 to provide the desired compound.

## Example 91

### Boc-Leu-Asn Amide of (1′R,2′S,5S)-5-(2-Amino-3-cyclohexyl-1-hydroxypropyl)-3-ethylimidazolidin-2-one.

The resultant compound of Example 124 is deprotected according to the procedure of Example 65 and coupled to Boc-Leu-Asn-OH according to the procedure of Example 66 to provide the desired compound.

## Example 92

### Boc-Leu-Asn Amide of (1′R,2′S,6S)-6-(2-Amino-3-cyclohexyl-1-hydroxypropyl)-3-aza-2-oxotetrahydropyran.

The resultant compound of Example 127 is deprotected according to the procedure of Example 65 and coupled to Boc-Leu-Asn-OH according to the procedure of Example 66 to provide the desired compound.

Example 93

Boc-Leu-Asn Amide of (1′R,2′S,4S)-4-(2-Amino-3-cyclohexyl-1-hydroxypropyl)-3-azapiperidin-2-one.

The resultant compound of Example 132 is deprotected according to the procedure of Example 65 and coupled to Boc-Leu-Asn-OH according to the procedure of Example 66 to provide the desired compound.

Example 94

Boc-Leu-Asn Amide of (1′R,2′S,6S)-6-(2-Amino-3-cyclohexyl-1-hydroxypropyl)-3,4-diaza-2-oxo-4-methyltetrahydropyran.

The resultant compound of Example 135 is deprotected according to the procedure of Example 65 and coupled to Boc-Leu-Asn-OH according to the procedure of Example 66 to provide the desired compound.

Example 95

Boc-Leu-Asn Amide of (1′R,2′S,5S)-5-(2-Amino-3-cyclohexyl-1-hydroxypropyl)-3-(hydroxyethyl)oxazolidin-2-one.

The resultant compound of Example 139 is deprotected according to the procedure of Example 65 and coupled to Boc-Leu-Asn-OH according to the procedure of Example 66 to provide the desired compound.

Example 96

Boc-Leu-Asn Amide of (2S,3R,4S)-(2-Amino-1-cyclohexyl-3,4-dihydroxy-6-methylheptane.

The resultant compound of Example 141 is deprotected according to the procedure of Example 65 and coupled to Boc-Leu-Asn-OH according to the procedure of Example 66 to provide the desired compound.

Example 97

Boc-Leu-Asn Amide of 3-Amino-4-cyclohexyl-2-hydroxy-1-(isopropylmercapto)butane.

The resultant compound of Example 144 is deprotected according to the procedure of Example 65 and coupled to Boc-Leu-Asn-OH according to the procedure of Example 66 to provide the desired compound.

Example 98

Boc-Leu-Asn Amide of 3-Amino-4-cyclohexyl-2-hydroxy-1-(isopropylsulfonyl)butane.

The resultant compound of Example 145 is deprotected according to the procedure of Example 65 and coupled to Boc-Leu-Asn-OH according to the procedure of Example 66 to provide the desired compound.

## Example 99

Boc-Leu-Asn Amide of (2S,3R,4S)-4-Amino-1-azido-5-cyclohexyl-2,3-dihydroxypentane.

The resultant compound of Example 149 is deprotected according to the procedure of Example 65 and coupled to Boc-Leu-Asn-OH according to the procedure of Example 66 to provide the desired compound.

## Example 100

Boc-Leu-Asn Amide of 2-(2-Amino-1-hydroxy-3-phenylpropyl)pyrrole.

The resultant compound of Example 150 is deprotected according to the procedure of Example 2 and coupled to Boc-Leu-Asn-OH according to the procedure of Example 66 to provide the desired compound.

## Example 101

N-(3-Methylbutyl)furan-3-carboxamide.

3-Furoic acid (7.07 g, 63 mmol) was coupled to isoamylamine using the mixed anhydride procedure described in Example 4 to give 7.68 g (67%) of the desired compound after recrystallization from hexane. $^1$H NMR ($CDCl_3$) δ0.95 (d,J=7Hz,6H), 1.49 (dt,J=8,7Hz,2H), 1.67 (heptet,J=7Hz,1H), 3.42 (m,2H), 5.73 (br,1H), 6.60 (dd,J=2,1Hz,1H), 7.43 (t,J=2Hz,1H), 7.92 (dd,J=2,1Hz,1H). Mass spectrum: $(M+H)^+$ = 182.
Anal. Calcd. for $C_{10}H_{15}NO_2$: C, 66.27; H, 8.34; N, 7.73.
Found: C, 66.68; H, 8.52; N, 7.73.

## Example 102

N-(3-Methylbutyl)-2-(2-(t-butyloxycarbonylamino)-1-hydroxy-3-phenylpropyl)furan-3-carboxamide.

Using the procedure of Example 1 but replacing N-methyl-1-cyclohexenecarboxamide with the resultant compound of Example 102 gives the desired compound.

## Example 103

N-(1-Hydroxy-3-methylpent-2-yl)thiophene-3-carboxamide.

Thiophene-3-carboxylic acid is coupled to isoleucinol using the mixed anhydride procedure of Example 4 to give the desired compound.

## Example 104

N-(1-Hydroxy-3-methylpent-2-yl)-2-(2-(t-butyloxycarbonylamino)-1-hydroxy-3-phenylpropyl)thiophene-3-carboxamide.

Using the procedure of Example 1 but replacing N-methyl-1-cyclohexenecarboxamide with the resultant compound of Example 103 and using one additional equivalent of n-butyllithium gives the desired compound.

## Example 105

(5S,4'S,5'R)-5-(3-(t-Butyloxycarbonyl)-4-(cyclohexylmethyl)-2,2-dimethyloxazolidin-5-yl)-3-methylenedihydrofuran-2(4H)-one.

A solution of 16.52 g (51 mmol) of the resultant compound of Example 241 in 15 ml of anhydrous tetrahydrofuran was treated with 3.98 g (61 mmol) of freshly activated zinc dust. With vigorous stirring, the mixture was treated with 10 g (56 mmol) of methyl 2-(bromomethyl)acrylate at a rate which maintained the temperature at 50-60 °C. Upon completion of the addition, the mixture was stirred at 50 °C for 1 h. After being allowed to cool, the mixture was poured into 100 ml of cold 1 M HCl and extracted with dichloromethane (3 X 100 ml). The combined organic layers were washed successively with saturated aqueous $NaHCO_3$ and $H_2O$, dried over $Na_2SO_4$, and concentrated. Silica gel chromatography using 9:1 hexane:ethyl acetate provided 10.83 g (61%) of the desired compound. $^1H$ NMR ($CDCl_3$) $\delta$ 0.8-2.0 (br envelope), 1.49 (s,9H), 1.54 (s,3H), 1.57 (s,3H), 2.93 (ddt,J=18,6,3Hz,1H), 3.05 (m,1H), 3.70 (m,1H), 4.07 (m,1H), 4.47 (ddd,J=13,9,6Hz,1H), 5.70 (br t,J=3Hz,1H), 6.28 (t,J=3Hz,1H). Mass spectrum: $(M+H)^+$ = 394.

Anal. Calcd. for $C_{22}H_{35}NO_5$: C, 67.15; H, 8.96; N, 3.56.
Found: C, 67.66; H, 9.11; N, 3.60.

## Example 106

(3S,5S,4'S,5'R)-5-(3-(t-Butyloxycarbonyl)-4-(cyclohexylmethyl)-2,2-dimethyloxazolidin-5-yl)-3-methyldihydrofuran-2(3H)-one.

A mixture of 8.03 g (20 mmol) of the resultant compound of Example 105 and 0.81 g of 10% palladium on carbon in 200 ml of ethyl acetate was shaken under 4 atmospheres of $H_2$. After filtration, concentration of the filtrate gave 7.58 g (94%) of the desired compound. $^1H$ NMR ($CDCl_3$) $\delta$ 0.8-2.0 (br envelope), 1.31 (s,3H), 1.48 (s,9H), 1.54 (s,3H), 1.58 (s,3H), 2.57 (m,1H), 2.68 (m,1H), 3.74 (m,1H), 4.04 (m,1H), 4.31 (ddd,J=13,9,6Hz,1H). Mass spectrum: $(M+H)^+$ = 396.

Example 107

3-(t-Butyloxycarbonyl)-4-(cyclohexylmethyl)-5-(1,4-dihydroxy-3-methylbutyl)-2,2-dimethyloxazolidine.

A mixture of 0.50 g (1.26 mmol) of the resultant compound of Example 106 and 0.15 g (4 mmol) of sodium borohydride in 50 ml of tetrahydrofuran was heated at reflux under $N_2$ atmosphere for 48 h. After being allowed to cool, the mixture was treated cautiously with aqueous $NH_4Cl$, extracted with ether, washed with saturated brine, dried over $MgSO_4$, and concentrated in vacuo. Silica gel chromatography using 2:1 chloroform/ethyl acetate gave 0.37 g (73%) of the desired compound. $^1H$ NMR ($CDCl_3$) δ 0.7-2.0 (br envelope), 0.94 (d,J=7Hz,3H), 1.49 (s,9H), 1.52 (s,3H), 1.55 (s,3H), 3.43 (dd,J=11,8Hz,1H), 3.55-3.7 (m,3H), 4.09 (br d,1H). Mass spectrum: $(M+H)^+$ = 400.

Example 108

3-(t-Butyloxycarbonyl)-4-(cyclohexylmethyl)-2,2-dimethyl-5-(4-methyltetrahydrofuran-2-yl)oxazolidine.

A solution of 51 mg (0.13 mmol) of the resultant compound of Example 107 and 0.037 ml (0.27 mmol) of triethylamine in 2 ml of dichloromethane was cooled to 0° C under $N_2$ atmosphere and treated with 0.012 mol (0.15 mmol) of methanesulfonyl chloride. After 1 h, the solution was diluted with dichloromethane, washed successively with 10% citric acid, water and saturated aqueous $NaHCO_3$, dried over $Na_2SO_4$, and concentrated in vacuo. The crude mesylate thus produced (59 mg) was taken up in 8 ml of dry tetrahydrofuran, treated with 20 mg (0.50 mmol) of sodium hydride (60% dispersion in oil), and heated at reflux for 2 h. After being allowed to cool, the solution was treated cautiously with saturated aqueous $NH_4Cl$, extracted with ether, dried over $MgSO_4$ and concentrated. Silica gel chromatography using 9:1 hexane/ethyl acetate gave 30 mg (75%) of the desired compound. $^1H$ NMR ($CDCl_3$) δ 0.7-2.4 (br envelope), 1.06 (d,J=7Hz,3H), 1.48 (s,9H), 1.52 (s,3H), 1.56 (s,3H), 3.30 (t,J=9Hz,1H), 3.66 (m,1H), 3.9-4.0 (m,3H). Mass spectrum: $(M+H)^+$ = 382.

Example 109

A. (3S,5S,4′S,5′R)-5-(3-(t-Butyloxycarbonyl)-4-(cyclohexylmethyl)-2,2-dimethyloxazolidin-5-yl)-2-hydroxy-3-methyltetrahydrofuran.

A solution of 1.00 g (2.5 mmol) of the resultant compound of Example 106 in 30 ml of anhydrous dichloromethane was cooled under $N_2$ atmosphere to -78° C and treated dropwise with 3.03 ml (3.0 mmol) of precooled diisobutylaluminum hydride (1.0 M in dichloromethane). After 1.5 h, the solution was treated cautiously with 5 ml of methanol, allowed to warm, diluted with 200 ml of ether, washed successively with 1 M HCl and saturated brine, dried over $MgSO_4$, and concentrated. Silica gel chromatography using 5:1 hexane/ ethyl acetate gave 0.95 g (94%) of the desired compound. Mass spectrum: $(M+H)^+$ = 398.

B. (4S,5R,1′S,3′S)-5-(4-(Benzylamino)-1-hydroxy-3-methybutyl)-3-(t-butyloxycarbonyl)-4-(cyclohexylmethyl)-2,2-dimethyloxazolidine.

A solution of the resultant compound of Example 109A (0.32 g, 0.81 mmol), benzylamine (0.13 ml, 1.2 mmol), sodium acetate (0.13 g, 2.0 mmol), and acetic acid (.07 ml, 1.2 mmol) in 25 ml of isopropanol was

treated with 0.10 g (1.6 mmol) of sodium cyanoborohydride. After being stirred at ambient temperature for 16 h, the mixture was partitioned between ethyl acetate and saturated aqueous $NaHCO_3$, and the organic layer was washed with saturated brine, dried over $MgSO_4$, and concentrated. Silica gel chromatography provided 0.33 g (85%) of the desired compound. $^1H$ NMR ($CDCl_3$) δ 0.8-1.8 (br envelope), 0.97 (d,J=7Hz,3H), 1.48 (s,9H), 1.53 (s,6H), 2.02 (m,1H), 2.09 (m,1H), 2.37 (br t,J=11Hz,1H), 2.79 (dd,J=11,2Hz,1H), 3.44 (br t,J=9Hz,1H), 3.55 (m,1H), 3.80 (s,2H), 4.11 (m,1H), 7.3-7.4 (m,5H). Mass spectrum: $(M+H)^+$ = 489.

## Example 110

### 2-(t-Butyloxycarbonylamino)-1-cyclopentyl-1-hydroxy-3-phenylpropane.

A solution of Boc-phenylalininal (1 mmol) in dry tetrahydrofuran is cooled under $N_2$ atmosphere to -78° C and treated dropwise with 1.2 mmol of cyclopentylmagnesium chloride (2.0 M in diethylether). After 2 h, the solution is neutralized, extracted with ether, washed with saturated brine, dried over $MgSO_4$, and concentrated in vacuo. Silica gel chromatography using hexane/ethyl acetate gives the desired compound.

## Example 111

### N-(3-Methylbutyl)-2-(2-(t-butyloxycarbonylamino)-1-hydroxy-3-phenylpropyl)cyclopentane-1-carboxamide.

A mixture of the resultant compound of Example 6 and 10% palladium on carbon in methanol is shaken under four atmospheres of $H_2$ for 4 h. Filtration and concentration of the resulting solution gives the desired compound.

## Example 112

### 4(S)-t-Butyloxycarbonylamino-5-cyclohexyl-3(R,S)-hydroxyl-1-pentene.

To a stirred -78° C solution of Boc-cyclohexylalanine methyl ester (10.2 g, 35.8 mmol) in dry toluene (60 ml) was added diisobutylaluminum hydride (34 ml of a 1.5 M solution in toluene). After 30 min, vinyl magnesium bromide (108 ml of 1 M solution in tetrahydrofuran (THF)) was added. After stirring for 15 h at 0° C, the mixture was carefully quenched with methanol, treated with Rochelle salts (22 ml of saturated aqueous solution in (40 ml $H_2O$), and filtered. After extracting the solids 5 times with ethyl acetate, the extracts and filtrate were combined and the organic phase was washed with brine, dried, filtered and evaporated to an oil (10.2 g). Chromatogrphy on silica gel eluting with hexane/ethyl acetate mixtures provided 6.1 g (60%) of the desired product.
Anal. Calcd. for $C_{16}H_{29}NO_3 \cdot H_2O$: C, 66.8; H, 10.3; N, 4.9.
Found: C, 66.9; H, 10.2; N, 4.7.

## Example 113

### 4(S)-Cyclohexylmethyl-5(R,S)-vinyl-2-oxazolidinone.

The resultant product of Example 112 (2.80 g, 9.88 mmol) in dry dimethylformamide (DMF) (50 ml) was added to a stirred suspension of NaH (593 mg of a 60% dispersion in oil, 14.8 mmol, hexane washed) in dry DMF (50 ml). After 3 h, the mixture was quenched (750 ml water + 100 ml brine) and extracted with ether (5 x 100 ml). The combined organic phase was washed with brine (3 x 50 ml), dried ($MgSO_4$), filtered and evaporated to an oil (2.23 g). The NMR spectrum of the crude product revealed an 82:18 mixture of 5S:5R diastereomers. Silica gel chromatography gave 80% recovery of pure diastereomers. 5S: Mass spectrum: $(M+1)^+$ = 210.

Anal. Calcd. for $C_{12}H_{19}NO_2$: C, 68.9; H, 9.1; N, 6.7.

Found: C, 68.4; H, 9.2; N, 6.5.

5R: Mass spectrum: $(M+1)^+$ = 210.

## Example 114

### (3S,4S)-3-Hydroxy-4-amino-5-cyclohexyl-1-pentene.

To the resultant 5S-diastereomer from Example 113 (2.06 g, 9.84 mmol) in dioxane (180 ml) and water (120 ml) was added barium hydroxide octahydrate (6.24 g, 19.8 mmol). The mixture was refluxed for 18 h, cooled, filtered, concentrated, taken up in water and extracted with ethyl acetate which was dried over $Na_2SO_4$ and evaporated to afford 1.64 g (91%) of the desired product, m.p. 59-61 °C.

Anal. Calcd. for $C_{11}H_{21}NO$: C, 72.08; H, 11.55; N, 7.64.

Found: C, 71.67; H, 11.68; N, 7.36.

## Example 115

### (3S,4S)-3-Hydroxy-4-tert-butyloxycarbonylamino-5-cyclohexyl-1-pentene.

To the resultant compound from Example 114 (1.62 g, 8.84 mmol) in methylene chloride (20 ml) was added di-tert-butyldicarbonate (1.93 g, 8.84 mmol). The mixture was stirred for 14 h, diluted with ethyl acetate, washed sequentially with 0.5 M $H_3PO_4$, saturated $NaHCO_3$ solution and brine, then dried over $Na_2SO_4$ and evaporated to afford 2.51 g (100%) of the desired compound.

## Example 116

### (3S,4S)-3-Methoxyethoxymethoxy-4-tert-butyloxycarbonylamino-5-cyclohexyl-1-pentene.

To the resultant compound from Example 115 (2.51 g, 8.84 mmol) in methylene chloride (20 ml) was added diisopropyl ethylamine (4.60 ml, 26.4 mmol) and methoxyethoxy chloromethane (3.00 ml, 26.3 mmol). After stirring at room temperature for 24 h the mixture was concentrated, diluted with ethyl acetate, washed with 0.5 M $H_3PO_4$, saturated $NaHCO_3$ solution, then brine, dried over $Na_2SO_4$, and evaporated. Chromatography on silica gel with ethyl acetate/hexane mixtures afforded 2.63 g (80%) of the desired product as an oil. EI-MS: $M^+$ = 371.

## Example 117

(2RS,3R,4S)-3-Methoxyethoxymethoxy-4-tert-butyloxycarbonylamino-5-cyclohexyl-1,2-oxopentane.

To the resultant compound from Example 116 (5.41 g, 14.56 mmol) in methylene chloride (50 ml) was added 3-chloroperbenzoic acid (6.28 g). After stirring at room temperature for 60 h the mixture was concentrated, diluted with ethyl acetate, washed with cold 1:1 15% aqueous $Na_2SO_3$ solution/saturated $NaHCO_3$ solution (2 x 200 ml), saturated $NaHCO_3$ solution (3 x 100 ml) then brine (1 x 100 ml), dried over $Na_2SO_4$, and evaporated to afford 4.57 g (81%) product as an oil. EI-MS: $M^+$ = 387.

Example 118

(2′S,1′R,5S)-3-Ethyl-5-(1′-methoxyethoxymethoxy-2′-tert-butyloxycarbonylamino-3′-cyclohexylpropyl)-oxazolidin-2-one.

To the resultant compound from Example 117 (310 mg, 0.80 mmol) in isopropanol (5 ml) was added ethyl amine (200 mg, 4 mmol). The mixture was heated at 70 °C for 48 h, evaporated and dissolved in methylene chloride (5 ml). To this solution was added triethylamine (0.34 ml, 2.4 mmol) and phosgene in toluene (1.0 ml, 1.2 mmol, 12.5% solution). After 2 h the mixture was diluted with ethyl acetate, washed with 0.5 M $H_3PO_4$, saturated $NaHCO_3$ solution then brine, dried over $Na_2SO_4$ and evaporated. Chromatography of the residue on silica gel with 1:1 ethyl acetate/hexane provided 14.3 mg (4%) of the 5R isomer followed by 63.0 mg (17%) of the desired 5S isomer, both as oils.

5S-Isomer: $^1$H NMR ($CDCl_3$,TMS) δ 4.83 (d,1H), 4.80 (d,1H), 4.58 (m,1H), 3.49 (s,3H), 1.43 (s,9H), 1.15 (t,3H).

5R-Isomer: MS $(M+H)^+$ = 459.

Example 119

(2′S,1′R,5S)-3-Methoxy-5-(1′-methoxyethoxymethoxy-2′-tert-butyloxycarbonylamino-3′-cyclohexylpropyl)-oxazolidin-2-one.

Prepared from the resultant compound of Example 117 using the procedure of Example 118 and replacing the ethyl amine with equal parts of methoxylamine hydrochloride and sodium bicarbonate.

Example 120

(2′S,1′R,5S)-3-(Benzyloxycarbonylmethylamino)-5-(1′-methoxyethoxymethoxy)-2′-tert-butyloxycarbonylamino-3′-cyclohexylpropyl)-oxazolidin-2-one.

Prepared from the resultant compound of Example 117 using the procedure of Example 118 and replacing the ethyl amine with one equivalent of 1-methyl-1-benzyloxycarbonylhydrazine.

Example 121

(2RS,3R,4S)-1,2-Dihydroxy-3-methoxyethoxymethoxy-4-tert-butyloxycarbonylamino-5-cyclohexylpentane.

To the resultant compound from Example 116 (1.00 g, 2.69 mmol) in tetrahydrofuran (20 ml) at 0° C was added osmium tetroxide (0.75 ml of a 2.5% solution in tert-butanol) and N-methylmorpholine N-oxide (347 mg, 2.95 mmol). The mixture was stirred at room temperature 16 h, diluted with ethyl acetate, washed with $NaHSO_3$ solution, saturated $NaHCO_3$ solution and brine, then dried over $Na_2SO_4$ and evaporated. Chromatography of the residue on silica gel with methanol/methylene chloride mixtures provided 887 mg (81%) of the desired product.
Anal. Calcd. for $C_{20}H_{39}NO_7 \cdot 0.3\ H_2O$: C, 58.46; H, 9.71; N, 3.41.
Found: C, 58.69; H, 9.53; N, 3.41.

## Example 122

(2R,3R,4S)-1-Benzyloxycarbonylethylamino-2-hydroxy-3-methoxyethoxymethoxy-4-tert-butyloxycarbonylamino-5-cyclohexylpentane.

Using the procedure of Example 118 with the resultant compound from Example 117 and replacing the phosgene with benzyl chloroformate provided the desired 2R isomer proceeded by the 2S isomer.
2R-Isomer: $^1H$ NMR ($CDCl_3$,TMS) $\delta$ 7.34 (m,5H), 5.13 (s,2H), 4.95 (d,1H), 4.79 (m,2H), 3.37 (s,3H), 1.43 (s,9H), 1.14 (m,3H).
2S-Isomer: $^1H$ NMR ($CDCl_3$,TMS) $\delta$ 7.35 (m,5H), 5.14 (d,1H), 5.12 (d,1H), 4.93 (d,1H), 4.80 (m,2H), 3.38 (s,3H), 1.43 (s,9H), 1.13 (t,3H).

## Example 123

(2S,3R,4S)-1-Benzyloxycarbonylethylamino-2-azido-3-methoxyethoxymethoxy-4-tert-butyloxycarbonylamino-5-cyclohexylpentane.

To triphenylphosphine (100.0 mg, 0.381 mmol) in tetrahydrofuran (THF, 0.6 ml) at -78° C was added diethyl azodicarboxylate (60 )l, 0.38 mmol) in THF (1 ml). To this mixture was added a solution of hydrazoic acid (0.46 mmol) in benzene (1 ml) then the resultant compound from Example 122 (180.0 mg, 0.318 mmol) in THF (1.4 ml) was added. After one hour the mixture was warmed to room temperature, stirred for 16 h, evaporated and chromatographed on silica gel with 20% ethyl acetate in hexane to afford 103.5 mg (55%) of the desired product as an oil. $^1H$ NMR ($CDCl_3$,TMS) $\delta$ 7.35 (m,5H), 5.15 (m,2H), 3.38 (s,3H), 1.45 (s,9H), 1.15 (m,3H).

## Example 124

(2′S,1′R,5S)-3-Ethyl-5-(1′-methoxyethoxymethoxy-2′-tert-butyloxycarbonylamino-3′-cyclohexylpropyl)-imidazolidin-2-one.

To the resultant compound from Example 123 (99.0 mg, 0.167 mmol) in methanol (2 ml) was added

triethylamine (75 µl, 0.54 mmol) and propane 1,3-dithiol (0.05 ml, 0.50 mmol). After 72 h the mixture was filtered and evaporated, and the crude amino compound was dissolved in toluene (5 ml) and heated to reflux for 72 h. Evaporation and chromatography on silica gel with ethyl acetate/hexane mixtures provided the desired product as an oil.

## Example 125

(3S,4R,5S)-3-Hydroxy-4-methoxyethoxymethoxy-5-tert-butyloxycarbonylamino-6-cyclohexylhexane nitrile.

To the resultant compound from Example 117 (627.0 mg, 1.62 mmol) in methanol (9 ml) was added potassium cyanide (320 mg, 4.91 mmol) in water (3 ml). The mixture was stirred for 32 h, concentrated, diluted with ethyl acetate, washed with water and brine, then dried over $Na_2SO_4$ and evaporated. Chromatography on silica gel with 2:1 hexane/ethyl acetate afforded 209 mg (31%) of the desired 3S-isomer as a solid followed by 150 mg (22%) of the 3R-isomer.
3S-Isomer: Anal. Calcd. for $C_{21}H_{38}N_2O_6$: C, 60.85; H, 9.24; N, 6.76.
Found: C, 60.76; H, 9.22; N, 6.72.
3R-Isomer: $^1H$ NMR ($CDCl_3$, TMS) $\delta$ 4.92 (d,1H), 4.75 (d,1H), 4.69 (d,1H), 3.40 (s,3H), 2.80 (d,2H), 1.45 (s,9H).

## Example 126

(3S,4R,5S)-1-Amino-3-hydroxy-4-methoxyethoxymethoxy-5-tert-butyloxycarbonylamino-6-cyclohexylhexane.

The resultant compound from Example 125 in 5:1 methanol/ammonia was treated with Raney-Nickel and stirred under 4 atmospheres of hydrogen for 18 h. The mixture was evaporated, dissolved in ethyl acetate, filtered, and evaporated to provide the desired product as an oil. $^1H$ NMR ($CDCl_3$,TMS) $\delta$ 4.97 (d,1H), 4.80 (d,1H), 4.76 (d,1H), 4.03 (m,1H), 3.90 (s,3H), 1.43 (s,9H).

## Example 127

(6S,1′R,2′S)-3-Aza-2-oxo-6-(1′-methoxyethoxymethoxy-2′-tert-butyloxycarbonylamino-3′-cyclchexylpropyl)-tetrahydropyran.

The resultant compound from Example 126 (59.0 mg, 0.141 mmol) in methylene chloride (2 ml) at 0° C was treated with triethylamine (100 µl, 0.71 mmol) then phosgene in toluene (0.22 mmol in 180 µl). The mixture was stirred at 0° C for 1 h, then at room temperature for 3h, poured into ethyl acetate, washed with 0.5 M $H_3PO_4$, saturated $NaHCO_3$ solution and brine, then dried over $Na_2SO_4$ and evaporated to afford 53.6 mg (86%) of the desired product as an oil. $^1H$ NMR ($CDCl_3$,TMS) $\delta$ 5.09 (m,1H), 4.90 (d,1H), 4.85 (s,2H), 4.35 (m,1H), 3.40 (s,3H), 1.47 (s,9H).

## Example 128

(2′S,1′R,4S)-2-Oxo-4-(1′-methoxyethoxymethoxy-2′-tert-butyloxycarbonylamino-3′-cyclohexylpropyl)-dioxolane.

The resultant compound of Example 121 was treated according to the procedure of Example 127 to provide the desired product as an oil.

Example 129

(3R,4R,5S)-1-Amino-3-hydroxy-4-methoxyethoxymethoxy-5-tert-butyloxycarbonylamino-6-cyclohexylhexane.

The resultant 3R-isomer from Example 125 was treated according to the procedure for Example 126 to provide the desired product.

Example 130

(3R,4R,5S)-1-Benzlyloxycarbonylamino-3-hydroxy-4-methoxyethoxymethoxy-5-tert-butyloxycarbonylamino-6-cyclohexylhexane.

The resultant compound from Example 129 was treated according to the procedure for Example 127 except that the phosgene in toluene was replaced with benzyl chloroformate to provide the desired product.

Example 131

(3S,4R,5S)-1-Benzyloxycarbonylamino-3-azido-4-methoxyethoxymethoxy-5-tert-butyloxycarbonylamino-6-cyclohoxylpentane.

The resultant compound from Example 130 was treated according to the procedure of Example 123 to provide the desired product.

Example 132

(4S,1′R,2′S)-3-Aza-4-(1′-methoxyethoxymethoxy-2′-tert-butyloxycarbonylamino-3′-cyclohexylpropyl)-piperidin-2-one.

The resultant compound from Example 131 was treated according to the procedure of Example 124 to provide the desired product.

Example 133

(2S,3R,4S)-1-(Benzyloxycarbonylamino)methylamino-2-hydroxy-3-methoxyethoxymethoxy-4-tert-butyloxycarbonylamino-5-cyclohexylpentane.

Prepared from the resultant compound of Example 117 using the procedure of Example 118 with the exceptions that the ethyl amine was replaced with one equivalent of 1-methyl-2-benzyloxycarbonylhydrazine and that the phosgene step was omitted. The desired product was isolated by chromatography on silica gel.

Example 134

(2S,3R,4S)-1-(Amino)methylamino-2-hydroxy-3-methoxyethoxymethoxy-4-tert-butyloxycarbonylamino-5-cyclohexylpentane.

The resultant compound from Example 133 and an equal weight of 10% palladium on carbon in methanol were stirred under a hydrogen atmosphere for 6 h. The mixture was filtered and evaporated to provide the desired product.

Example 135

(6S,1′R,2′S)-3,4-Diaza-2-oxo-4-methyl-6-(1′-methoxyethoxymethoxy-2′-tert-butyloxycarbonylamino-3′-cyclohexylpropyl)tetrahydropyran.

The resultant compound from Example 134 was treated according to the procedure of Example 118 to provide the desired product.

Example 136

N-Benzyloxycarbonyl-2-aminoethanol.

To ethanolamine (9.0 ml, 149 mmol) in methylene chloride (100 ml) at 0° C was added benzyl chloroformate (10.0 ml, 70 mmol). The mixture was stirred at 0° C for 30 min, then at room temperature for 1 h, poured into ethyl acetate, washed with 2 M HCl, saturated $NaHCO_3$ solution, and brine, then dried over $Na_2SO_4$ and evaporated to provide 12.91 g (94%) of the desired compound as a white solid. $^1H$ NMR ($CDCl_3$,TMS) δ 7.47 (m,5H), 5.11 (s,2H), 3.73 (m,2H), 3.38 (m,2H).

Example 137

1-Methoxymethoxy-2-benzyloxycarbonylaminoethane.

To the resultant compound from Example 136 (12.91 g, 66.1 mmol) in methylene chloride (100 ml) was added diisopropylethylamine (24.0 ml, 138 mmol) and chloromethylmethylether (10.0 ml, 132 mmol). After 4 h the mixture was evaporated, dissolved in ethyl acetate, washed with 0.5 M $H_3PO_4$, saturated $NaHCO_3$ solution, and brine, then dried over $Na_2SO_4$, and evaporated to afford 15.27 g (97%) of the desired product

as an oil. $^1$H NMR ($CDCl_3$,TMS) δ 7.47 (m,5H), 5.12 (s,2H), 4.62 (s,2H), 3.62 (m,2H), 3.42 (m,2H), 3.35 (s,3H).

## Example 138

### 1-Methoxymethoxy-2-aminoethane.

The resultant compound from Example 137 (7.60 g, 31.2 mmol) and 10% palladium on carbon (3 g) in methanol (60 ml) were stirred under a hydrogen atmosphere for 24 h. The mixture was filtered, evaporated and distilled to afford 2.02 g (60%) of the desired product as an oil. b.p. 60-70 °C, 45 mm; $^1$H NMR ($CDCl_3$,TMS) δ 4.65 (s,2H), 3.56 (t,3H), 3.37 (s,3H), 2.88 (t,3H).

## Example 139

### (2′S,1′R,5S)-3-(2-Methoxymethoxyethyl)-5′-(1′-methoxyethoxymethoxy-2′-tert-butyloxycarbonylamino-3′-cyclohexylpropyl)-oxazolidin-2-one.

The resultant compound from Example 117 was treated accordingly to the procedure for Example 118 except that the ethyl amine was replaced with three equivalents of the resultant compound from Example 138 to provide the desired product as an oil.

## Example 140

### 2(S)-t-Butyloxycarbonylamino-1-cyclohexyl-6-methylhept-3-ene.

To a stirred -78 °C solution of Boc-cyclohexylalanine methyl ester (40 g, 140 mmol) in anhydrous toluene (250 ml) was added diisobutylaluminum hydride (130 M%, 1.5 M solution in toluene, 121.4 ml) at a rate to keep the internal temperature below -60 °C. After stirring for an additional 20 minutes at -78 °C, the aldehyde solution is used immediately as described below.

To a potassium hydride (35% dispersion in oil, 32.09 g) suspension in a 0 °C mixture of anhydrous THF/DMSO (1000 ml/200 ml) under dry $N_2$ was added 1,1,1,3,3,3-hexamethyldisilazane (209 M%, 49.07 g) dropwise. After stirring at 0 °C for 1 hour, the resulting solution was added via cannula to a 0 °C flask containing isopentyltriphenylphosphonium bromide (209 M%, 125.66 g). The mixture was stirred vigorously for 1 hour at which time it was cooled to -78 °C. The -78 °C aldehyde solution prepared above was then added via cannula. After stirring at -78 °C for 15 minutes, the mixture was allowed to slowly warm to room temperature and then heated to 40 °C for 12 hours. The mixture was then cooled to room temperature and quenched with methanol (7.65 ml) followed by aqueous Rochelle salts (100 ml saturated solution and 500 ml $H_2O$). The mixture was then extracted with ethyl acetate (2x). The combined extracts were washed with water and brine. Drying ($MgSO_4$) and evaporating provided crude alkene which was chromatographed on silica gel (ether/hexane) to give 16.5 g (38%) of the desired compound as an 85:15 mixture of cis:trans isomers. Mp = 53-55 °C. Mass spectrum: $M^+$ = 309.

Anal. Calcd. for $C_{19}H_{35}NO_2$: C, 73.7; H, 11.4; N, 4.5.
Found: C, 73.8; H, 11.4; N, 4.5.

## Example 141

2(S)-t-Butyloxycarbonylamino-1-cyclohexyl-3,4-dihydroxy-6-methylheptane: The 3(R)4(S), 3(S)4(S), 3(R)4(R), and 3(S)4(R) Diastereomers.

To a solution of the resultant compound of Example 140 (8.50, 27.5 mmol) in dry THF (150 ml) were added $OsO_4$ (2.8 ml of a 2.5% solution in t-butanol and N-methylmorpholine N-oxide (9.28 g, 68.7 mmol). After 4 days the mixture was partitioned between ether (200 ml) and brine (100 ml). The aqueous layer was back-extracted with ether (2 x 100 ml), and the combined organic phase was washed with 10% $Na_2SO_3$, 0.1 M $H_3PO_4$, and brine. Drying ($MgSO_4$) and evaporating provided a residue (10.81 g) which was chromatographed on silica gel to elute a 60% yield of the 4 diols in the following order.
3(R),4(S) Mass spectrum: $(M+H)^+$ = 344.
Anal Calcd. for $C_{19}H_{37}NO_4$: C, 66.4; H, 10.9; N, 4.1.
Found: C, 66.4; H, 10.8; N, 3.9.
3(S),4(S) Mass spectrum: $(M+H)^+$ = 344.
Anal Calcd. for $C_{19}H_{37}NO_4$: C, 66.4; H, 10.9; N, 5.1.
Found: C, 66.4; H, 11.1; N, 4.0.
3(R),4(R) Mass spectrum: $(M+H)^+$ = 344.
3(S),4(R) Mass spectrum: $(M+H)^+$ = 344.
Anal Calcd. for $C_{19}H_{37}NO_4$: C, 66.4; H, 10.9; N, 4.1.
Found: C, 66.0; H, 10.7; N, 4.0.

## Example 142

2-t-Butyloxycarbonylamino-1-cyclohexylbut-3-ene.

To a stirred suspension of methyltriphenyl phosphonium bromide (10.97 g, 30.70 mmol) in anhydrous tetrahydrofuran (200 ml) at -78°C (dry ice/acetone bath) under an argon atmosphere, was added n-butyllithium (19.8 ml of a 1.55 M hexane solution) dropwise over the course of 5 min. After 10 min, the -78°C bath was replaced with a 0°C bath for .5 h, at which time the resulting orange solution was cooled again to -78°C. The solution was then added dropwise by cannula to a stirred -78°C solution of Boc-cyclohexylalaninal (27.91 mmol) in anhydrous tetrahydrofuran (30 ml) over the course of .5 h. The mixture was then allowed to warm to room temperature during a 3 h period after which water (150 ml) was added. Extraction with hexane (4 x 100 ml) provided a combined organic phase which was washed with brine (100 ml), dried ($Na_2SO_4$), and concentrated. Chromatography with ether/hexane (1/9) provided the desired compound. Mass spectrum: $(M+H)^+$ = 254.

## Example 143

3-t-Butyloxycarbonylamino-4-cyclohexyl-1,2-oxobutane.

To a stirred solution of the resultant compound of Example 142 (2.0 mmol) in dichloromethane (20 ml) was added m-chloroperbenzoic acid (MCPBA, 1.51 g of 80% MCPBA, 7.0 mmol). After 68 h the reaction mixture was cooled to 0°C, and 0°C 10% $Na_2SO_3$ (5 ml) was added with stirring. After 15 min, the solid was filtered off and extracted with dichloromethane. The combined organic phase was washed sequentially with 0°C 10% $Na_2SO_3$ (6 ml), saturated $NaHCO_3$ (2 x 6 ml), and water (5 ml). Drying ($MgSO_4$), filtering, concentrating and chromatography on 50 g of $SiO_2$ (hexane/ether, 3/1) gave the desired compound. Mass spectrum: $(M+H)^+$ = 270.

Example 144

3-t-Butyloxycarbonylamino-4-cyclohexyl-2-hydroxy-1-isopropylmercaptobutane.

To a stirred solution of the resultant compound of Example 143 (0.87 mmol) in methanol (8.7 ml) was added isopropyl mercaptan (0.87 mmol) and triethylamine (0.87 mmol). The resultant solution was refluxed for 2 h and then evaporated to give a residue which was chromatographed on 15 g of 40 ) $SiO_2$ (7/3, hexane/ether) to give the desired compound. Mass spectrum: $(M+H)^+$ = 346.

Example 145

3-t-Butyloxycarbonylamino-4-cyclohexyl-2-hydroxy-1-isopropylsulfonylbutane.

Treating the resultant compound of Example 144 with 2.5 equivalents of 3-chloroperoxybenzoic acid in dichloromethane, gave the desired compound after work-up as described in Example 143. Mass spectrum: $(M+H)^+$ = 418. Anal Calcd. for $C_{21}H_{33}NO_5S \cdot 0.5\ H_2O$: C, 59.10; H, 9.45; N, 3.28.
Found: C, 58.90; H, 9.46; N, 3.03.

Example 146

(3S,4S)-3-tert-Butyldimethylsilyloxy-4-tert-butoxycarbonylamino-5-cyclohexyl-1-pentene.

To the resultant compound from Example 115 (0.264 g, 0.932 mmol) in DMF (4 ml) was added tert-butyldimethylsilyl chloride (0.300 g, 1.99 mmol) and imidazole (0.269 g, 3.95 mmol). The mixture was stirred at room temperature for 12 hours, poured into ethyl acetate and washed sequentially with 0.5 M $H_3PO_4$, saturated $NaHCO_3$ solution and brine, then dried over $Na_2SO_4$ and evaporated to afford 0.355 g (96%) of the desired compound. Mass spectrum: $(M+H)^+$ = 398.

Example 147

(2RS,3R,4S)-3-tert-Butyldimethylsilyloxy-4-tert-butoxycarbonylamino-5-cyclohexyl-1,2-oxopentane.

The resultant compound from Example 146 (0.355 g, 0.893 mmol) in methylene chloride (8 ml) was treated with m-chloroperbenzoic acid (0.758 g, 3.51 mmol) and stirred at ambient temperature for 14 hours. The mixture was concentrated, dissolved in ethyl acetate, washed sequentially with cold 10% aqueous $Na_2SO_3$ solution, saturated $NaHCO_3$ solution and brine, and then dried over $Na_2SO_4$ and evaporated to afford 0.374 g (100%) of the desired compound. Mass spectrum: $(M+H)^+$ = 404.

Example 148

(2RS,3R,4S)-3-Hydroxy-4-tert-butoxycarbonylamino-5-cyclohexyl-1,2-oxopentane.

The resultant compound from Example 147 (2.10 g, 5.07 mmol) was treated with 1 M tetrabutylammonium fluoride in tetrahydrofuran (10 ml). The mixture was stirred at 0 °C for 1 hour, poured into ethyl acetate, washed with water and brine, then dried over $Na_2SO_4$ and evaporated. Chromatography on silica gel (0.5% methanol in chloroform) afforded 1.3 g (74%) of the desired compound. Mass spectrum: $(M+H)^+$ = 300.

Example 149

(2S,3R,4S)-1-Azido-2,3-dihydroxy-4-tert-butoxycarbonylamino-5-cyclohexylpentane.

The resultant compound from Example 148 (1.12 g, 3.74 mmol), ammonium chloride (0.374 g, 6.98 mmol) and sodium azide (0.580 g, 8.92 mmol) were refluxed in methanol (25 ml) for 12 hours. The mixture was concentrated, then taken up in ethyl acetate, washed with water and brine, dried over $Na_2SO_4$ and evaporated. Chromatography on silica gel (20% ether in hexane) afforded 0.461 g (36%) of the desired compound followed by 0.323 g (25%) of the 4R-isomer. 4S-Diasteriomer: m.p. 93-94 °C. 4R-Diastereomer: mass spectrum: $(M+H)^+$ = 343.

Example 150

1-(t-Butyloxycarbonyl)-2-(2-(t-butyloxycarbonylamino)-1-hydroxy-3-phenylpropyl)pyrrole.

A solution of 1-Boc-2-lithiopyrrole (J. Org. Chem. **1981**, 46, 157) is cooled to -78 °C and treated with Boc-phenylalaninal (Chem. Pharm. Bull. **1982**, 30, 1921). After 0.5 h, the solution is treated with saturated $NH_4Cl$, extracted with ether, dried over $MgSO_4$, and concentrated to the crude product which is purified by silica gel chromatography to give the desired compound.

Example 151

5-(2-(t-Butyloxycarbonylamino)-1-hydroxy-3-phenylpropyl)-1-methylpyrrazole.

Using the procedure of Example 150, 5-lithio-1-methylpyrrazole (Justus Liebigs Ann. Chem. **1959**, 625, 55) is condensed with Boc-phenylalaninal to give the desired compound.

Example 152

2-(2-(t-Butyloxycarbonylamino)-1-hydroxy-3-phenylpropyl)-1-methylimidazole.

Using the procedure of Example 150, 2-lithio-1-methylimidazole (J. Org. Chem. **1973**, 38, 3762) is condensed with Boc-phenylalaninal to give the desired compound.

Example 153

5-(2-(t-Butyloxycarbonylamino)-1-hydroxy-3-phenylpropyl)-1-methyltetrazole.

Using the procedure of Example 150, 5-lithio-1-methyltetrazole (Can. J. Chem. **1971**, 49, 2139) is condensed with Boc-phenylalaninal to give the desired compound.

Example 154

2-(2-(t-Butyloxycarbonylamino)-1-hydroxy-3-phenylpropyl)-4,5-dihydrofuran.

Using the procedure of Example 150, 2-lithio-4,5-dihydrofuran (Tetrahedron Lett. **1977**, 4187) is condensed with Boc-phenylalaninal to give the desired compound.

Example 155

6-(2-(t-Butyloxycarbonylamino)-1-hydroxy-3-phenylpropyl)-3,4-dihydro(2H)pyran.

Using the procedure of Example 150, 6-lithio-3,4-dihydro(2H)pyran (Acad. Sci., Paris, Ser. C, **1977**, 284, 281) is condensed with Boc-phenylalaninal to give the desired compound.

Example 156

2-(2-(t-Butyloxycarbonylamino)-1-hydroxy-3-phenylpropyl)-4,4-dimethyloxazoline.

Using the procedure of Example 150, 2-lithio-4,4-dimethyloxazoline (J. Amer. Chem. Soc. **1970**, 92, 6676) is condensed with Boc-phenylalaninal to give the desired compound.

Example 157

5-(2-(t-Butyloxycarbonylamino)-1-hydroxy-3-phenylpropyl)isothiazole-4-carboxylic Acid.

Using the procedure of Example 150, lithium 5-lithioisothiazole-4-carboxylate (J. Chem. Soc. **1964**, 446) is condensed with Boc-phenylalaninal to give the desired compound.

Example 158

2-(2-(t-Butyloxycarbonylamino)-1-hydroxy-3-phenylpropyl)thiazole.

Using the procedure of Example 150, 2-lithiothiazole (Bull. Soc. Chim. Fr. **1962**, 2072) is condensed with Boc-phenylalaninal to give the desired compound.

Example 159

2-(2-(t-Butyloxycarbonylamino)-1-hydroxy-3-phenylpropyl)tetrahydrofuran

Using the procedure of Example 106, the resultant compound of Example 154 is hydrogenated to give the desired compound.

Example 160

2-(2-(t-Butyloxycarbonylamino)-1-hydroxy-3-phenylpropyl)tetrahydro(2H)pyran.

Using the procedure of Example 106, the resultant compound of Example 155 is hydrogenated to give the desired compound.

Example 161

2-(2-(t-Butyloxycarbonylamino)-1-hydroxy-4-methylpentyl)benzothiazole.

Using the procedure of Example 150, 2-lithiobenzothiazole (J. Organomet. Chem. **1974**, 82, 209) is condensed with Boc-leucinal to give the desired compound.

Example 162

(3S,4S)-4-t-Butyloxycarbonylamino-3-hydroxy-6-methyl-1-heptene.

To a rapidly stirred -78° C solution of Boc-leucinal (1.5 g, 6.97 mmol) in anhydrous tetrahydrofuran (THF) (10 ml) was added a -78° C solution of vinyl magnesium bromide (7 mmol) in anhydrous THF (40 ml) dropwise over the course of 15 min; 2 h later the mixture was acidified to pH 7. The organic phase was separated, washed with brine (2 x 10 ml) and dried ($Na_2SO_4$). Filtration and evaporation provided an oil which was purified by flash chromatography on silica gel with 7/3 hexane-ether. There was obtained a 53% yield of product, mp 57-59° C.
Anal. Calcd. for $C_{13}H_{25}NO_3$: C, 64.17; H, 10.36; N, 5.76.
Found: C, 64.19; H, 10.13; N, 5.66.

Example 163

4(S)-Isobutyl-5(S)-vinyl-2-oxazolidinone.

To a solution of (3S,4S)-4-t-butyloxycarbonylamino-3-hydroxy-6-methyl-1-heptane (10.5 g, 0.043 mol) in dimethylformamide (DMF) (100 ml) was added sodium hydride (2.4 g of 50% dispersion) portionwise at 0-5° C. After stirring for 20 h at room temperature, the reaction mixture was poured into cold aqueous NaCl solution. The resulting mixture was extracted with methylene chloride and the organic phase was washed several times with brine solution. Drying over $MgSO_4$ and evaporation gave a residue which was chromatographed on silica gel eluting with hexane/ethyl acetate mixtures. There was obtained 5.7 g (78%) of the desired compound as an oil. NMR (300 MHz, $CDCl_3$, ppm): 0.9 (2d,6H), 1.35-1.75 (m,3H), 3.6 (m,1H), 4.55 (t,1H), 5.4 (m,2H), 5.9 (m,1H).

Example 164

4(S)-Isobutyl-5(S)-(2-mesyloxyethyl)-2-oxazolidinone.

To a 0° C solution of 4(S)-isobutyl-5(S)-vinyl-2-oxazolidinone (4.7 g, 0.028 mol) in THF (20 ml) was added 9-BBN (9-borabicyclo(3.3.1)nonane, 75 ml, 0.0375 mol in THF) by dropwise addition. After stirring for 5 h at room temperature, the reaction was quenched by the addition of water (1 ml). A solution of NaOH (6.7 g) in water (21 ml) was then added followed by careful addition of $H_2O_2$ (18 ml of 30%). The resulting mixture was heated at 65° C for 1 h, the THF was partially evaporated and the residue was distributed between ethyl acetate and brine solution. The organic phase was washed with brine solution and dried over $MgSO_4$. Evaporation of the solvent gave a residual oil which was chromatographed on silica gel eluting with 5% MeOH in methylene chloride. The pure fractions were combined and evaporated to give 4.62 g of 4(S)-isobutyl-5(S)-(2-hydroxyethyl)-2-oxazolidinone. A solution of this material (3.95 g, 0.021 mol) and triethylamine (3.2 g, 0.032 mol) in methylene chloride (40 ml) was cooled to 0° C and treated by dropwise addition with methanesulfonyl chloride (2.89 g, 0.025 mol). After stirring for 1 h at 0-5° C, the methylene chloride was washed successively with 0.5 N HCl, aqueous $NaHCO_3$ and brine solution. The organic solution was dried and evaporated to a solid product. Recrystallization from hexane/methylene chloride gave 3.9 g (70%) of product, mp 99-100° C.
Anal. Calcd. for $C_{10}H_{19}NO_5S$: C, 45.27; H, 7.22; N, 5.28.
Found: C, 45.38; H, 7.18; N, 5.23.

Example 165

4(S)-Isobutyl-5(S)-(2-(phenethylmercapto)ethyl)-2-oxazolidinone.

To a 0° C solution of 4(S)-isobutyl-5(S)-(2-mesyloxyethyl)-2-oxazolidinone (500 mg, 1.88 mmol) and phenethyl mercaptan (273 mg, 1.98 mmol) in THF (6 ml) was added NaH (95 mg, 1.98 mmol of a 50% dispersion) all at once. The reaction was stirred for 3 h at room temperature and then distributed between methylene chloride and brine solution. The organic layer was washed with brine solution, dried over $MgSO_4$ and evaporated. The residue was chromatographed on silica gel eluting with 65/35 hexane/ethyl acetate to give 570 mg (98%) of the desired product as an oil.
Anal. Calcd. for $C_{17}H_{25}NO_2S$: C, 66.41; H, 8.20; N, 4.56.
Found: C, 66.60; H, 8.32; N, 4.58.

## Example 166

### 4(S)-Isobutyl-5(S)-(2-(phenethylsulfonyl)ethyl)-2-oxazolidinone.

To a solution of 4(S)-isobutyl-5(S)-(2-(phenethylmercapto)ethyl)-2-oxazolidinone (0.49 g, 1.59 mmol) in methylene chloride (6 ml) was added 0.756 g (3.5 mmol) of m-chloroperoxybenzoic acid. After stirring for 1 h at room temperature, the methylene chloride solution was washed successively with aqueous $NaHSO_3$ and aqueous NaOH. The organic layer was dried and evaporated to a solid product.
Trituration with hexane/ether (50/50) gave 495 mg (92%) of product, mp 100-101 °C.
Anal. Calcd. for $C_{17}H_{25}NO_4S$: C, 60.15; H, 7.42; N, 4.13.
Found: C, 60.27; H, 7.42; N, 4.00.

## Example 167

### (3S,4S)-4-Amino-3-hydroxy-6-methyl-1-phenethylmercaptoheptane.

A solution of 4(S)-isobutyl-5(S)-(2-(phenethylmercapto)ethyl)-2-oxazolidinone (0.52 g, 1.69 mmol) and barium hydroxide octahydrate (1.06 g, 3.38 mmol) in dioxane (60 ml) and water (40 ml) was heated at reflux under $N_2$ for 21 h. The solid barium carbonate was filtered and the filtrate was partially evaporated. The residue was diluted with water and the resulting solution was extracted with ether. The organic extract was washed with brine solution, dried over $MgSO_4$ and evaporated to a residue. Trituration with cold hexane gave 365 mg (77%) of product, mp 95-96 °C.
Anal. Calcd. for $C_{16}H_{27}NOS$: C, 68.28; H, 9.67; N, 4.98.
Found: C, 67.99; H, 9.66; N, 4.75.

## Example 168

### (3S,4S)-4-Amino-3-hydroxy-6-methyl-1-phenethylsulfonylheptane.

Using the procedure of Example 167 with the resultant compound of Example 166, gave the desired compound, mp 153-154 °C.
Anal. Calcd. for $C_{16}H_{27}NO_3S \cdot H_2O$: C, 61.31; H, 8.68; N, 4.47.
Found: C, 60.66; H, 8.63; N, 4.19.

## Example 169

### (5S,4′S,5′R)-5-(3-(t-Butyloxycarbonyl)-4-(cyclohexylmethyl)-2,2-dimethyloxazolidin-5-yl)-3,3-dimethyldihydrofuran-2(3H)-one.

A solution of 0.21 ml (1.5 mmol) of dry diisopropylamine in 15 ml of anhydrous tetrahydrofuran was cooled under $N_2$ atmosphere to -78 °C and treated with 0.56 ml (1.4 mmol) of n-butyllithium (2.5 M in hexane). The solution was allowed to warm for 5 min, recooled to -78 °C, and treated with a solution of 0.50 g (1.3 mmol) of the resultant compound of Example 106 in 5 ml of tetrahydrofuran. After addition, the

solution was stirred for 15 min at -78°C, treated with 0.08 ml (1.3 mmol) of iodomethane, and allowed to warm to ambient temperature for 15 min. The mixture was diluted with ether, washed successively with 10% $Na_2S_2O_3$ and saturated brine, dried over $MgSO_4$, and concentrated in vacuo. Chromatography on silica gel using 5.5:1 hexane/ethyl acetate provided 0.46 g (88%) of the desired compound. $^1H$ NMR ($CDCl_3$) δ 0.7-1.9 (br envelope), 1.28 (s,3H), 1.31 (s,3H), 1.49 (s,9H), 1,54 (s,3H), 1.57 (s,3H), 1.97 (dd,J=13,10Hz,1H), 2.22 (m,1H), 3,73 (m,1H), 4.04 (m,1H), 4.36 (br q,1H). Mass spectrum: $(M+H)^+$ = 410.
Anal. Calcd. for $C_{23}H_{39}NO_5$: C, 67.45; N, 9.60; N, 3.42.
Found: C, 67.69; H, 9.64; N, 3.42.

Example 170

3-(t-Butyloxycarbonyl)-4-(cyclohexylmethyl)-5-(1,4-dihydroxy-3,3-dimethylbutyl)-2,2-dimethyloxazolidine.

Using the procedure of Example 107 but replacing the resultant compound of Example 106 with the resultant compound of Example 169 gave, after chromatography on silica gel using 2:1 hexane/ethyl acetate, the desired compound in 77% yield. $^1H$ NMR ($CDCl_3$) δ 0.7-2.0 (br envelope), 0.94 (s,3H), 0.97 (s,3H), 1.49 (s,9H), 1.53 (s,3H), 1.56 (s,3H), 3.42 (d,J=11HZ,1H), 3.49 (d,J=11Hz,1H), 3.56 (m,1H), 3.68 (m,1H), 4.09 (br d,1H). Mass spectrum: $(M+H)^+$ = 414.
Anal. Calcd. for $C_{23}H_{43}NO_5$: C, 66.79; H, 10.48; N, 3.39.
Found: C, 66.05; H, 10.16; N, 3.40.

Example 171

Boc-Gly-Asn Amide of 5-(2-Amino-1-hydroxy-3-phenylpropyl)-1-methylpyrrazole.

The resultant compound of Example 151 is deprotected according to the procedure of Example 2 and coupled to Cbz-asparagine according to the procedure of Example 66. After purification, the resulting compound is hydrogenolyzed according to the procedure of Example 23 and coupled to Boc-glycine according to the mixed anhydride procedure of Example 4 to provide the desired compound.

Example 172

Cbz-Ser-Ile Amide of 2-(2-Amino-1-hydroxy-3-phenylpropyl)-1-methylimidazole.

The resultant compound of Example 152 is deprotected according to the procedure of Example 2 and coupled to Boc-Ile-OH according to the procedure of Example 4. After purification, the resulting compound is deprotected according to the procedure of Example 2 and coupled to Cbz-serine according to the procedure of Example 66 to provide the desired compound.

Example 173

Boc-Leu-Asn Amide of 5-(2-Amino-1-hydroxy-3-phenylpropyl)-1-methyltetrazole.

The resultant compound of Example 153 is deprotected according to the procedure of Example 2 and coupled to Boc-Leu-Asn-OH according to the procedure of Example 66 to provide the desired compound.

Example 174

Boc-Leu-Asn Amide of 2-(2-Amino-1-hydroxy-3-phenylpropyl)-4,5-dihydrofuran.

The resultant compound of Example 154 is deprotected according to the procedure of Example 2 and coupled to Boc-Leu-Asn-OH according to the procedure of Example 66 to provide the desired compound.

Example 175

Boc-Leu-Asn Amide of 6-(2-Amino-1-hydroxy-3-phenylpropyl)-3,4-dihydro(2H)pyran

The resultant compound of Example 155 is deprotected according to the procedure of Example 2 and coupled to Boc-Leu-Asn-OH according to the procedure of Example 66 to provide the desired compound.

Example 176

Boc-Leu-Asn Amide of 2-(2-Amino-1-hydroxy-3-phenylpropyl)-4,4-dimethyloxazoline.

The resultant compound of Example 156 is deprotected according to the procedure of Example 2 and coupled to Boc-Leu-Asn-OH according to the procedure of Example 66 to provide the desired compound.

Example 177

Boc-Leu-Asn Amide of 2-(2-Amino-1-hydroxy-3-phenylpropyl)thiazole.

The resultant compound of Example 158 is deprotected according to the procedure of Example 2 and coupled to Boc-Leu-Asn-OH according to the procedure of Example 66 to provide the desired compound.

Example 178

Boc-Gln-Asn Amide of 2-(2-Amino-1-hydroxy-3-phenylpropyl)tetrahydro(2H)pyran

The resultant compound of Example 160 is deprotected according to the procedure of Example 2 and coupled to Cbz-asparagine according to the procedure of Example 66. After purification, the resulting compound is hydrogenolyzed according to the procedure of Example 23 and coupled to Boc-glutamine

according to the procedure of Example 66 to provide the desired compound.

Example 179

Boc-Leu-Asn Amide of 2-(2-Amino-1-hydroxy-4-methylpentyl)benzothiazole.

The resultant compound of Example 161 is deprotected according to the procedure of Example 2 and coupled to Boc-Leu-Asn-OH according to the procedure of Example 66 to provide the desired compound.

Example 180

Boc-Leu-Asn Amide of (3S,4S)-4-Amino-3-hydroxy-6-methyl-1-(phenethylmercapto)heptane.

The resultant compound of Example 167 is coupled to Boc-Leu-Asn-OH according to the procedure of Example 66 to provide the desired compound.

Example 181

Boc-Leu-Asn Amide of (3S,4S)-4-Amino-3-hydroxy-6-methyl-1-(phenethylsulfonyl)heptane.

The resultant compound of Example 168 is coupled to Boc-Leu-Asn-OH according to the procedure of Example 66 to provide the desired compound.

Example 182

Cbz-Leu-Asn Amide of 2-(2-Amino-1-hydroxy-3-phenylpropyl)tetrahydrofuran.

The resultant compound of Example 159 is deprotected according to the procedure of Example 2 and coupled to Cbz-Leu-Asn-OH according to the procedure of Example 66 to provide the desired compound.

Example 183

(5-(3-(Benzyloxycarbonyl)oxazolidin-5-on-4-yl)pentanoyl)-Leu-Asn Amide of 2-(2-Amino-1-hydroxy-3-phenylpropyl)tetrahydrofuran.

The resultant compound of Example 182 is hydrogenolyzed according to the procedure of Example 23 and coupled to the resultant compound of Example 61 according to the procedure of Example 4 to provide the desired compound.

## Example 184

Boc-Leu-Asn Amide of 5-(2-Amino-1-hydroxy-3-phenylpropyl)isothiazole-4-carboxylic Acid.

The resultant compound of Example 157 is deprotected according to the procedure of Example 2 and coupled to Boc-Leu-Asn-OH according to the procedure of Example 3 to provide the desired compound.

## Example 185

Boc-Leu-Asn Amide of (2S,4S,5S)-N-(3-Methylbutyl)-5-amino-6-cyclohexylhexan-2,4-diol-2-carboxamide.

(2S,4S,5S)-N-(3-Methylbutyl)-5-(t-butyloxycarbonylamino)-6-cyclohexylhexan-2,4-diol-2-carboxamide (J. Med. Chem. **1987**, 30, 1978) is deprotected according to the procedure of Example 2 and coupled to Boc-Leu-Asn-OH according to the procedure of Example 66 to give the desired compound.

## Example 186

Boc-Leu-Asn Amide of (4S,5S)-N-Methyl-5-amino-6-phenylhexan-1,4-diol-2-carboxamide.

(4S,5S)-N-Methyl-5-(t-butyloxycarbonylamino)-6-phenylhexan-1,4-diol-2-carboxamide (J. Org. Chem. **1986**, 51, 3921) was deprotected according to the procedure of Example 2 and coupled to Boc-Leu-Asn-OH according to the procedure of Example 66 to give the desired compound. Mass spectrum: $(M+H)^+ = 576$.

## Example 187

Boc-Leu-Asn Amide of (2S,4S,5S)-N-Isobutyl-5-amino-1-chloro-6-cyclohexylhexan-2,4-diol-2-carboxamide.

(2R,4S,5S)-5-(t-Butyloxycarbonylamino)-6-cyclohexyl-2-(N-isobutylcarboxamido)-1-hexen-4-ol-1,2-oxide (J. Med. Chem. **1987**, 30, 1978) is deprotected according to the procedure of Example 2 and coupled to Boc-Leu-Asn-OH according to the procedure of Example 66 to give the desired compound.

## Example 188

Boc-Leu-Asn Amide of (2R,4S,5S)-N-Isobutyl-5-amino-1-azido-6-cyclohexylhexan-2,4-diol-2-carboxamide.

(2R,4S,5S)-N-Isobutyl-1-azido-5-(t-butyloxycarbonylamino)-6-cyclohexylhexan-2,4-diol-2-carboxamide (J. Med. Chem. **1987**, 30, 1978) is deprotected according to the procedure of Example 2 and coupled to Boc-Leu-Asn-OH according to the procedure of Example 66 to give the desired compound.

Example 189

6-Carboxy-6-(dimethylamino)hexanoyl-Leu-Asn Amide of 2-(2-Amino-1-hydroxy-3-phenylpropyl)tetrahydrofuran.

A mixture of 50 mg of the resultant compound of Example 183, 16 mg of 10% palladium on carbon, 0.45 ml of 37% aqueous formaldehyde and 5.5 mg of sodium acetate in 5 ml of methanol is stirred under a hydrogen atmosphere for 12 h. The resulting mixture is filtered through Celite, diluted with chloroform, dried over $Na_2SO_4$, and concentrated. The crude product is purified by silica gel chromatography to give the desired compound.

Example 190

6-(Benzyloxycarbonyl)amino-6-carboxyhexanoyl-Leu-Asn Amide of 2-(2-Amino-1-hydroxy-3-phenylpropyl)-tetrahydrofuran.

A solution of 0.042 mmol of the resultant compound of Example 183 in 2 ml of tetrahydrofuran and 1 ml of water is treated with 1.9 mg (0.046 mmol) of lithium hydroxide monohydrate. After completion of the reaction, the solution is acidified with HCl, extracted with chloroform, dried over $MgSO_4$, and concentrated to give the desired compound.

Example 191

6-(Benzyloxycarbonylamino)-6-(methoxycarbonyl)hexanoic Acid.

A solution of 0.20 g (0.62 mmol) of the resultant compound of Example 61 in methanol was treated with sodium methoxide and stirred at ambient temperature for 2 h. The solvent was removed in vacuo, and the residue was acidified with 1N HCl, extracted with chloroform, and concentrated to give 159 mg of the desired compound.

Example 192

6-(Benzyloxycarbonylamino)-6-(methoxycarbonyl)hexanoyl-Leu-Asn Amide of 2-(2-Amino-1-hydroxy-3-phenylpropyl)tetrahydrofuran.

The resultant compound of Example 182 is hydrogenolyzed according to the procedure of Example 23 and coupled to the resultant compound of Example 191 using the mixed anhydride procedure of Example 4 to give the desired compound.

Example 193

6-Amino-6-(methoxycarbonyl)hexanoyl-Leu-Asn Amide of 2-(2-Amino-1-hydroxy-3-phenylpropyl)tetrahydrofuran.

The resultant compound of Example 192 is hydrogenated using the procedure of Example 23 to give the desired compound.

Example 194

6-Amino-6-carboxyhexanoyl-Leu-Asn Amide of 2-(2-Amino-1-hydroxy-3-phenylpropyl)tetrahydrofuran.

Using the procedure of Example 190 with the resultant compound of Example 193 gives a crude lithium salt which is neutralized to pH 7 and extracted with chloroform to give the desired compound.

Example 195

Cbz-Ile-Ser-OMe.

To H-Ser-OMe•HCl (5.7 g, 36.6 mmol) in 120 ml of anhydrous dimethylformamide (DMF) at 5°C was added 4-methylmorpholine (3.7 g, 36.6 mmol) and the mixture was stirred at 5°C for 0.5 h. Cbz-Ile-OH (9.8 g, 37 mmol), 1-hydroxybenzotriazole (HOBT) monohydrate (5.4 g, 40 mm) and N-ethyl-N′-(dimethylaminopropyl)carbodiimide (EDAC) (7 g, 36.6 mmol) were added sequentially. The mixture was stirred at 0-5°C for 3 h and at room temperature for 12 h. The mixture was poured into ethyl acetate, washed with citric acid, dilute $NaHCO_3$ solution, and water. The organic portion was dried and filtered. The filtrate was evaporated under reduced pressure to give a solid. The crude product was recrystallized from ethyl acetate and hexane to yield 9 g (67.7%) of product. $^1H$ NMR ($CDCl_3$, TMS) δ0.9-1.0 (m,6H), 3.8 (s,3H), 3.9-4.1 (m,3H), 4.7 (m,1H), 5.1 (q,2H), 7.35 (s,5H). Mass spectrum: $(M+H)^+$ = 367.

Example 196

H-Ile-Ser-OMe HCl

The resultant compound of Example 195 (3.4 g, 9.29 mmol) was hydrogenated at 4 atm $H_2$ with 20% Pd/C in methanol/HCl to obtain 2 g (80%) of the desired product. $^1H$ NMR (DMSO, TMS) δ 0.8-1.0 (m,6H), 3.65 (s,3H), 3.8 (m,2H), 4.4 (m,1H), 5.3 (t,1H). Mass spectrum: $(M+H)^+$ = 233.

Example 197

Cbz-Phe-Pro-O-t-Bu.

A mixture of Cbz-Phe-OH (3.89 g, 13 mmol) and H-Pro-O-t-Bu (2 g, 11.7 mmol) in 50 ml anhydrous DMF was added with HOBT monohydrate (2 g, 15 mmol) and EDAC (2.2 g, 11.9 mmol) at 0°C. The mixture was stirred at 0-5°C for 3 h and then at room temperature for 16 h. The mixture was poured into

ethyl acetate, washed with citric acid, dilute $NaHCO_3$ solution, and then water. The organic layer was dried ($MgSO_4$) and evaporated to a crude product which was chromatographed on silica gel eluting with ethyl acetate:hexane (2:3) to afford 4 g (68%) of desired product. $^1H$ NMR ($CDCl_3$, TMS) $\delta$ 1.5 (s,9H), 2.9-3.1 (m,2H), 3.4-3.7 (m,2H), 5 (q,2H), 7.3 (m,5H).

## Example 198

### Cbz-Phe-$^R$Pro-OH

The resultant compound of Example 197 (3.3 g, 7.29 mmol) was dissolved in 300 ml anhydrous tetrahydrofuran (THF) and 14 ml of 1 M $BH_3$ in THF (14 mmol) was added at -20 °C. After addition was complete, the reaction mixture was stirred at -20 °C for 4 h, and at room temperature for 12 h. The reaction mixture was cooled back to -20 °C and methanol was added dropwise until $H_2$ ceased to evolve. The solvent was evaporated under reduced pressure to a residual liquid. Some methanol was added and evaporated again. The residual oil was chromatographed on silica gel eluting with ethyl acetate:hexane (1:4) to afford 1.4 g (37.6%) of pure product.

The above resulting compound (600 mg, 1.43 mmol) was stirred in 7 ml 4 M HCl in dioxane for 12 h. The mixture was evaporated to gummy solid and chased with $CHCl_3$ several times to afford 550 mg (96%) of the desired product. $^1H$ NMR (DMSO, TMS) $\delta$ 2.7 (m,2H), 3.4 (m,4H), 4.05 (m,1H), 4.95 (q,2H), 7.2-7.4 (m,10 H). Mass spectrum: $(M+H)^+$ = 383.

## Example 199

### Cbz-Phe-$^R$Pro-Ile-Ser-OMe.

The resultant compounds of Example 198 (600 mg, 1.43 mmol) and Example 196 (376 mg, 1.4 mmol) were mixed in 8 ml anhydrous DMF. After cooling to 0 °C, 4-methylmorpholine (303 mg, 3.0 mmol) was added followed by HOBT monohydrate (270 mg, 2 mmol) and EDAC (287 mg, 1.5 mmol). The mixture was stirred at 0-5 °C for 3 h and at room temperature for 12 h. The mixture was poured into ethyl acetate, washed with dilute $NaHCO_3$ solution, water, dried, and filtered. The filtrate was evaporated to an oil which was chromatographed on silica gel eluting with 5% methanol/chloroform to afford 550 mg of product (66.2%). $^1H$ NMR ($CDCl_3$, TMS) $\delta$ 0.85-0.95 (m,6H), 2.7 (m,2H), 3-3.2 (m,4H), 5 (q,2H), 7.2-7.4 (m,10 H). Mass spectrum: $(M+H)^+$ = 597.

## Example 200

### Boc-Leu-Asn-Phe-$^R$Pro-Ile-Ser-OMe.

The resultant compound of Example 199 (300 mg, 0.5 mmol) was hydrogenated with 20% Pd/C at 4 atm $H_2$ in methanol/HCl to obtain 180 mg (69.8%) of the deprotected product which was used in the next step without further purification.

130 mg (0.253 mmol) of the above product (dihydrochloride salt) in 5 ml anhydrous DMF was treated at 0 °C with 4-methylmorpholine (60 mg, 0.6 mmol) followed by Boc-Leu-Asn-OH (104 mg, 0.3 mmol), HOBT monohydrate (108 mg, 0.8 mmol), and EDAC (57 mg, 0.297 mmol). The mixture was stirred at 0-5 °C for 3 h and at room temperature for 12 h. The mixture was poured into ethyl acetate, washed with dilute $NaHCO_3$ solution, water, dried, and filtered. The filtrate was evaporated to an oil which was chromatographed on

silica gel eluting with 5% methanol/chloroform to afford 70 mg (35%) of the desired product. $^1H$ NMR (DMSO, 140 °C, TMS) δ 0.87 (m,12H), 1.4 (s,9H), 3.6 (s,3H), 3.7 (m,1H), 3.95 (m,1H), 4.05 (m,1H), 4.3 (m,1H), 4.4 (m,1H), 7.2 (br s,5H). Mass spectrum: $(M+H)^+$ = 790.

Example 201

Cbz-Phe-$^R$Pro-$NHCH_2Ph$.

Using the procedure of Example 199 but replacing H-Ile-Ser-OMe•HCl with benzyl amine gives the desired compound after chromatography on silica gel.

Example 202

Boc-Leu-Asn-Phe-$^R$Pro-$NHCH_2Ph$.

The resultant compound from Example 201 is deprotected and coupled as described in Example 200 to give the desired compound.

Example 203

Boc-Leu-Asn-Phe-$^R$Pip-$NHCH_2Ph$.

Cbz Phe-$^R$Pip-OH is prepared starting from (L)-pipecolinic acid t-butyl ester (Pip-O-t-Bu) using the procedures described in Examples 197 and 198. This material is coupled sequentially to benzylamine and then to Boc-Leu-Asn-OH as described in Examples 199 and 200 to give the desired compound.

Example 204

Boc-Leu-Asn-Phe-$^R$Azd-$NHCH_2Ph$.

Likewise, replacing Pro-O-t-Bu in Examples 197 and 198 with (S)-(-)-2-azetidine carboxylic acid tert-butyl ester (Azd-O-t-Bu) gives Cbz-Phe-$^R$Azd-OH. This is coupled as described in Examples 199 and 200 with benzylamine and Boc-Leu-Asn-OH to give the desired product.

Example 205

3-(3(R)-(3-(tert-Butyloxycarbonyl)-2,2-dimethyl-4(S)-(cyclohexylmethyl)-5(R)-oxazolidinyl)-2(S)-chloro-3-hydroxy-1-oxopropyl)-4(S)-(phenylmethyl)-2-oxazolidinone.

The procedure of D.A. Evans, E.B. Sjogren, A.E. Weber, and R.E. Conn (Tetrahedron Lett. **1987**, 28(1), 39) was adapted. A solution of 4-(S)-3-(chloroacetyl)-4-(phenylmethyl)-2-oxazolidinone (1.273 g, 5.018 mmol) in 20 ml freshly distilled $Et_2O$ was cooled to -78° C under an argon atmosphere. Di-n-butylboryl triflate (1.51 g, 5.53 mmol) was added dropwise to the stirred solution, then triethylamine (0.85 ml, 620 mg, 6.10 mmol) is added dropwise. The cooling bath was removed after 5 min, and the resulting mixture was stirred at ambient temperature for 2 hr. The mixture was recooled to -78° C, then treated dropwise with a solution of the resultant compound from Example 241 (1.30 g, 3.98 mmol) in 10 ml ether. After stirring at -78° C for 0.75 hr and then at 0° C for 2.5 h, the reaction is quenched with 20 ml 0.5 M aqueous $NaHSO_4$ and 30 ml $Et_2O$ at 0° C. The aqueous phase is further extracted with 2 X 20 ml $Et_2O$, and the combined organic phases are washed with 20 ml $H_2O$, and the organic phase concentrated under reduced pressure. The residue was dissolved in 20 ml $Et_2O$, cooled to 0° C, and treated dropwise with 15 ml of 1:1 methanol/30% aqueous $H_2O_2$. The mixture was stirred for 1 hr at 0° C, then poured into 20 ml saturated aqueous $NaHCO_3$ and extracted with 2 X 40 ml $Et_2O$. The combined organic phases were washed with 20 ml brine, dried ($Na_2SO_4$), and the filtrate concentrated under reduced pressure to afford the crude product. Purification by column chromatography (0.5 % MeOH in $CH_2Cl_2$) afforded 671 mg (48%) of the desired product. $^1H$ NMR ($CDCl_3$) 0.85-1.72 (several m,12H), 1.48 (s,9H), 1.51 (s,3H), 1.52 (s,3H), 1.95 (bd,1H), 2,85 (dd,1H), 3.28 (dd,1H), 3.35 (bs,1H), 3.92 (bd,1H), 4.0 (ddd,1H), 4.23 (dd,1H), 4.26 (dd,1H), 4.71 (m,1H), 6.05 (d,1H), 7.2-7.37 (m,5H). Mass spectrum $(M+H)^+$ = 579.

Anal. Calcd. for $C_{30}H_{43}N_2O_7Cl$: C, 62.22; H, 7.48; N, 4.84.

Found: C, 62.00; H, 7.53; N, 4.61.

## Example 206

### 3-(3(R)-(3-(tert-Butyloxycarbonyl)-2,2-dimethyl-4(S)-(cyclohexylmethyl)-5(R)-oxazolidinyl)-2(S)-chloro-3-((3-ethyl)-carbamoyloxy)-1-oxopropyl)-4(S)-phenylmethyl)-2-oxazolidinone.

The resultant compound from Example 205 (223 mg, 0.386 mmol) was dissolved in 4 ml dry THF, and ethyl isocyanate (135 mg, 1.90 mmol) was added. The solution was warmed to 60° for 7 days, during which time additional portions of ethyl isocyante (270 mg, 3.8 mmol and 140 mg, 1.9 mmol) were added. The solution was concentrated and flash chromatographed (5% EtOAc-$CH_2Cl_2$) to produce 121 mg (48%) of the desired compound. $^1H$ NMR ($CDCl_3$) 0.9-1.85 (several bm,13H), 1.13 (bt,3H), 1.47 (s,9H), 1.53 (s,3H), 1.66 (s,3H), 2.84 (dd,1H), 3.16 (bm,2H), 3.31 (dd,1H), 3.9 (bm,1H), 4.2 (bm,2H), 4.34 (m,1H), 4.53 (m,1H), 4.78 (m,1H), 5.17 (m,1H), 6.25 (d,1H), 7.17-7.4 (m,5H). Mass spectrum $(M+H)^+$ = 650.

## Example 207

### 5(S)-(3-(tert-Butyloxycarbonyl)-2,2-dimethyl-4(S)-(cyclohexylmethyl)-5(R)-oxazolidinyl)-4(R)-carboxy-3-ethyl-2-oxazolidinone.

The procedure of D.A. Evans, E.B. Sjogren, A.E. Weber, and R.E. Conn (Tetrahedron Lett. **1987**, 28(1), 39) was adapted. A solution of the resultant compound from Example 206 (117 mg, 0.180 mmol) in 2.0 ml of THF was cooled to 0° C and treated with a solution of lithium hydroxide-monohydrate (16 mg, 0.38 mmol) in 0.8 ml $H_2O$. The resulting solution was stirred at 0° C for 2 hr. The THF was removed under reduced pressure, and the residue partitioned between 20 ml $CH_2Cl_2$ and 5 ml of 1.0 N aqueous $NaHSO_4$. The aqueous phase was further extracted with $CH_2Cl_2$ (2 X 10 ml), and the combined organic phases were washed with 15 ml brine, dried ($MgSO_4$), filtered and concentrated under reduced pressure to produce 109 mg of crude mixture of products (oxazolidinone plus carboxylic acid). This mixture was dissolved in 5 mL dry DMF, the solution was cooled to 0° under an argon atmosphere, and a solution of potassium tert-butoxide (0.90 ml, 1.0 M in THF, 0.90 mmol) was introduced into the reaction. After 45 min at 0° and an additional 1 hr at room temperature, the solvent was removed under high vacuum. The residue was diluted

with 3 ml $Et_2O$, cooled to 0°, and acidified with 1.0 M $NaHSO_4$. The mixture was extracted with 4 X 10 ml $CH_2Cl_2$, and the combined organic extracts were washed with 15 ml brine, dried ($MgSO_4$), and concentrated under reduced pressure. The crude mixture was purified by flash chromatography (10% MeOH-$CH_2Cl_2$ to 20% MeOH-$CH_2Cl_2$) to provide 36.4 mg (53%) of the desired acid. $^1H$ NMR ($CDCl_3$) 0.8-1.95 (several bm,13H), 1.19 (bt,3H), 1.48 (s,9H), 1.58 (bs,3H), 1.64 (bs,3H), 3.08 (m,1H), 3.64 (bm,1H), 3.96 (dd,1H), 4.1 (bm,1H), 4.12 (bd,1H), 4.58 (dd,1H).

## Example 208

### 5(S)-(3-(tert-Butyloxycarbonyl)-2,2-dimethyl-4(S)-(cyclohexylmethyl)-5(R)-oxazolidinyl)-4(R)-(n-butylcarboxamido)-3-ethyl-2-oxazolidinone.

Using the general procedure of Example 66, the resultant compound from Example 207 (34.3 mg, 0.0755 mmol) was coupled to n-butylamine (7.4 mg, 0.10 mmol) to provide the desired compound. $^1H$ NMR ($CDCl_3$) 0.8-1.75 (several bm, 17H), 0.95 (t,3H), 1.15 (t,3H), 1.46 (s,9H), 1.51 (bs,3H), 1.62 (bs,3H), 1.89 (bd,1H), 3.02 (m,1H), 3.25 (m,1H), 3.44 (m,1H), 3.62 (m,1H), 3.98 (bd,1H), 4.11 (bd plus bm,2H), 4.47 (bd,1H), 5.83 (bt,1H). High resolution mass spectrum. Calcd. for $C_{27}H_{48}N_{30}$: 510.3538. Found: 510.3538.

## Example 209

### 5(S)-(2(S)-Amino-3-cyclohexyl-1-hydroxypropyl)-4(R)-(n-butylcarboxamido)-3-ethyl-2-oxazolidinone.

The resultant compound from Example 208 (6.4 mg, 0.013 mmol) was dissolved in 0.6 ml dry $CH_2Cl_2$, cooled to -10° ($N_2$), and the resulting solution treated with 0.6 ml trifluoroacetic acid. After 1.5 h at -10° to 0°, the solvent was removed under reduced pressure without warming, then the residue was dissolved in 1 ml THF and 0.5 ml $H_2O$. The resulting solution was stirred at ambient temperature for 2.5 hr, at which time the solution was concentrated under reduced pressure and the residue partitioned between 15 ml $CH_2Cl_2$ and 10 ml 1.0 M $Na_2CO_3$. The aqueous phase was further extracted (2 X 10 ml $CH_2Cl_2$), and the combined organic phases were washed with 15 ml brine, dried ($Na_2SO_4$) and concentrated to yield 5.1 mg (109%) of the desired product as a viscous oil. $^1H$ NMR ($CDCl_3$) 0.8-2.0 (several bm,17H), 0.94 (t,3H), 1.06 (t,3H), 3.05 (dq,1H), 3.15 (bt,1H), 3.35 (bm,3H), 3.72 (m,1H), 4.29 (d,1H), 4.57 (dd,1H), 6.3 (bt,1H). Mass spectrum $(M+H)^+$ = 370.

## Example 210

### Boc-Leu-Asn Amide of 5(S)-(2(S)-Amino-3-cyclohexyl-1(R)-hydroxypropyl)-4(R)-(N-butylcarbamoyl)-3-ethyl-2-oxazolidinone.

The resultant compound of Example 209 is coupled to Boc-Leu-Asn-OH according to the procedure of Example 66 to give the desired compound.

## Example 211

3-(3(S)-(3-(tert-Butyloxycarbonyl)-2,2-dimethyl-4(S)-(cyclohexylmethyl)-5(R)-oxazolidinyl)-2(R)-bromo-3-hydroxy-1-oxopropyl)-4(R)-(phenylmethyl)-2-oxazolidinone.

Using the procedure of Example 207, the boryl enolate derived from the resultant compound from Example 206 is allowed to react with the resultant compound from Example 241, to provide the desired product.

## Example 212

3-(3(S)-(3-(tert-Butyloxycarbonyl)-2,2-dimethyl-4(S)-(cyclohexylmethyl)-5(R)-oxazolidinyl)-2(R)-bromo-3-(N-methylcarbamoyloxy)-1-oxopropyl)-4(R)-(phenylmethyl)-2-oxazolidinone.

Using the procedure of Example 208, the resultant compound from Example 211 is treated with methyl isocyanate to provide the desired compound.

## Example 213

3(S)-(3-(tert-Butyloxycarbonyl)-2,2-dimethyl-4(S)-(cyclohexylmethyl)-5(R)-oxazolidinyl)-2(R)-bromo-3-(N-methylcarbamoyloxy)propanoic acid

Using the procedure of Example 209, the resultant compound from Example 212 is hydrolyzed with lithium hydroxide-monohydrate to afford the desired product.

## Example 214

5(R)-(3-(tert-Butyloxycarbony)-2,2-dimethyl-4(S)-(cyclohexylmethyl)-5(R)-oxazolidinyl)-4(S)-carboxyl-3-methyl-2-oxazolidinone.

Using the procedure of Example 210, the resultant compound from Example 213 is cyclized with potassium tert-butoxide in DMF, affording the desired compound.

## Example 215

4(R)-Methyl-3-(4-pentenoyl)-5(S)-phenyl-2-oxazolidinone.

To a solution of 4(R)-methyl-5(S)-phenyl-2-oxazolidinone (prepared according to D.A. Evans, et al., J. Am. Chem. Soc., **1981**, 103, 2127) (1.00 g, 5.64 mmol) in 15 ml dry THF, cooled to -78° C under a nitrogen atmosphere, was added dropwise a solution of n-butyllithium (2.26 ml, 2.5 M in hexanes, 5.65 mmol). After stirring at -78° C for 5 min, a solution of 4-pentenoyl chloride (0.669 g, 5.64 mmol) in 2 ml THF was added dropwise over 3 min. The resulting solution was stirred for 15 min at -78° C, then allowed to warm to room temperature and stir another 1.5 hr. The reaction was quenched by the addition of 3 ml saturated aqueous

$NaHCO_3$, and the resulting mixture stirred, then concentrated under reduced pressure. The residue was partitioned between 50 ml $Et_2O$ and 25 ml $H_2O$. The aqueous phase was further extracted (3 X 25 ml $Et_2O$), and the combined organic phases were washed with 50 ml brine, dried over $MgSO_4$, filtered, and the filtrate concentrated under reduced pressure. Purification by column chromatography gave 1.03 g (70%) of the desired product. $^1H$ NMR ($CDCl_3$) δ 0.90 (d,3H), 2.45 (m,2H), 3.07 (m,2H), 4.77 (dq,1H), 5.03 (ddd,1H), 5.10 (ddd,1H), 5.67 (d,1H), 5.88 (m,1H), 7.29-7.46 (m,5H); Mass spectrum $(M+H)^+$ = 260.

## Example 216

### 3-(3(R)-(3-(tert-Butyloxycarbonyl)-2,2-dimethyl-4(S)-(cyclohexylmethyl)-5(R)-oxazolidinyl)-3-hydroxy-2(R)-(2-propenyl)-1-oxopropyl)-4(R)-methyl-5(S)-phenyl-2-oxazolidinone.

The procedure of D.A. Evans, et al., J. Am. Chem. Soc. **1981**, 103, 2127 is adapted. A solution of the resultant compound from Example 215 (984 mg, 3.80 mmol) in 4.0 ml dry $CH_2Cl_2$ was cooled to -78° C under an argon atmosphere, and treated dropwise with di-n-butylboryl triflate (1.15 g, 4.20 mmol). The resulting mixture was stirred and the cooling bath removed until the suspension had dissolved. After recooling to -78° C, diisopropylethylamine (594 mg, 4.60 mmol) was added dropwise over 5 min, and the mixture stirred for 30 min at -78° C, followed by an additional 1 hr at 0° C. The solution was cooled to -78° C, and then a solution of the resultant compound from Example 241 (1.21 g, 3.72 mmol) in 3.0 ml $CH_2Cl_2$ was added dropwise via cannula. The reaction was stirred at -78° C for 45 min, then at 0° C for an additional 2.5 hr. The reaction was quenched with 2.5 ml pH 7 phosphate buffer, 5.0 ml methanol, and a solution of 2.5 ml 30% aqueous $H_2O_2$ in 5.0 ml methanol. After stirring at 0° C for 1 hr, the mixture was partitioned between 30 ml pH 7 phosphate and 30 ml $CH_2Cl_2$. The aqueous phase was extracted (4 x 25 ml $CH_2Cl_2$), and the combined organic extracts were washed with 30 ml brine, dried over $MgSO_4$, filtered, and the filtrate concentrated under reduced pressure. Purification by column chromatography gave 1.279 g (59%) of the desired compound. $^1H$ NMR ($CDCl_3$) δ 0.88 (d,3H), 0.9-1.75 (several br m,12H), 1.49 (s,9H), 1.50 (s,3H), 1.65 (s,3H), 1.90 (br d,1H), 2.42 (br d,1H), 2.58 (br m,1H), 2.80 (br m,1H), 3.87 (br m,2H), 4.1 (br m,3H), 4.82 (dq,1H), 5.02 (m,1H), 5.08 (m,1H), 5.66 (d,1H), 5.83 (br m,1H), 7.3-7.46 (br m,5H); Mass spectrum: $(M+H)^+$ = 585.

## Example 217

### 3-(3(R)-(3-(tert-Butyloxycarbonyl)-2,2-dimethyl-4(S)-(cyclohexylmethyl)-5(R)-oxazolidinyl)-3-hydroxy-2(R)-(2-hydroxyethyl)-1-oxopropyl)-4(R)-methyl-5(S)-phenyl-2-oxazolidinone.

The procedure of D.A. Evans and E.B. Sjogren (Tetrahedron Lett. **1986**, 27(41), 4961) is adapted. A solution of the resultant compound from Example 216 (290 mg, 0.50 mmol) in 5 ml of $CH_2Cl_2$ containing 0.1 mg Sudan III dye, is cooled to -78° C under a nitrogen atmosphere and a dilute mixture of ozone in oxygen is passed through the solution until the pink color disappears. Excess ozone is removed with a stream of oxygen, and then dimethylsulfide (310 mg, 5.0 mmol) is added. The cooling bath is removed, and the solution is stirred at ambient temperature for 1 h. The solution is then concentrated under reduced pressure, and the residue is dissolved in ethanol (2 ml), cooled to 0° C, and treated with sodium cyanoborohydride (32 mg, 0.5 mmol). The solution is stirred until TLC indicates complete reaction. The ethanol is removed under reduced pressure, and the residue is partitioned between 10 ml $CH_2Cl_2$ and 5 ml water. The aqueous phase is extracted with 5 ml $CH_2Cl_2$, and the combined organic phases are washed with brine, dried over $Na_2SO_4$ and concentrated under reduced pressure. Column chromatography provides the desired product.

## Example 218

3-((2(R)-(3-(tert-Butyloxycarbonyl)-2,2-dimethyl-4(S)-(cyclohexylmethyl)-5(R)-oxazolidinyl)-2,3,4,5-tetrahydrofuran-3(R)-yl)carbonyl)-4(R)-methyl-5(S)-phenyl-2-oxazolidinone.

A solution of the resultant compound from Example 217 (235 mg, 0.400 mmol) in ml dry $CH_2Cl_2$ is cooled to -30°C under a dry nitrogen atmosphere, and sequentially treated with triethylamine (67 )l, 49 mg, 0.480 mmol) and methanesulfonyl chloride (31 )l, 46 mg, 0.40 mmol). The resulting solution is stirred at -30°C for 1 hr. The reaction is quenched with 1 ml pH 7 phosphate buffer, and the resulting mixture is partitioned between 15 ml $CH_2Cl_2$ and 5 ml water. The aqueous phase is extracted (2 X 5 ml $CH_2Cl_2$), and the combined organic phases are washed (5 ml saturated aqueous $CuSO_4$, 5 ml water, 5 ml brine), dried over $MgSO_4$, filtered and concentrated under reduced pressure. The residue is further dried under high vacuum to afford the crude mesylate. The mesylate is then dissolved in 2 ml DMF, cooled to 0°C and treated with sodium hydride (16 mg, 0.40 mmol). The reaction mixture is allowed to warm to room temperature and stir until TLC indicates complete reaction. The DMF is removed under high vacuum, and the residue is partitioned between 15 ml $CH_2Cl_2$ and 5 ml $H_2O$. The aqueous phase is extracted (2 X 5 ml $CH_2Cl_2$), and the combined organic phases are washed with 5 ml brine, dried over $MgSO_4$, and the filtrate concentrated under reduced pressure. Purification by column chromatography produces the desired compound.

Example 219

2(R)-(3-(tert-Butyloxycarbonyl)-2,2-dimethyl-4(S)-(cyclohexylmethyl)-5(R)-oxazolidinyl)-2,3,4,5-tetrahydrofuran-3-(R)-carboxylic acid.

Using the procedure of Example 209, the resultant compound from Example 218 is hydrolyzed with lithium hydroxide-monohydrate to afford the desired compound.

Example 220

3-(3(R)-(3-(tert-Butyloxycarbonyl)-2,2-dimethyl-4(S)-(4-methexyphenyl)methyl)-5(R)-oxazolidinyl)-3-hydroxy-2(R)-(2-propenyl)-1-oxopropyl)-4(R)-methyl-5(S)-phenyl-2-oxazolidinone.

Using the procedure of Example 216, the resultant compound from Example 243 is allowed to react with the boryl enolate derived from the resultant compound from Example 215 to afford the desired product.

Example 221

3-((2(R)-(3-(tert-Butyloxycarbonyl)-2,2-dimethyl-4(S)-(4-methoxyphenyl)methyl)-5(R)-oxazolidinyl)-5(R,S)-methyl-2,3,4,5-tetrahydrofuran-3(R)-yl)carbonyl)-4(R)-methyl-5(S)-phenyl-2-oxazolidinone.

A solution of the resultant compound from Example 220 (292 mg, 0.500 mmol) in 2 ml ether is treated with 2 ml of saturated aqueous $NaHCO_3$, cooled to 0°C, and a solution of iodine (127 mg, 0.500 mmol) in 0.5 ml ether. The iodomethyl-tetrahydrofuran is isolated by extraction with $Et_2O$, and the ether extract is washed with 5% aqueous $NaHCO_3$, brine, then concentrated under reduced pressure. The crude iodide is then dissolved in 10 ml toluene, a,a′-(azoisobutyronitrile) (2 mg) is added, and the solution is treated with tributyltin hydride (146 mg, 0.501 mmol), added dropwise. The resulting solution is stirred at room

temperature until TLC indicates complete reaction. The mixture is then partitioned between 20 ml hexane and 20 ml acetonitrile. The acetonitrile phase is extracted with hexane (2 X 10 ml), then concentrated under reduced pressure. Column chromatography affords the desired product.

## Example 222

2(R)-(3-(tert-Butyloxycarbonyl)-2,2-dimethyl-4(S)-(cyclohexylmethyl)-5(R)-oxazolidinyl)-5(R,S)methyl-2,3,4,5-tetrahydrofuran-3(R)-carboxylic Acid.

Using the procedure of Example 209, the resultant compound from Example 221 is hydrolyzed with lithium hydroxide-monohydrate to produce the desired compound.

## Example 223

2(R)-(3-(tert-Butyloxycarbonyl)-2,2-dimethyl-4(S-(hydroxyphenyl)methyl-5(R)-oxazolidinyl)-5(R,S)-methyl-2,3,4,5-tetrahydrofuran-3(R)-carboxylic Acid.

The procedure of E.H. Vickery, L.F. Pahler, and E.J. Eisenbraun (J. Org. Chem. **1979**, 44, 4444) is adapted. A solution of the resultant compound from Example 222 in $CH_2Cl_2$ is cooled to -78 °C under a nitrogen atmosphere, and treated with boron tribromide (1 equivalent). After stir ring at -78 °C for 6 hr, the reaction is quenched by adding to a stirred mixture of 10% $NaHCO_3$ and EtOAc at 0 °C. Extractive workup with EtOAc is followed by drying over $Na_2SO_4$, filtration, and concentration of the filtrate under reduced pressure. Column chromatography affords the desired product.

## Example 224

Methyl 3(R)-(3-(tert-Butyloxycarbonyl)-2,2-dimethyl-4(S)-(cyclohexylmethyl)-5(R)-oxazolidinyl)-3-hydroxy-2(R)-(2-propenyl)propionate.

The procedure of D.A. Evans and A.E. Weber (J. Am. Chem. Soc. **1987**, 109, 7151) is adapted. A solution of the resultant compound from Example 216 (175 mg, 0.299 mmol) is dissolved in 0.7 ml dry $CH_2Cl_2$ and 0.7 ml dry methanol, cooled to 0 °C under a nitrogen atmosphere, and treated with a mixture resulting from the addition of methylmagnesium bromide (1.31 mmol) to 2.0 ml methanol. The resulting mixture is stirred at 0 °C for 3 hr, and at room temperature for 0.5 hr. The mixture is quenched with pH 7 phosphate buffer and extracted with $CH_2Cl_2$ (4 X 25 ml). The combined organic phases were dried over $MgSO_4$, and the filtrate is concentrated in vacuo. The residue is purified by column chromatography to afford the desired compound.

## Example 225

Methyl 3(R)-(3-(tert-Butyloxycarbonyl)-2,2-dimethyl-4(S)-(cyclohexylmethyl)-5(R)-oxazolidinyl)-3-hydroxy-2(R)-(3-hydroxypropyl)propionate.

The procedure of D.A. Evans and A.E. Weber (J. Am. Chem. Soc. **1987**, 109, 7151) is adapted. To a 0° C suspension of di-cyclohexylborane (1.5 equivalents) in dry $CH_2Cl_2$ is added a solution of the resultant compound from Example 224 (1.0 equivalents). The cooling bath is removed, and the resulting mixture is stirred at room temperature for 5 hr. The mixture is recooled to 0° C and treated with 30% aqueous $H_2O_2$ in 1.0 M NaOH. After 1 hr, the mixture is partitioned between $H_2O$ and $CH_2Cl_2$, then the organic phase dried over $MgSO_4$, and the filtrate concentrated under reduced pressure. Purification of the residue by column chromatography affords the desired product.

## Example 226

### 2(R)-(3-(tert-Butyloxycarbonyl)-2,2-dimethyl-4(S)-(cyclohexylmethyl)-5(R)-oxazolidinyl)-3(R)-(methoxycarbonyl)-2,3,4,5-tetrahydropyran.

Using the procedure of Example 218, the resultant compound from Example 225 is cyclized to afford the desired compound.

## Example 227

### N-(5(S)-(3-(tert-Butyloxycarbonyl)-2,2-dimethyl-4(S)-(cyclohexylmethyl)-5(R)-oxazolidinyl)-3-methyl-2-oxazolidinon-4(R)-carbonyl)-Ile-Ser-Methyl Ester.

The resultant compound of Example 210 is coupled to H-Ile-Ser-OMe according to the procedure of Example 66 to give the desired compound.

## Example 228

### N-(Boc-Leu-Asn Amide of 5(S)-(2(S)-Amino-3-cyclohexyl-1(R)-hydroxypropyl)-3-methyl-2-oxazolidinon-4(R)-carbonyl)-Ile-Ser Methyl Ester.

The resultant compound of Example 227 is deprotected according to the procedure of Example 2 and coupled to Boc-Leu-Asn-OH according to the procedure of Example 66 to give the desired compound.

## Example 229

### 5(R)-(3-(tert-Butyloxycarbonyl)-2,2-dimethyl-4(S)-(cyclohexylmethyl)-5(R)-oxazolidinyl)-3-methyl-4(S)-(N-(2-methylbutyl)carbamoyl)-2-oxazolidinone.

The resultant compound of Example 214 is coupled to (2-methylbutyl)amine according to the procedure of Example 66 to give the desired compound.

## Example 230

5(R)-(((2R)-Isobutyl-3-morpholinocarbonylpropanoyl)-Asn Amide of 2(S)-Amino-3-cyclohexyl-1(R)-hydroxypropyl)-3-methyl-4(S)-(N-(2-methylbutyl)carbamoyl)-2-oxazolidinone.

The resultant compound of Example 229 is deprotected according to the procedure of Example 2 and coupled to Cbz-Asn-OH according to the procedure of Example 66. After purification, the above compound is deprotected according to the procedure of Example 23 and coupled to the resultant compound of Example 26 using the mixed anhydride procedure of Example 4 to give the desired compound.

Example 231

N-(2(R)-(3-(tert-Butyloxycarbonyl)-2,2-dimethyl-4(S)-(cyclohexylmethyl)-5(R)-oxazolidinyl)-2,3,4,5-tetrahydrofuran-3(R)-carbonyl)-Ile-Ser Methyl Ester.

The resultant compound of Example 219 is coupled to H-Ile-Ser-OMe according to the procedure of Example 66 to give the desired compound.

Example 232

N-(2(R)-((3,4-cis-Dihydroxypyrrolidinylcarbonyl)-Leu-Asn Amide of 2(S)-Amino-3-cyclohexyl-1(R)-hydroxypropyl)-2,3,4,5-tetrahydrofuran-3(R)-carbonyl)-Ile-Ser Methyl Ester.

The resultant compound of Example 231 is deprotected according to the procedure of Example 2 and coupled to Cbz-Asn-OH according to the procedure of Example 66.

After purification, the above compound is deprotected according to the procedure of Example 23 and coupled to the resultant compound of Example 27 using the procedure of Example 66 to give the desired compound.

Example 233

N-(2(R)-(3-(tert-Butyloxycarbonyl)-2,2-dimethyl-4(S)-((4-methoxyphenyl)methyl)-5(R)-oxazolidinyl)-5-methyl-2,3,4,5-tetrahydrofuran-3(R)-carbonyl)-Ile-Ser Methyl Ester.

The resultant compound of Example 222 is coupled to H-Ile-Ser-OMe according to the procedure of Example 66 to give the desired compound.

Example 234

N-(2(R)-((4-Sulphonylmorpholinylcarbonyl)-Leu-AsnAmide of 2(S)-Amino-1(R)-hydroxy-3-(4-methoxyphenyl)-propyl)-5-methyl-2,3,4,5-tetrahydrofuran-3(R)-carbonyl)-Ile-SerMethyl Ester.

The resultant compound of Example 233 is deprotected according to the procedure of Example 2 and coupled to Cbz-Asn-OH according to the procedure of Example 66. After purification, the above compound is deprotected according to the procedure of Example 23 and coupled to the resultant compound of Example 29 using the mixed anhydride procedure of Example 4 to give the desired compound.

Example 235

2(R)-(3-(tert-Butyloxycarbonyl)-2,2-dimethyl-4(S)-((4-hydroxyphenyl)methyl)-5(R)-oxazolidinyl)-5-methyl-3(R)-(N-(2-methylbutyl)carbamoyl)-2,3,4,5-tetrahydrofuran.

The resultant compound of Example 223 is coupled to (2-methylbutyl)amine according to the procedure of Example 66 to give the desired compound.

Example 236

2(R)-((3-Benzyloxycarbonylaminopropyl)-Leu-AsnAmide of 2(S)-Amino-1(R)-hydroxy-3-(4-methoxyphenyl)propyl)-5-methyl-3(R)-(N-(2-methylbutyl)carbamoyl)-2,3,4,5 tetrahydrofuran.

The resultant compound of Example 235 is deprotected according to the procedure of Example 2 and coupled to Cbz-Asn-OH according to the procedure of Example 66. After purification, the above compound is deprotected according to the procedure of Example 23 and coupled to the resultant compound of Example 50 using the procedure of Example 66 to give the desired compound.

Example 237

2(R)-(Cbz-Sar-Leu-Asn Amide of 2(S)-Amino-3-cyclohexyl-1(R)-hydroxypropyl)-3(R)-methoxycarbonyl-2,3,4,5-tetrahydropyran.

The resultant compound of Example 226 is deprotected according to the procedure of Example 2 and coupled to Cbz-Asn-OH according to the procedure of Example 66. After purification, the above compound is deprotected according to the procedure of Example 23 and coupled to the resultant compound of Example 52 using the mixed anhydride procedure of Example 4 to give the desired compound.

Example 238

2(R)-(N-(3-Aminopropyl)-Leu-Asn Amide of 2(S)-Amino-1(R)-hydroxy-3-((4-methoxyphenyl)propyl)-5-methyl-3(R)-(N-(2-methylbutyl)carbamoyl)-2,3,4,5-tetrahydrofuran.

The resultant compound of Example 236 is deprotected according to the procedure of Example 23 to give the desired compound.

Example 239

2(R)-(H-Sar-Leu-Asn Amide of 2(S)-Amino-3-cyclohexyl-1(R)-hydroxypropyl)-3(R)-methoxycarbonyl-2,3,4,5-tetrahydropyran.

The resultant compound of Example 237 is deprotected according to the procedure of Example 23 to give the desired compound.

Example 240

3-(t-Butyloxycarbonyl)-4-(cyclohexylmethyl)-2,2-dimethyl-5-vinyloxazolidine.

The procedure of S. Thaisrivong (J. Med. Chem. **1987**, 30, 976) was employed. A solution of 40 g of the resultant compound of Example 112 and 102 g of 2-methoxypropene in 250 ml of dichloromethane was stirred at room temperature. Solid pyridinium p-toluenesulfonate (PPTS) (177 g) was added slowly to the reaction mixture. After addition was complete, the reaction was stirred for 1 h and neutralized by addition of solid sodium bicarbonate. The solids were filtered and the filtrate was concentrated. Flash chromatography on silica gel gave 57 g of the desired compound. IR ($CDCl_3$) 1690 (C=O carbamate) $cm^{-1}$; $^1H$ NMR ($CDCl_3$) $\delta$ 5.95 (m,1H), 5.32 (m,1H), 5.20 (dt,1H), 4.27 (dd,1H), 1.47 (s,9H).
Anal. Calcd. for $C_{19}H_{33}NO_3$: C, 70.55; H, 10.28; N, 4.33.
Found: C, 70.47; H, 10.27; N, 4.09.

Example 241

3-(t-Butyloxycarbonyl)-4-(cyclohexylmethyl)-2,2-dimethyloxazolidine-5-carboxaldehyde.

A solution of 10 g of the resultant compound of Example 240 in 150 ml of 2:1 dichloromethane:methanol was cooled in an dry-ice acetone bath. Ozone was bubbled through the solution until a blue color persisted (1 h). Dry nitrogen was then bubbled through the reaction mixture to remove excess dissolved ozone. The reaction mixture was cannulated into a suspension of 8 g zinc dust, 8 ml glacial acetic acid, 200 ml water, and 200 ml of methanol cooled to -45° C. After 5 min the bath was removed and the mixture allowed to warm to room temperature overnight. 100 ml of saturated sodium chloride was added and the entire reaction mixture extracted with two 300 ml portions of dichloromethane. The combined dichloromethane extracts were decanted, dried ($MgSO_4$), filtered, and evaporated. The crude aldehyde was purified by flash chromatography (1:4) ethyl acetate:hexane to give 9.7 g of the desired compound as a mixture of diastereomers (3:1 trans:cis) as judged by the integrated resonances of the two aldehyde protons. IR ($CDCl_3$) 1735 (C=O aldehyde), 1690 (C=O carbamate) $cm^{-1}$; $^1H$ NMR ($CDCl_3$) $\delta$ 9.83 (s,1H,CHO), 9.73 (d,1H,CHO cis diastereomer), 4.14 (m,1H), 1.46 (s,9H).
Anal. Calcd. for $C_{18}H_{31}NO_4$: C, 66.43; H, 9.60; N, 4.30.
Found: C, 65.27; H, 9.79; N, 4.20.

Equilibration of Aldehyde Isomers

A suspension of 25 g of the above aldehyde in 300 ml of methanol and powdered potassium carbonate (10.7 g) was stirred at room temperature for 6 h. The reaction mixture was cooled in an ice-water bath and

treated with 9.3 g of glacial acetic acid for 5 min. A solution of 0.5 M sodium dihydrogen phosphate (300 ml) was added to the mixture. After 30 min, the solution was concentrated to one-half the volume under reduced pressure and extracted with ether (600 ml). The combined ether extracts were dried ($MgSO_4$), filtered, and concentrated. The aldehyde was purified by flash chromatography using (1:4) ethyl acetate:hexane to give 19.5 g of the desired compound as an 8:1 mixture of trans:cis diastereomers.

## Example 242

### 3-(t-Butyloxycarbonyl)-2,2-dimethyl-4-(phenylmethyl)-oxazolidine-5-carboxaldehyde.

Using the procedures of Examples 112, 240, and 241 but replacing Boc-cyclohexylalanine methyl ester with Boc-phenylalanine methyl ester gives the desired compound.

## Example 243

### 3-(t-Butyloxycarbonyl)-2,2-dimethyl-4-((4-methoxyphenyl)methyl)-oxazolidine-5-carboxaldehyde.

Using the procedures of Examples 112, 240, and 241 but replacing Boc-cyclohexylalanine methyl ester with Boc-(4-methoxy)phenylalanine methyl ester gives the desired compound.

## Example 244

### 3-(Benzyloxyacetyl)-4(S)-isopropyl-2-oxazolidinone.

A solution of 5.00 g (38.7 mmol) of 4(S)-isopropyl-2-oxazolidinone in 70 ml freshly distilled THF was cooled to -78 °C under a nitrogen atmosphere, then treated dropwise with 15.5 ml of a 2.5 M solution of n-butyllithium (in hexanes). Then 6.72 ml (7.86 g, 42.6 mmol) of benzyloxyacetyl chloride was added dropwise to the solution over 5 min. After stirring at -78 °C for 5 min, the cooling bath was removed, and the resulting solution was stirred at ambient temperature for 1 h. The reaction was quenched with 5 ml saturated aqueous $NaHCO_3$, and the mixture concentrated under reduced pressure. The residue was partitioned between 30 ml $Et_2O$ and 20 ml 1.0 M aqueous NaOH, and the aqueous phase was extracted with 3 X 30 ml $Et_2O$. The combined organic phases were washed with 30 ml $H_2O$, then with 30 ml brine, dried ($MgSO_4$), filtered, and concentrated under reduced pressure to afford the crude product. Crystallization from $Et_2O$ yielded 8.95 g (23%) of the desired compound. $^1H$ NMR ($CDCl_3$) $\delta$ 0.88 (d,3H), 0.94 (d,3H), 2.44 (m,1H), 4.26 (dd,1H), 4.34 (t,1H), 4.45 (m,1H), 4.67 (s,2H), 4.70 (s,2H), 7.26-7.43 (m,5H).
Anal. Calcd. for $C_{15}H_{19}NO_4$: C, 64.97; H, 6.91; N, 5.02.
Found: C, 64.93; H, 6.78; N, 5.02.

## Example 245

### 3-(3(R)-(3-(tert-Butyloxycarbonyl)-2,2-dimethyl-4(S)-cyclohexylmethyl-5(R)-oxazolidinyl)-3-hydroxy-2(S)-benzyloxy-1-oxopropyl)-4(S)-isopropyl-2-oxazolidinone.

A solution of the resultant compound from Example 244 (2.775 g, 10.01 mmol) in 10 ml $CH_2Cl_2$ (freshly distilled from $P_2O_5$) was cooled to -78° C (under an argon atmosphere), and treated dropwise over 15 min with di-n-butylboron triflate (3.025 g, 11.04 mmol). The resulting suspension was stirred at -78° C for 10 min, then diisopropylethylamine (2.11 ml, 1.57 g, 12.1 mmol, freshly distilled from $CaH_2$) was added dropwise over 10 min. The mixture was stirred at -78° C for 45 min, then warmed to 0° C for an additional 15 min. After recooling to -78° C, a solution of the resultant compound from Example 241 (2.172 g, 6.674 mmol, f4;1 mixture of epimers) in 5 ml $CH_2Cl_2$ was added via cannula, and the resulting yellow mixture was allowed to react at -78° C for 1.5 h, then warmed to 0° C, and stirred for an additional 15 min. The reaction was quenched with 20 ml methanol and 10 ml of pH 7.0 phosphate buffer. The resulting mixture was stirred 30 min at 0° C, then treated with 9 ml of 30% aqueous $H_2O_2$, and stirring was continued an additional 1 h. The mixture was extracted with 4 X 50 ml $CH_2Cl_2$, and the combined organic extracts were washed with 50 ml brine, dried ($MgSO_4$), and the filtrate concentrated under reduced pressure to give 6.708 g of viscous oil. Purification by column chromatography (2% MeOH-$CH_2Cl_2$) afforded 1.761 g (44%) of the desired compound. $^1H$ NMR ($CDCl_3$) δ 0.83 (d,3H), 0.93 (d,3H), 0.9-1.3 and 1.45-1.72 (several br m,12H), 1.45 (s,9H), 1.50 (br s,3H), 1.55 (br s, 3H), 1.92 (br d,1H), 2.35 (m,1H), 2.55 (br s,1H), 3.78 (ddd,1H), 3.96 (br d,1H), 4.12 (br m,1H), 4.2-4.3 (m,2H), 4.38 (m,1H), 4.65 (br s,2H), 5.57 (br d,1H), 7.3-7.4 (m,5H). Mass spectrum: $(M+H)^+$ = 603.

## Example 246

### 3-(3(R)-(3-(tert-Butyloxycarbonyl)-2,2-dimethyl-4(S)-cyclohexylmethyl-5(R)-oxazolidinyl)-2(S),3-dihydroxy-1-oxopropyl)-4(S)-isopropyl-2-oxazolidinone.

A solution of the resultant compound from Example 245 (0.5 mmol) in 5 ml ethanol is exposed to 1 atmosphere of hydrogen gas in the presence of 0.5 g of 10% palladium on carbon at ambient temperature. The mixture is stirred for 18 hr, then filtered through a pad of celite, and the filtrate is concentrated under reduced pressure to give the desired product.

## Example 247

### 3-((5(S)-(3-(tert-Butyloxycarbonyl)-2,2-dimethyl-4(S)-cyclohexylmethyl-5(R)-oxazolidinyl)-1,3-dioxolan-2-one-4(S)-yl)carbonyl)-4(S)-isopropyl-2-oxazolidinone.

To a solution of the resultant compound from Example 246 (0.5 mmol) in 2 ml 1,2-dichloroethane is added 0.5 mmol of 1,1′-carbonyldiimidazolide, and the resulting solution warmed to reflux for 24 h. The solution is cooled, diluted with 40 ml $CH_2Cl_2$, and washed with 20 ml 0.1 M $NaHSO_4$. The organic phase is washed with 20 ml brine, dried ($Na_2SO_4$), and the filtrate is concentrated under reduced pressure to afford the desired product.

## Example 248

### 5(R)-(3-(tert-Butyloxycarbonyl)-2,2-dimethyl-4(S)-cyclohexylmethyl-5(R)-oxazolidinyl)-4(S)-carboxy-1,3-dioxolan-2-one.

An ice-cooled solution of the resultant compound from Example 247 in THF is treated with a solution of 2.0 equivalents of lithium hydroxide monohydrate in 30% aqueous hydrogen peroxide (8 equivalent of

$H_2O_2$). The resulting mixture is stirred at 0° C for 3 h, then treated with 8 equivalents of 1.5 M aqueous sodium sulfite at 0° C. After 1 h, the mixture is acidified to pH 2 with 1.0 M aqueous $NaHSO_4$, and the product is isolated by extraction with ethyl acetate (3X). The combined organic extracts are washed with brine, dried ($MgSO_4$), filtered, and the filtrate concentrated under reduced pressure to give the crude product. Purification by column chromatography affords the desired product.

## Example 249

### (4R,5S)-4-Methyl-5-phenyloxazolidine-2-one.

Into a dry 300 ml single-necked, round-bottomed flask equipped for magnetic stirring was placed 40.2 g (0.27 mol) of (1S,2R)-2-amino-2-phenylpropanol, 36 ml (35.1 g, 0.30 mol) of diethyl carbonate, and ca. 2 g (~5 mol %) of anhydrous potassium carbonate. The flask was fitted for distillation through a 12" Vigreux column and placed in an oil bath pre-equilibrated to 150° C. The stirred solution was heated until ethanol distillation ceased (overnight). Upon cooling to room temperature, the contents of the flask solidified. The reaction product was dissolved in dichloromethane (200 ml), washed once with brine (50 ml), dried over anhydrous sodium sulfate, filtered, and concentrated in vacuo to give a colorless solid. Recrystallization from toluene afforded 40.8 g (85%) of the desired compound as a white, crystalline solid, mp 120-122° C (Lit.[39] mp 116-117° C). IR ($CHCl_3$) 3462, 3400-3100, 2990, 1755, 1400, 1388, 1350, 1235 $cm^{-1}$; $^1H$ NMR (90 MHz, $CDCl_3$) $\delta$ 7.30 (s,5H), 6.4 (br,1H), 5.67 (d,J = 8Hz, 3H), 4.20 (quintet,J = 7Hz,1H), 0.80 (d,J = 7Hz,3H); $^{13}C$ NMR ($CDCl_3$) $\delta$ 159.9, 135.0, 128.4, 81.0, 52.4, 17.4; $(a)_D$ + 177.2° (c2.21, $CHCl_3$) (Lit.[5] $(a)_D$ + 158.4° (c0.44, $CHCl_3$)).
Anal. Calcd. for $C_{10}H_{11}NO_2$: C, 67.78; H, 6.26; N, 7.90.
Found: C, 67.42; H, 6.19; N, 7.87.

## Example 250

### 5(S)-(3-(tert-Butyloxycarbonyl)-2,2-dimethyl-4(S)-benzyl-5(R)-oxazolidinyl)-4(R)-carboxy-1,3-dioxolan-2-one.

Using the procedures of Examples 244, 245, 246, 247, and 248 but replacing 4(S)-isopropyl-2-oxazolidinone with the resultant compound of Example 249 and replacing the resultant compound of Example 241 with the resultant compound of Example 242 gives the desired compound.

## Example 251

### 3-(Bromoacetyl)-4(S)-isopropyl-2-oxazolidinone.

Using the procedure of Example 244, 4(S)-isopropyl-2-oxazolidinone was acylated with bromoacetyl bromide to give the desired product. $^1H$ NMR ($CDCl_3$) $\delta$ 0.92 (d,3H), 0.95 (d,3H), 2.42 (m,1H), 4.27 (dd,1H), 4.35 (t,1H), 4.43 (d,1H), 4.47 (m,1H), 4.61 (d,1H). Mass spectrum: $(M+NH_4)^+$ = 267.

## Example 252

3-(Isothiocyanoacetyl)-4(S)-isopropyl-2-oxazolidinone.

Using the procedure of D.A. Evans and A.E. Weber (J. Am. Chem. Soc. **1986**, 108, 6757) the resultant compound from Example 251 was transformed into the desired compound. $^1$H NMR ($CDCl_3$) δ 0.90 (d,3H), 0.95 (d,3H), 2.47 (m,1H), 4.31 (dd,1H), 4.39 (t,1H), 4.48 (dt,1H), 4.84 (s,2H). Mass spectrum: $(M+NH_4)^+$ = 246.

Example 253

3-(5(S)-(3-(tert-Butyloxycarbonyl)-2,2-dimethyl-4(S)-cyclohexylmethyl-5(R)-oxazolidinyl)-2-thionooxazolidinyl)-4-(S)-carbonyl)(4S)-isopropyl-2-oxazolidinone.

The procedure of D.A. Evans and A.E. Weber (J. Am. Chem. Soc. **1987**, 109, 7151) is adapted. A solution of the resultant compound from Example 252 (1.25 g, 5.48 mmol) in 11 ml THF was added via cannula to a -78° C mixture of stannous triflate (2.49 g, 5.98 mmol) and N-ethyl piperidine (0.958 ml, 789 mg, 6.97 mmol) in 24 ml THF, under a nitrogen atmosphere. After stirring at -78° C for 1.5 h, a solution of the resultant compound from Example 241 (1.62 g, 4.98 mmol) in 11 ml THF was added dropwise to the mixture. Stirring was continued for 4 h at -78° C, at which time the reaction mixture was warmed to 0° C and quenched by the addition of 20 ml of pH 7 phosphate buffer. The slurry was filtered through Celite, and the solids were washed with 2 X 50 ml $CH_2Cl_2$. The filtrate was extracted with 4 X 100 ml $CH_2Cl_2$, and the combined organic extracts were washed with 100 ml 1 M aqueous $NaHSO_4$, then dried over $Na_2SO_4$. The filtrate was concentrated under reduced pressure, and the residue purified by column chromatography to afford 1.66 g (60%) of the desired compound. $^1$H NMR ($CDCl_3$) δ 0.91 (d,3H), 0.95 (d,3H), 0.9-1.75 (several br m,12H), 1.50 (s,9H), 1.52 (s,3H), 1.59 (s,3H), 1.86 (br d,1H), 2.34 (m,1H), 3.93 (br m,1H), 4.15 (br m,1H), 4.35 (m,1H), 4.42 (br d,1H), 4.48 (m,1H), 5.02 (br s,1H), 5.49 (br d,1H), 7.72 (br s,1H).

Example 254

5(S)-(3-tert-Butyloxycarbonyl)-2,2-dimethyl-4(S)-cyclohexylmethyl-5(R)-oxazolidinyl)-4(S)-methoxycarbonyl-2-thionooxazolidine.

Using the procedure of Example 224 with the resultant compound of Example 253 gives the desired compound.

Example 255

5(S)-(3-(tert-Butyloxocarbonyl)-2,2-dimethyl-4(S)-cyclohexylmethyl-5(R)-oxazolidinyl)-4(S)-methoxycarbonyl-2-oxazolidinone.

The procedure of D. Hoppe and R. Follmann (Chem. Ber. **1976**, 109, 3047) is adapted. A 1 M solution of the resultant compound from Example 254 in formic acid is cooled to 0° C and treated with 2 equivalents of 30% aqueous $H_2O_2$. After stirring at 0°-5° C for 30 min, the resulting mixture is carefully added to a stirred mixture of 2 M aqueous $Na_2CO_3$ and $CHCl_3$. Extractive workup is followed by drying over $Na_2SO_4$ and concentration of the filtrate under reduced pressure. Purification by column chromatography affords the desired compound.

Example 256

N-(Boc-Leu-Asn Amide of 5(R)-(2(S)-Amino-3-cyclohexyl-1(R)-hydroxypropyl)-1,3-dioxolan-2-on-4(S)-carbonyl)-Ile-Ser Methyl Ester.

The resultant compound of Example 248 is coupled to H-Ile-Ser-OMe according to the procedure of Example 66. The resulting compound from that coupling is deprotected according to the procedure of Example 2 and coupled to Boc-Leu-Asn-OH according to the procedure of Example 66 to give the desired compound.

Example 257

N-(Boc-Leu-Asn Amide of 5(S)-(2(S)-Amino-3-phenyl-1(R)-hydroxypropyl)-1,3-dioxolan-2-on-4(S)-carbonyl)-Ile-Ser Methyl Ester.

The resultant compound of Example 250 is coupled to H-Ile-Ser-OMe according to the procedure of Example 66. The resulting compound from that coupling is deprotected according to the procedure of Example 2 and coupled to Boc-Leu-Asn-OH according to the procedure of Example 66 to give the desired compound.

Example 258

Boc-Leu-Asn Amide of 5(S)-(2(S)-Amino-3-cyclohexyl-1(R)-hydroxypropyl)-4(S)-methoxycarbonyl)-2-thionooxazolidine.

The resultant compound of Example 254 is deprotected according to the procedure of Example 2 and coupled to Boc-Leu-Asn-OH according to the procedure of Example 66 to give the desired compound.

Example 259

Boc-Leu-Asn Amide of 5(S)-(2(S)-Amino-3-cyclohexyl-1(R)-hydroxypropyl)-4(S)-(methoxycarbonyl)-oxazolidine-2-one.

The resultant compound of Example 255 is deprotected according to the procedure of Example 2 and coupled to Boc-Leu-Asn-OH according to the procedure of Example 66 to give the desired compound.

Example 260

(N-Methyl-2,3-dihydroxypropylamino)carbonyl-Leu-Asn Amide of N-(3-Methylbutyl)-2-(2-amino-1-hydroxy-3-phenylpropyl)furan-3-carboxamide.

The resultant compound of Example 102 is deprotected according to the procedure of Example 2 and coupled to Cbz-Asn-OH according to the procedure of Example 66. After purification, the above compound is deprotected according to the procedure of Example 23 and coupled to the resultant compound of Example 39 using the procedure of Example 66 to give the desired compound.

Example 261

5-(Methoxycarbonyl)pentanoyl-Leu-Asn Amide of N-(3-Methylbutyl)-2-(2-amino-1-hydroxy-3-phenylpropyl)furan-3-carboxamide.

The resultant compound of Example 102 is deprotected according to the procedure of Example 2 and coupled to Cbz-Asn-OH according to the procedure of Example 66. After purification, the above compound is deprotected according to the procedure of Example 23 and coupled to the resultant compound of Example 41 using the mixed anhydride procedure of Example 4 to give the desired compound.

Example 262

5-Carboxypentanoyl-Leu-Asn Amide of N-(3-Methylbutyl)-2-(2-amino-1-hydroxy-3-phenylpropyl)furan-3-carboxamide.

The resultant compound of Example 261 is hydrolyzed according to the procedure of Example 9 to provide the desired compound.

Example 263

2-Methylpropanoyl-Leu-Asn Amide of N-(3-Methylbutyl)-2-(2-amino-1-hydroxy-3-phenylpropyl)furan-3-carboxamide.

The resultant compound of Example 102 is deprotected according to the procedure of Example 2 and coupled to Cbz-Asn-OH according to the procedure of Example 66. After purification, the above compound is deprotected according to the procedure of Example 23 and coupled to the resultant compound of Example 42 using the mixed anhydride procedure of Example 4 to give the desired compound.

Example 264

(2R)-2-Isobutyl-3-(N-methyl-N-(2-methoxyethoxymethoxyethyl)aminocarbonyl)propanoyl-Asn Amide of N-(3-Methylbutyl)-2-(2-amino-1-hydroxy-3-phenylpropyl)furan 3-carboxamide.

The resultant compound of Example 102 is deprotected according to the procedure of Example 2 and coupled to Cbz-Asn-OH according to the procedure of Example 66. After purification, the above compound

is deprotected according to the procedure of Example 23 and coupled to the resultant compound of Example 47 using the mixed anhydride procedure of Example 4 to give the desired compound.

Example 265

(3-Hydroxypropyloxycarbonyl)-Leu-Asn Amide of N-(3-Methylbutyl)-2-(2-amino-1-hydroxy-3-phenylpropyl)furan-3-carboxamide.

The resultant compound of Example 102 is deprotected according to the procedure of Example 2 and coupled to Cbz-Asn-OH according to the procedure of Example 66. After purification, the above compound is deprotected according to the procedure of Example 23 and coupled to the resultant compound of Example 59 using the procedure of Example 66 to give the desired compound.

Example 266

(2,3-Dihydroxypropyloxycarbonyl)-Leu-Asn Amide of N-(3-Methylbutyl)-2-(2-amino-1-hydroxy-3-phenylpropyl)-furan-3-carboxamide.

The resultant compound of Example 102 is deprotected according to the procedure of Example 2 and coupled to Cbz-Asn-OH according to the procedure of Example 66. After purification, the above compound is deprotected according to the procedure of Example 23 and coupled to the resultant compound of Example 64 using the procedure of Example 66 to give the desired compound.

Example 267

2-(3-Phenyl-1-propen-1-yl)cyclopentanecarboxylic Acid Methyl Ester.

To a stirred solution of 15.6 g (100 mmol) of 2-formyl-cyclopentanecarboxylic acid methyl ester (J. Med. Chem. **1982**, 25, 250-258) in 500 ml of THF at -78° C is added 25.2 g (100 mmol) of ((2-phenyl-1-lithioethyl)sulfonyl)benzene (Bull. Soc. Chim. Fr. **1976**, 513-518) in 100 ml of THF. After 30 m, 9.4 ml (100 mmol) of acetic anhydride is added. When the reaction mixture has warmed to room temperature, excess Na(Hg) is added. After 10 h, the solution is decanted and concentrated. The residue is dissolved in 500 ml of dichloromethane, washed with water, dried over $MgSO_4$, filtered, and concentrated under reduced pressure to afford the desired product.

Example 268

2-(3-Phenyl-1,2-epoxyprop-1-yl)cyclopentanecarboxylic Acid Methyl Ester.

To a solution of 24.4 g (100 mmol) of the resultant compound of Example 267 in 500 ml of THF is added 100 ml of 1 M aqueous LiOH. After 10 h at reflux, the solution is concentrated, redissolved in 500 ml of $CH_3CN$, and treated with 25.4 g of $I_2$. After 12 h, the reaction mixture is concentrated, redissolved in

methanol, and treated with 2.3 g of Na. After 24 h, the reaction mixture is concentrated under reduced pressure, dissolved in 500 ml of dichloromethane, washed with water, filtered, and concentrated under reduced pressure to afford the desired product.

## Example 269

### 2-(3-Phenyl-2-azido-1-hydroxyprop-1-yl)cyclopentanecarboxylic Acid Methyl Ester.

To a stirred solution of 26 g (100 mmol) of the resultant compound of Example 268 in 500 ml of DMF is added 14.7 g (300 mmol) of $LiN_3$ and the mixture heated to 150° C for 12 h. The reaction mixture is then concentrated under reduced pressure, and the residue dissolved in $CH_2Cl_2$, washed with a pH 6 phosphate buffer, dried over $MgSO_4$, and concentrated under reduced pressure. Chromatography of the residue on silica gel affords the desired product.

## Example 270

### 2-(3-Phenyl-2-t-butyloxycarbonylamino-1-hydroxyprop-1-yl)cyclopentanecarboxylic Acid Methyl Ester.

A stirred solution of 30.3 g (100 mmol) of the resultant compound of Example 269 in 500 ml of MeOH is hydrogenated over 1 g of 10% Pd-C. After 24 h, the reaction is degassed, the catalyst removed by filtration, and the filtrate treated with a slight excess of di-t-butyldicarbonate. Removal of the solvent and chromatography of the residue on silica gel affords the desired product.

## Example 271

### 2-(3-Phenyl-2-t-butyloxycarbonylamino-1-t-butyldimethylsilyloxyprop-1-yl)cyclopentanecarboxylic Acid Methyl Ester.

To a stirred solution of 37.7 g (100 mmol) of the resultant compound of Example 270 in 500 ml of dichloromethane is added 23.0 ml of t-butyldimethylsilyl trifluoromethane sulfonate and 12.8 ml of 2,6-lutidine. After 6 h, the solution is washed with water, dried over $MgSO_4$, and concentrated. Chromatography of the residue on silica gel affords the desired product.

## Example 272

### 2-(3-(4-Benzyloxyphenyl)-2-t-butyloxycarbonylamino1-t-butyldimethylsilyloxyprop-1-yl)cyclopentanecarboxylic Acid Methyl Ester.

Using the procedures of Examples 267, 268, 269, 270, and 271 but substituting ((2-(4-benzyloxyphenyl)-1-lithioethyl)sulfonyl)benzene for ((2-phenyl-1-lithioethyl)sulfonyl)benzene affords the desired product.

Example 273

2-(3-Phenyl-2-t-butyloxycarbonylamino-1-t-butyldimethylsilyloxyprop-1-yl)cyclopropanecarboxylic Acid Methyl Ester.

Using the procedures of Examples 267, 268, 269, 270, and 271 but substituting 2-formylcyclopropanecarboxylic acid methyl ester for 2-formylcyclopentanecarboxylic acid methyl ester affords the desired product.

Example 274

2-(3-Phenyl-2-t-butyloxycarbonylamino-1-t-butyldimethylsilyloxyprop-1-yl)cyclohexanecarboxylic Acid Methyl Ester.

Using the procedures of Examples 267, 268, 269, 270, and 271 but substituting 2-formylcyclohexanecarboxylic acid methyl ester for 2-formylcyclopentanecarboxylic methyl ester affords the desired product.

Example 275

2-(3-Phenyl-2-t-butyloxycarbonylamino-1-t-butyldimethylsilyloxyprop-1-yl)cyclobutanecarboxylic Acid Methyl Ester.

Using the procedures of Examples 267, 268, 269, 270, and 271 but substituting 2-formylcyclobutanecarboxylic acid methyl ester for 2-formylcyclopentanecarboxylic acid methyl ester affords the desired product.

Example 276

2-(3-Phenyl-2-t-butyloxycarbonylamino-1-t-butyldimethylsilyloxyprop-1-yl)benzenecarboxylic Acid.

To a stirred solution of 2.01 g (10 mmol) of 2-bromobenzoic acid in 100 ml of THF at -100° C is added 20 ml of 1 M n-BuLi in hexane. After 15 m, a solution of 2.49 g (10 mmol) N-t-butyloxycarbonylphenylalaninal is added, and the solution allowed to warm to -78° C. After 1 h, 4.59 ml (20 mmol) of t-butyldimethylsilyl trifluoromethane sulfonate is added, and the solution gradually allowed to warm to room temperature. Addition of saturated aqueous NaCl acidified to pH 4 with HCl, separation of the organic phase, drying over $Na_2SO_4$, and concentration affords the desired product.

Example 277

2-(3-Phenyl-2-t-butyloxycarbonylamino-1-t-butyldimethylsilyloxyprop-1-yl)cyclopent-1-enecarboxylic Acid.

Using the procedure described in Example 276, but substituting 2-bromocyclopent-1-enecarboxylic acid for 2-bromobenzoic acid affords the desired product.

Example 278

2-(3-Phenyl-2-t-butyloxycarbonylamino-1-t-butyldimethylsilyloxyprop-1-yl)cyclohex-1-enecarboxylic Acid.

Using the procedure described in Example 276, but substituting 2-bromocyclohex-1-enecarboxylic acid for 2-bromobenzoic acid affords the desired product.

Example 279

6-Phenyl-5-t-butyloxycarbonylamino-4-t-butyldimethylsilyloxy-2-hexenoic Acid.

Using the procedure described in Example 276, but substituting 2-bromopropenoic acid for 2-bromobenzoic acid affords the desired product.

Example 280

2-(3-(4-Benzyloxy-1-phenyl)-2-t-butoxycarbonylamino-1-t-butyldimethylsilyloxyprop-1-yl)cyclopent-1-enecarboxylic Acid.

Using the procedure described in Example 277, but substituting N-t-butyloxycarbonyl-0-benzyltyrosinal for N-t-butyloxycarbonylphenylalaninal affords the desired product.

Example 281

2-(3-Phenyl-2-t-butyloxycarbonylamino-1-azidoprop-1-yl)cyclopentanecarboxylic Acic Methyl Ester.

To a stirred solution of 37.7 g (100 mmol) of the resultant compound of Example 270 in 500 ml of $CH_2Cl_2$ at 0° C is added 13.9 ml (100 mmol) of triethylamine and 7.73 ml (100 mmol) of methanesulfonyl chloride. After 1 h, the reaction is washed with a pH 3 buffer, dried over $MgSO_4$, concentrated, and redissolved in DMF containing 14.7 g (100 mmol) of $LiN_3$. After heating at 150° C for 12 h, the cooled reaction mixture is concentrated, the residue dissolved in $CH_2Cl_2$, washed with water, and concentrated. Chromatography of the residue on silica gel affords the desired product.

Example 282

((4-Morpholinyl)carbonyl)-Leu-Asn-Amide of 2-(3-Phenyl-2-amino-1-hydroxyprop-1-yl)cyclopentanecarboxylic Acic Methyl Ester.

The resultant compound of Example 271 is deprotected according to the procedure of Example 2 and coupled to Cbz-asparagine using the carbodiimide coupling procedure of Example 66. The resulting compound is then hydrogenolyzed according to the procedure of Example 23 and then coupled to the resultant compound of Example 9 using the mixed anhydride method of Example 4 to provide the desired compound.

Example 283

Cbz-((β,β-di-Me)β-Ala)-Leu-Asn-Amide of 2-(3-(4-hydroxyphenyl)-2-amino-1-hydroxyprop-1-yl)-cyclopentanecarboxylic Acic Methyl Ester.

The resultant compound of Example 272 is deprotected according to the procedure of Example 2 and coupled to Cbz-asparagine using the carbodiimide coupling procedure of Example 66. The resulting compound is then hydrogenolyzed according to the procedure of Example 23 and then coupled to the resultant compound of Example 12 using the mixed anhydride method of Example 4 to provide the desired compound.

Example 284

((β,β-di-Me)β-Ala)-Leu-Asn-Amide of 2-(3-Phenyl-2-amino-1-hydroxyprop-yl)cyclopropanecarboxylic Acid Methyl Ester.

The resultant compound of Example 273 is deprotected according to the procedure of Example 2 and coupled to Cbz-asparagine using the carbodiimide coupling procedure of Example 66. The resulting compound is then hydrogenolyzed according to the procedure of Example 23 and then coupled to the resultant compound of Example 12 using the mixed anhydride method of Example 4. The resultant compound is then hydrogenolyzed according to the procedure of Example 23 to afford the desired compound.

Example 285

(N,N-Dimethyl-Gly)-Leu-Asn-Amide of 2-(3-Phenyl-2-amino-1-hydroxyprop-1-yl)cyclohexanecarboxylicAcid Methyl Ester.

The resultant compound of Example 274 is deprotected according to the procedure of Example 2 and coupled to Cbz-asparagine using the carbodiimide coupling procedure of Example 66. The resulting compound is then hydrogenolyzed according to the procedure of Example 23 and then coupled to the resultant compound of Example 54 using the procedure of Example 66 to provide the desired compound.

Example 286

N-((N-Cbz-4-piperidinyl)carbonyl)-Leu-Asn Amide of 2-(3-Phenyl-2-amino-1-hydroxyprop-1-yl)-cyclobutanecarboxylic Acid Methyl Ester.

The resultant compound of Example 275 is deprotected according to the procedure of Example 2 and coupled to Cbz-asparagine using the carbodiimide coupling procedure of Example 66. The resulting compound is then hydrogenolyzed according to the procedure of Example 23 and then coupled to the resultant compound of Example 56 using the mixed anhydride method of Example 4 to provide the desired compound.

## Example 287

N-(4-Piperidinyl)carbonyl)-Leu-Asn Amide of 2-(3-Phenyl-2-amino-1-hydroxyprop-1-yl)benzenecarboxylic Acid.

The resultant compound of Example 276 is deprotected according to the procedure of Example 2 and coupled to Cbz-asparagine using the carbodiimide coupling procedure of Example 66. The resulting compound is then hydrogenolyzed according to the procedure of Example 23 and then coupled to the resultant compound of Example 56 using the mixed anhydride method of Example 4. The resulting compound is then hydrogenolyzed according to the procedure of Example 23. Treatment of the resulting compound under the conditions of Example 9 effects hydrolysis to afford the desired compound.

## Example 288

3-Benzyloxypropanoyl-Leu-Asn Amide of 2-(3-Phenyl-2-amino-1-hydroxyprop-1-yl)cyclopent-1-enecarboxylic Acid

The resultant compound of Example 277 is deprotected according to the procedure of Example 2 and coupled to Cbz-asparagine using the carbodiimide coupling procedure of Example 66. The resulting compound is then hydrogenolyzed according to the procedure of Example 23 and then coupled to the resultant compound of Example 63 using the mixed anhydride procedure of Example 4. Treatment of the resulting compound under the conditions of Example 9 effects hydrolysis to provide the desired compound.

## Example 289

3-Hydroxypropanoyl-Leu-Asn Amide of 2-(3-Phenyl-2-amino-1-hydroxyprop-1-yl)cyclohex-1-enecarboxylic Acid.

The resultant compound of Example 278 is deprotected according to the procedure of Example 2 and coupled to Cbz-asparagine using the carbodiimide coupling procedure of Example 66. The resulting compound is then hydrogenolyzed according to the procedure of Example 23 and then coupled to the resultant compound of Example 63 using the mixed anhydride method of Example 4. Hydrogenolysis of the resulting compound according to the procedure of Example 23 followed by treatment under the conditions of Example 9 effects hydrolysis to afford the desired compound.

## Example 290

Boc-Gln-Ala Amide of 6-Phenyl-5-amino-4-hydroxy-2-hexenoic Acid.

The resultant compound of Example 279 is deprotected according to the procedure of Example 2 and coupled to Boc-alanine using the mixed anhydride coupling procedure of Example 4. The resulting compound is then deprotected according to the procedure of Example 2 and then coupled to Boc-glutamine using the carbodiimide coupling procedure of Example 66. Treatment of the resulting compound under the conditions of Example 9 effects hydrolysis to afford the desired compound.

Example 291

Boc-Leu-Asn Amide of 2(3-(4-Hydroxyphenyl)-2-amino-1-hydroxyprop-1-yl)cyclopent-1-enecarboxylic Acid.

The resultant compound of Example 280 is deprotected according to the procedure of Example 2 and coupled to the resultant compound of Example 3 using the carbodiimide coupling procedure of Example 66. Treatment of the resulting compound under the conditions of Example 9 effects hydrolysis to afford the desired compound.

Example 292

Boc-Leu-Asn-3-Amide of 2-(3-Phenyl-2,3-diaminoprop-1-yl)cyclopentanecarboxylic Acid Methyl Ester.

The resultant compound of Example 281 is deprotected according to the procedure of Example 2 and coupled to Boc-Leu-Asn-OH using the carbodiimide coupling procedure of Example 66. Hydrogenolysis under the conditions of Example 23 affords the desired compound.

Example 293

Cbz-Leu-Asn-Amide of Phenylalaninal

To a stirred solution of 5.02 g (15.2 mmol) of Asn-Phe-OMe hydrochloride (Yajima, H.; Kiso, Y.; Kitagawa, K.; Chem. Pharm. Bull. **1974**, 22, 1079-1086) in 100 ml of dichloromethane was added 2.12 ml (15.2 mmol) of triethylamine and 5.88 g of Cbz-Leu-p-nitrophenyl ester. After 36 hours, the solid product was filtered from the reaction mixture and washed with several portions of dichloromethane. An 824 mg (1.52 mmol) portion of the resulting white solid was dissolved in 48 ml of ethanol and 80 ml of tetrahydrofuran and treated with 260 mg (6.88 mmol) of sodium borohydride. After 8 h an additional 55 mg of sodium borohydride was added, and then the reaction mixture was cooled to -5 °C. After 14 h, the reaction mixture was neutralized by the addition of a dry sulfonic acid resin, filtered, and evaporated under reduced pressure. A solution of the residue in methanol was adsorbed onto silica gel by evaporation of the slurry, and the resulting dry powder was poured onto the top of a silica gel column packed in chloroform. Elution of the column with a chloroform:methanol gradient afforded 200 mg of Z-Leu-Asn-amide of phenylalaninol as a white solid. To a solution of 152 mg (0.296 mmol) of this alcohol in 4 ml of dry DMSO was added 170 mg (0.887 mmol) of 1-ethyl-3-(3-dimethylaminopropyl)carbodiimide hydrochloride followed by 0.012 ml (0.15 mmol) of dichloroacetic acid. After 2 hours at room temperature, the reaction mixture was added to 40 ml of rapidly stirred water. The resulting white precipitate was removed by filtration and washed several times with water and then dried in vacuo to afford 120 mg of Z-Leu-Asn-amide of

phenylalaninal as the aldehyde hydrate. $^1$H NMR ($d_6$-DMSO, TMS) δ 8.06 (d,1H), 7.48 (d,2H), 7.4-7.1 (m,11H), 6.92 (br s,1H), 5.90 (d,1H), 5.71 (d,1H), 5.00 (q,2H), 4.70 (m,1H), 4.50 (m,1H), 4.02 (m,1H), 3.83 (m,1H), 2.85 (m,1H), 2.65-2.35 (m,3H), 1.60 m,1H), 1.40 (m,2H), 0.85 (t,6H). After three days in $d_6$-DMSO, the hydrate is converted to the aldehyde form, δ 9.40 (s,1H,CHO). Mass spectrum: $(M+H)^+$ = 511.

## Example 294

### Cbz-Leu-Asn Amide of 5(S)-(2(S)-Amino-3-cyclohexyl-1(R)-hydroxy)-3(S)-methyl-2,3,4,5-tetrahydrofuran-2-one.

The resultant compound of Example 106 was deprotected according to the procedure of Example 2 and coupled to Cbz-Leu-Asn-OH according to the procedure of Example 66 to provide, after silica gel chromatography using 5% methanol in chloroform, a 70% yield of the desired compound. $^1$H NMR ($d_6$-DMSO) d 0.6-1.8 (br envelope), 0.85 (m,6H), 1.11 (d,J=7Hz,3H), 2.2-2.7 (br envelope), 3.50 (m,1H), 4.00 (m,2H), 4.21 (m,1H), 4.51 (m,1H), 5.02 (m,2H), 5.34 m,1H), 6.89 (br s,1H), 7.03 (br d,J=10Hz,1H), 7.36 (m,6H), 7.48 (br d,J=8Hz,1H), 8.31 (br d,J=8Hz,1H). Mass spectrum: $(M+H)^+$ = 617.

## Example 295

### N-(5-(Methoxycarbonyl)pentanoyl)-Leu-Asn Amide of 5(S)-(2(S)-Amino-3-cyclohexyl-1(R)-hydroxy)-3(S)-methyl-2,3,4,5-tetrahydrofuran-2-one.

The resultant compound of Example 294 (0.51 g, 0.83 mmol) was added to a mixture of 0.06 g of 10% palladium on carbon in 15 ml of methanol and stirred under 1 atmosphere of $H_2$ for 24 h. The mixture was filtered through Celite and concentrated to the crude amine, a portion (0.30 g, 0.62 mmol) of which was taken up in 10 ml of dichloromethane and 0.5 ml of dimethylformamide, treated with 0.20 g (0.80 mmol) of N-(5-(methoxycarbonyl)pentanoyloxy)succinimide, and stirred at ambient temperature for 16 h. The resulting mixture was concentrated in vacuo, taken up in ethyl acetate, washed sequentially with 10% citric acid, water, aqueous $NaHCO_3$, and saturated brine, dried over $MgSO_4$, and concentrated. Silica gel chromatography using 5% methanol in chloroform gave 0.17 g (45%) of the desired compound. $^1$H NMR ($d_6$-DMSO) δ 0.7-1.8 (br envelope), 0.83 (d,J=7Hz,3H), 0.87 (d,J=7Hz,3H), 1.11 (d,J=7Hz,3H), 2.1-2.7 (br envelope), 3.51 (m,1H), 3.58 (s,3H), 3.98 (m,1H), 4.24 (m,2H), 4.48 (q,J=7Hz,1H), 5.33 (br d,J=6Hz,1H), 6.90 (br s,1H), 7.00 (br d,J=9Hz,1H), 7.37 (br s,1H), 8.04 (br d,J=8Hz,1H), 8.32 (br d,J=8Hz,1H). Mass spectrum: $(M+H)^+$ = 625.

## Example 296

### A. 2-(t-Butyloxycarbonylamino)-1,5-diphenylpent-3-ene.

A suspension of 20 g (53 mmol) of phenyl 2-(t-butyloxycarbonylamino)-3-phenylpropyl sulfone (Tetrahedron Lett. **1986**, 27, 2095) in 500 ml of dry tetrahydrofuran was cooled under inert atmosphere to -78°C and treated with 65 ml (135 mmol) of n-butyllithium. The resulting suspension was warmed to 0°C and stirred for 5 min during which the solution became homogenous. After being recooled to -78°C, the solution was treated with 15 ml (130 mmol) of phenylacetaldehyde, stirred for 40 min, and quenched with 10% aqueous citric acid. The mixture was extracted with ether, filtered, washed with three portions of aqueous sodium bisulfite, concentrated in vacuo, dissolved in chloroform, dried over $MgSO_4$, and concentrated in vacuo. The crude residue was taken up in 500 ml of dry dichloromethane, treated sequentially

with 0.8 g (7.3 mmol) of 4-dimethylaminopyridine, 13 ml (93 mmol) of triethylamine, and 7.5 ml (80 mmol) of acetic anhydride. The resulting solution was stirred for 2 h at ambient temperature, treated with water, stirred for 10 min, washed sequentially with 10% aqueous citric acid and aqueous $NaHCO_3$, dried over $MgSO_4$, and concentrated in vacuo. The resulting crude mixture was taken up in 500 ml of methanol, cooled to 0°C, and treated with 150 g (0.32 mol) of 5% sodium amalgam. After 2 h, an additional 100 g (0.21 mol) of sodium amalgam was added and the mixture was stirred at ambient temperature for 1 h. The resulting suspension was treated with ether and aqueous ammonium chloride and stirred until the mercury became a freely flowing pool. The mixture was extracted with ether, filtered, washed sequentially with water and saturated brine, and concentrated in vacuo. The residue was taken up in dichloromethane, dried over $MgSO_4$, and concentrated to a yellow oil. Purification by flash chromatography using 1:1 dichloromethane:hexane gave 9.31 g ((52%) of the desired compound (4:1 mixture of geometric isomers) as an oil which solidified upon standing. $^1H$ NMR ($CDCl_3$, major isomer) 1.40 (s, 9 H), 2.7-2.9 (m, 2 H), 3.32 (d, J = 7 Hz, 2 H), 4.4 (br, 2 H), 5.43 (dd, J = 15, 6 Hz, 1 H), 5.64 (dt, J = 15, 7 Hz, 1 H), 7.0-7.3 (m, 10 H). Mass spectrum $(M+1)^+$ = 338.

B. Acetyl-valinyl-valine.

A solution of 1.0 g (4.6 mmol) of valinyl-valine in 40 ml of water and 4 ml of saturated aqueous $NaHCO_3$ was cooled to 0°C, treated with 0.2 ml of acetic anhydride, stirred for 15 min, treated with an additional 0.2 ml portion of acetic anhydride, stirred for 1 h, and treated with an additional 0.2 ml portion of acetic anhydride. The resulting solution was stirred overnight at ambient temperature, concentrated in vacuo, acidified with 6 N HCl, extracted with 10% methanol in chloroform, dried over $MgSO_4$, and concentrated in vacuo to give 1.02 g (86%) of the desired compound. $^1H$ NMR (DMSO-$d_6$) 0.87 (m, 12 H), 1.86 (s, 3 H), 1.93 (heptet, J = 7 Hz, 1 H), 2.03 (heptet, J = 7 Hz, 1 H), 4.09 (dd, J = 8, 6 Hz, 1 H), 4.27 (dd, J = 9, 7 Hz, 1 H), 7.86 (d, J = 9 Hz, 1 H), 7.94 (d, J = 8 Hz, 1 H).

C. 2-(N-(N-Acetyl-valyl-valyl)amino)-1,5-diphenylpent-3-ene.

The resultant compound of Example 296A (160 mg, 0.47 mmol) was deprotected according to the procedure of Example 2 and coupled to Ac-Val-Val-OH according to the procedure of Example 66 to give a crude product which was triturated with ether, filtered, and air-dried to give 111 mg (49%) of the desired compound. Mass spectrum $(M+1)^+$ = 478.

D. 2-(N-(N-Acetyl-valyl-valyl)amino)-3,4-dihydroxy-1,5-diphenylpentane.

Using the procedure of Example 141 with the resultant compound of Example 296C gave the desired compound.

Example 297

A. 1-(2-Furanyl)-1-hydroxy-3-phenyl-2-(triphenylmethyl)aminopropane.

A solution of 18 mmol of n-butyllithium in 40 ml of tetrahydrofuran/hexane was cooled to -78°C and treated with 1.3 ml (18 mmol) of furan. The resulting solution was stirred at -23°C for 4 h, recooled to -78°C, and treated with a solution of 2.35 g (6 mmol) of N-(triphenylmethyl)-phenylalaninal in 20 ml of tetrahydrofuran. After stirring for 10 min, the solution was allowed to warm for 10 min, quenched with aqueous ammonium chloride, extracted with ether, dried over $MgSO_4$, and concentrated in vacuo. Flash chromatography using 20% ethyl acetate in hexane gave 2.12 g (77%) of the desired compound as a 2.2:1

mixture of isomers.

B. 2-(t-Butyloxycarbonyl)amino-1-(2-tetrahydrofuranyl)-1-hydroxy-3-phenylpropane.

Using the procedure of Example 23 with 1.20 g (2.6 mmol) of the resultant compound of Example 297A gave a crude amine, which was taken up in 10 ml of dichloromethane and treated with 1.2 g (5.2 mmol) of di-t-butyldicarbonate. After 6 h, the solution was concentrated, and the residue was purified by flash chromatography using 30% ethyl acetate in hexane to give 0.2 g (24%) of the desired compound. $^1$H NMR ($CDCl_3$) 1.40 (s, 9 H), 1.7-2.0 (m, 4 H), 2.69 (s, 1 H), 2.86 (dd, J = 14, 9 Hz, 1 H), 2.94 (dd, J = 14, 7 Hz, 1 H), 3.26 (d, J = 9 Hz, 1 H), 3.7-3.9 (m, 4 H), 5.03 (br d, J = 9 Hz, 1 H), 7.25 (m, 5 H). Mass spectrum $(M+1)^+$ = 322.

C. Cbz-Leu-Asn Amide of 2-amino-1-(2-tetrahydrofuranyl)-1-hydroxy-3-phenylpropane.

The resultant compound of Example 297B (26 mg, 0.08 mmol) was deprotected according to the procedure of Example 2 and coupled to Cbz-Leu-Asn-OH according to the procedure of Example 66 to give, after purification by flash chromatography using 6.5% methanol in chloroform, 34 mg (72%) of the desired compound, m.p. 198-200 °C. Mass spectrum $(M+1)^+$ = 583.
Anal. Calcd. for $C_{31}H_{42}N_4O_7$: C, 63.90; H, 7.26; N,9.62. Found: C, 63.60; H, 7.27; N, 9.54.

## Example 298

A. N-(Acetyl-valinyl-valinyl)-phenylalaninol.

Using the procedure of Example 66, Ac-Val-Val-OH was coupled to phenylalaninol. The crude product was filtered, washed with dichloromethane, and dried in vacuo to give 146 mg (64%) of the desired compound as a white solid. $^1$H NMR (DMSO-$d_6$) 0.79 (m, 12 H), 1.86 (s, 3 H), 1.90 (m, 2 H), 2.60 (dd, J = 14, 8 Hz, 1 H), 2.88 (dd, J = 14, 5 Hz, 1 H), 3.25 (m, 2 H), 3.91 (m, 1 H), 4.05 (br t, 1 H), 4.15 (br t, 1 H), 4.75 (t, J = 4 Hz, 1 H), 7.20 (m, 5 H), 7.68 (d, J = 9 Hz, 1 H), 7.89 (d, J = 9 Hz, 1H).

B. N-(Acetyl-valinyl-valinyl)-phenylalaninal.

A solution of 0.05 ml of dimethylsulfoxide in 1 ml of dichloromethane was cooled to -63 °C, treated with 0.1 ml (0.2 mmol) of oxalyl chloride (2 M in dichloromethane), stirred for 10 min, and treated with a solution of 30 mg (0.077 mmol) of the resultant compound of Example 298A in 0.7 ml of dimethylsulfoxide and 0.5 ml of dichloromethane. After being allowed to stir for 40 min, the solution was treated with 0.085 ml of triethylamine, stirred for 10 min, and quenched by pouring into aqueous citric acid. The product was extracted with 10% methanol in chloroform, dried over $MgSO_4$, and concentrated. Purification by flash chromatography using 7.5% methanol in chloroform gave 14 mg (48%) of the desired compound, m.p. 213-220 °C (dec). Mass spectrum $(M+1)^+$ = 390.
Anal. Calcd. for $C_{21}H_{31}N_3O_4 \cdot 0.5\ H_2O$: C, 63.30; H, 8.09; N, 10.54. Found: C, 63.19; H, 8.12; N, 10.18.

## Example 299

A. 4-Benzyl-3-(t-butyloxycarbonyl)-2,2-dimethyl-5-(hydroxymethyl)-oxazolidine.

A precooled (0°C) solution of sodium borohydride (40 mg, 1.05 mmol) in 2 ml of methanol was treated with a solution of 218 mg (0.68 mmol) of the resultant compound of Example 242 in 3 ml of 1:1 methanol:tetrahydrofuran. After being allowed to stir at ambient temperature for 2 h, the solution was quenched with aqueous $NH_4Cl$, extracted with ether, washed with 1 N NaOH and saturated brine, dried over $MgSO_4$, and concentrated. Flash chromatography using 25% ethyl acetate in hexane gave 199 mg (91%) of the desired compound. $^1H$ NMR ($CDCl_3$) 1.53 (s, 9 H), 1.54 (s, 6 H), 1.71 (t, J = 6 Hz, 1 H), 2.75 (br, 1 H), 3.2-3.4 (m, 3 H), 3.92 (br, IH), 4.02 (m, 1 H), 7.2-7.3 (m, 5 H). Mass spectrum: $(M+H)^+$ = 322.

B. Cbz-Leu-Asn Amide of 3-Amino-1,2-dihydroxy-4-phenylbutane.

The resultant compound of Example 299A was deprotected according to the procedure of Example 2 and coupled to Cbz-Leu-Asn-OH according to the procedure of Example 66 to give, after trituration with 2% methanol in chloroform, filtration, washings with chloroform, and air-drying, the desired compound (41 mg, 48%) as a white solid, m.p. 214-215°C. Mass spectrum: $(M+H)^+$ = 543.
Anal. Calcd. for $C_{28}H_{38}N_8O_7 \cdot 0.5\ H_2O$: C, 60.97; H, 7.13; N, 10.16. Found: C, 61.27; H, 6.99; N, 10.14.

Example 300

A. (cis)-Methyl 3-(4-Benzyl-3-(t-butyloxycarbonyl)-2,2-dimethyl-oxazolidin-5-yl)propenoate.

A suspension of 0.21 g (5.2 mmol) of sodium hydride (60% dispersion in mineral oil) in 15 ml of dry tetrahydrofuran was cooled to 0°C, treated with 1.52 g (4.8 mmol) of bis(2,2,2-trifluoroethyl)-(methoxycarbonylmethyl)phosphonate in 5 ml of tetrahydrofuran, stirred for 10 min at 0°C, treated with a solution of 1.0 g (3.13 mmol) of the resultant compound of Example 242 in 5 ml of tetrahydrofuran, and stirred at ambient temperature for 1 h. The resulting solution was quenched with aqueous $NH_4Cl$, extracted with ether, washed with saturated brine, dried over $MgSO_4$, and concentrated. Flash chromatography using 15% ethyl acetate in hexane gave 0.82 g (69%) of the desired compound.

B. (cis)-3-(4-Benzyl-3-(t-butyloxycarbonyl)-2,2-dimethyl- oxazolidin-5-yl)propenoic Acid.

A solution of 218 mg (0.58 mmol) of the resultant compound of Example 300A in 4.6 ml of dioxane was treated with 2.3 ml (1.2 mmol) of 0.5M aqueous lithium hydroxide. After 2 h, the solution was diluted with chloroform, acidified with 1 N HCl, partitioned, and the aqueous layer was washed with additional chloroform. The combined organic layers were dried over $MgSO_4$ and concentrated to give the desired compound as a colorless oil.

C. (cis)-N-(3-Methylbutyl)-3-(4-benzyl-3-(t-butyloxycarbonyl)-2,2-dimethyl-oxazolidin-5-yl)propenamide.

According to the mixed anhydride procedure of Example 4, the resultant compound of Example 300B was coupled to isoamylamine to give 251 mg (100%) of the desired compound as a colorless oil. $^1H$ NMR ($CDCl_3$) δ 0.93 (d, J = 7 Hz, 6 H), 1.41 (t, J = 7 Hz, 2 H), 1.51 (s, 9 H), 1.57 (s, 6 H), 1.62 (heptet, J = 7 Hz, 1 H), 2.9-3.4 (m, 4 H), 3.96 (m, 1 H), 5.20 (m, 1 H), 5.7-6.1 (m, 3 H), 7.15-7.3 (m, 5 H). Mass spectrum: $(M+H)^+$ = 431.

D. (cis)-N-(3-Methylbutyl)-5-amino-4-hydroxy-6-phenyl-2-hexenamide.

A solution of 48 mg (0.11 mmol) of the resultant compound of Example 300C in 1 ml of dichloromethane was treated with 1 ml of trifluoroacetic acid. After being stirred for 1.75 h, the solution was concentrated in vacuo, taken up in 3 ml of 2:1 tetrahydrofuran:water, stirred for 45 min, treated with solid $K_2CO_3$, extracted with chloroform, dired over $MgSO_4$, and concentrated to give 21 mg (64%) of the desired compound. $^1H$ NMR ($CDCl_3$) $\delta$ 0.92 (d, J = 7 Hz, 6 H), 1.43 (m, 2 H), 1.63 (heptet, J = 7 Hz, 1 H), 2.9-3.1 (m, 3 H), 3.33 (m, 2 H), 4.50 (m, 1 H), 5.36 (dd, J = 12, 2 Hz, 1 H), 5.43 (br, 1 H), 6.22 (dd, J = 12, 6 Hz, 1 H), 7.2-7.35 (m, 5 H). Mass spectrum: $(M+H)^+$ = 291.

E. Cbz-Leu-Asn Amide of (cis)-N-(3-Methylbutyl)-5-amino-4-hydroxy-6-phenyl-2-hexenamide.

Using the procedure of Example 66, Cbz-Leu-Asn-OH was coupled to the resultant compound of Example 300D to give, after flash chromatography using 7.5% methanol in chloroform, 23 mg (49%) of the desired compound as a white solid, m.p. 196-198°C (dec). Mass spectrum: $(M+H)^+$ = 652.
Anal. Calcd. for $C_{33}H_{49}N_5O_7$: C, 64.50; H, 7.58; N, 10.74. Found: C, 64.16; H, 7.55; N, 10.67.

## Example 301

A. (cis)-3-(4-Benzyl-3-(t-butyloxycarbonyl)-2,2-dimethyl- oxazolidin-5-yl)propenoyl-Ile-Ser-Pro-Ile-Methyl Ester.

According to the procedure of Example 4, the resultant compound of Example 300B was coupled to H-Ile-Ser-Pro-Ile-$OCH_3$ to give the desired compound in 100% yield. Mass spectrum: $(M+H)^+$ = 786.

B. Cbz-Leu-Asn Amide of (cis)-5-amino-4-hydroxy-6-phenyl- 2-hexenoyl-Ile-Ser-Pro-Ile Methyl Ester.

Using the procedures of Example 300D and 300E with the resultant compound of Example 301A gave, after flash chromatography using 8.5% methanol in chloroform, the desired compound (20 mg) in 33% yield, m.p. 210-215°C (dec). Mass spectrum: $(M+Na)^+$ = 1029.

## Example 302

A. 1-(4,5-Dihydrofuran-2-yl)-1-hydroxy-3-phenyl-2-(triphenylmethyl)aminopropane.

Dihydrofuran (16 ml) was cooled under inert atmosphere to -78°C and treated dropwise with 4.5 ml (7.5 mmol) of tert-butyllithium in cyclohexane. The resulting suspension was treated with 9 ml of dry tetrahydrofuran, allowed to warm to 0°C for 20 min, recooled to -78°C, and treated with a solution of 1.0 g (2.5 mmol) of N-(triphenylmethyl)- phenylalaninal in 10 ml of tetrahydrofuran. The resulting solution was stirred at -78°C for 10 min, warmed to 0°C for 5 min, and quenched cautiously with aqueous ammonium chloride. The product was extracted with ether, dried over $MgSO_4$, and concentrated in vacuo to give the desired compound as a crude foam.

B. 2-(2-Amino-1-hydroxy-3-phenyl)propyl-2-hydroxy-2,3,4,5-tetrahydrofuran.

The resultant compound of Example 302A (1.37 g, 2.5 mmol) was treated with 50 ml of tetrahydrofuran and 50 ml of 1 N HCl and heated at reflux for 0.5 h. After being allowed to cool, the mixture was concentrated in vacuo, taken up in dilute aqueous HCl, washed with three portions of hexane, concentrated in vacuo, taken up in methanol/chloroform, and concentrated to a brown foam.

C. Boc-Phe-Leu Amide of 2-(2-Amino-1-hydroxy-3-phenyl)propyl-2-hydroxy-2,3,4,5-tetrahydrofuran.

The resultant compound of Example 302B and 0.91 g (1.9 mmol) of N-(Boc-phenylalaninyl leucinyloxy)-succinimide in 50 ml of dichloromethane were treated with 1.0 ml (9 mmol) of 4-methylmorpholine. After being stirred for 3.5 h, the solution was diluted with ethyl acetate, washed sequentially with aqueous ammonium hydroxide, 10% aqueous citric acid, aqueous $NaHCO_3$, and saturated brine; dried over $MgSO_4$, and concentrated. The product was purified by flash chromatography using 50% ethyl acetate in hexane to give 0.33 g (29%) of the desired compound, m.p. 110-113°C (dec). Mass spectrum: $(M+H-H_2O)^+$ = 580. Anal. Calcd. for $C_{33}H_{47}N_3O_7$: C, 66.31; H, 7.92; N, 7.03. Found: C, 67.31; H, 7.78; N, 6.80.

Example 303

A. N-Methyl-2-(1-hydroxy-3-phenyl-2-(triphenylmethylamino)propyl-benzamide.

A solution of 0.29 g (2.1 mmol) of N-methylbenzamide in 15 ml of tetrahydrofuran was cooled under inert atmosphere to -78°C and treated with 2.1 ml (4.4 mmol) of n-butyllithium. The resulting solution was warmed to 0°C for 30 min, recooled to -78°C, and treated with a solution of 0.42 g (1.1 mmol) of N-(triphenylmethyl)phenylalaninal in 5 ml of tetrahydrofuran. After stirring for 5 min, the solution was quenched with aqueous ammonium chloride, extracted with ether, dried over $MgSO_4$, and concentrated in vacuo. Flash chromatography using 40% ethyl acetate in hexane gave 0.24 g (42%) of the desired compound. $^1H$ NMR ($CDCl_3$) d 2.26 (dd, J = 14, 10 Hz, 1 H), 2.59 (dd, J 14, 2 Hz, 1 H), 2.64 (d, J = 6 Hz, 3 H), 2.92 (br m, 1 H), 3.12 (br, 1 H), 4.72 (m, 1 H), 4.83 (m, 1 H), 4.89 (br, 1 H), 6.23 (d, J = 7 Hz, 1 H), 6.8-7.6 (m, 23 H).

B. N-Methyl-2-(2-amino-1-hydroxy-3-phenylpropyl)-benzamide.

A mixture of 75 mg (0.14 mmol) of the resultant compound of Example 303A and 20 mg of 10% palladium on carbon in 2 ml of methanol and 0.3 ml of 1 N HCl was stirred under 1 atmosphere of hydrogen for 3 h. The resulting solution was filtered through Celite and concentrated to give the desired compound.

C. 2′-Ac-Val-Val Amide of N-Methyl-2-(2-amino-1-hydroxy-3-phenylpropyl)-benzamide.

Using the procedure of Example 66, Ac-Val-Val-OH was coupled to the resultant compound of Example 303C to give, after silica gel chromatography using 3% methanol in chloroform, the desired compound in 20% yield.

Example 304

Cbz-Leu-Asn Amide of 2-Amino-1-cyclohexyl-3,4-dihydroxy-6-methylheptane.

The resultant compound of Example 141 (1.39 mmol) was deprotected according to the procedure of Example 2 and coupled to Cbz-Leu-Asn-OH according to the procedure of Example 66 to give a crude product, which crystallized from ethyl acetate to give 204 mg (24%) of the desired compound as a white solid, m.p. 189-190°C. Mass spectrum: $(M+H)^+$ =
Anal. Calcd. for $C_{32}H_{52}N_4O_7 \cdot 0.25\ H_2O$: C, 59.14; H, 8.84; N, 8.62. Found: C, 59.15; H, 8.30; N, 8.60.

## Example 305

### (5(S)-(3-tert-Butyloxycarbonyl)-2,2-dimethyl-4(S)-cyclohexylmethyl-5(R)-oxazolidinyl)-2-thionoxazolidinon-4(S)-carbonyl)-Ile-Ser Methyl Ester.

The resultant compound from Example 253 (1.18 g, 2.13 mmol) was reacted according to the procedure of Example 213 to provide 865 mg (92%) of the desired compound. The resulting carboxylic acid (200 mg, 452 mmol) was reacted according to the procedure of Example 199, using 121 mg (450 mmol) of the resultant product of Example 196, to give 205 mg (69%) after column chromatography with ethyl acetate. $^1$H NMR (DMSO-$d_6$) 0.82 (d,3H), 0.96 (d,3H), 1.09-1.88 (several br m,15H), 3.34 (s,3H), 3.61 (br m,2H), 3.95 (br m,2H), 4.30 (br m,2H), 4.55 (d,1H), 4.66 (dd,1H), 5.20 (t,1H), 8.45 (d,1H), 8.50 (d,1H).
Anal. Cald. for $C_{31}H_{52}N_4O_4S$: C, 56.69; H, 7.98; N, 8.53.
Found: C, 56.40; H, 7.91; N, 8.18.

## Example 306

### N-Boc-Leu-Asn Amide of (5(S)-(3-tert-Butyloxycarbonyl)-2,2-dimethyl-4(S)-cyclohexylmethyl-5(R)-oxazolidinyl)-2-thionooxazolidinon-4(S)-carbonyl)-Ile-Ser Methyl Ester.

The resultant compound from Example 305 (183 mg, 0.279 mmol) was dissolved in 2 ml methanol and treated with acetyl chloride (0.119 ml, 131 mg, 1.67 mmol). The resulting solution was stirred overnight at room temperature. Sodium bicarbonate was added until gas evolution ceased, then methylene chloride was added and the mixture filtered through celite. The cloudy filtrate was evaporated to give 165 mg (114%) of a tan powder. This crude amino alcohol was reacted with Boc-Leu-Asn-OH (97 mg, 1.68 mmol) according to the procedure of Example 66. Column chromatography (5% MeOH-$CH_2Cl_2$) afforded 32mg (14%) of the desired compound. $^1$H NMR (DMSO-$d_6$) 0.85 (br m,12H), 1.05-1.80 (several br m,27H), 3.5-3.75 (several br m,6H), 4.38(br m,2H), 4.53 (m,2H), 4.63 (t,1H), 4.99 (t,1H), 5.59 (br d,1H), 6.89 (br s,1H), 6.95 (d,1H), 7.11 (d,1H), 7.32 (s,1H), 7.94 (t,2H), 8.40 (d,1H), 10.26 (s,1H). Mass spectrum: $(M+H)^+$ = 844.

## Example 307

### 3-(5(R)-(3-(tert-Butyloxycarbonyl)-2,2-dimethyl-4(S)-cyclohexylmethyl-5(R)-oxazolidinyl)-2-thionooxazolidnyl)-4(R)-carbonyl)-4(R)-phenylmethyl-2-oxazolidinone.

The procedure of D.A. Evans and A. E. Weber (*J. Am. Chem. Soc.* **1987**, 109, 7151) is adapted. A solution of the resultant compound from Example 306 (1.05 g, 3.80 mmol) in 9 ml THF was added via cannula to a -78° mixture of stannous triflate (2.0 g, 4.81 mmol) in 20 ml THF. N-Ethyl piperidine (0.826 ml, 681 mg, 6.01 mmol) was then added dropwise. After stirring at -78° for 1.5 h, a solution of the resultant

compound from Example 241 (1.30 g, 4.00 mmol) in 9 ml THF was added dropwise to the mixture over 5 minutes. Stirring was continued for 4 h at -78°, at which time the reaction mixture was warmed at 0° and quenched by the addition of 20 ml of pH 7 phosphate buffer. The slurry was filtered through Celite, and the solids were washed with 2 X 50 ml $CH_2Cl_2$. The filtrate was extracted with 4 X 100 ml $CH_2Cl_2$, the combined extracts were washed with 100 ml of 1 M aq. $NaHSO_4$, then dried over $Na_2SO_4$. The filtrate was concentrated under reduced pressure, and the residue was purified by column chromatography to afford 563 mg (23%) of the desired compound. $^1H$ NMR ($CDCl_3$) 0.95-1.75 (several br m,13H), 2.88 (m,2H), 4.14 (m,2H), 4.49 (t,1H), 5.19 (br m 1H), 7.18-7.35 (br m,5H). Mass spectrum: $(M+NH_4)^+$ = 619.

## Example 308

### 5(R)-(3-(tert-Butyloxycarbonyl)-2,2-dimethyl-4(S)-cyclohexylmethyl-5(R)-oxazolidinyl)-4(R)-carboxyl-2-thionooxazolidinone.

The procedure of D.A. Evans, J.A. Ellman, R. L. Dorow (*Tetrahedron. Lett.* **1987**, 28, 11) is adapted. A solution of the resultant compound from Example 307 (540 mg, 0.897 mmol) in 2.4 ml water and 7.3 ml THF was cooled to 0°, and a solution of lithium hydroxide monohydrate (75 mg, 79 mmol) in 7 ml water was added. The reaction was allowed to stir at 0° for 3 h. The THF was evaporated under reduced pressure, and the aqueous residue was acidified to pH 2.0 by dropwise addition of 1 M sodium bisulfate. Extraction with 20 ml ethyl acetate provided the crude product, which was purified by column chromatography to afford 174 mg (44%) of the desired compound. $^1H$ NMR (DMSO-$d_6$) 0.80-1.87 (several br m), 4.85 (t,1H), 5.1 (t,1H), 10.4 (s,1H). Mass spectrum $(M+H)^+$ = 443.

## Example 309

### (5(R)-(3-(tert-Butyloxycarbonyl)-2,2-dimethyl-4(S)-cyclohexylmethyl-5(R)-oxazolidinyl)-2-thioxazolidinon-4(R)-carbonyl)-Ile-Ser-Methyl Ester.

Using the procedure of Example 199, the resultant compound of Example 308 (174 mg, 0.393 mmol) was coupled to H-Ile-Ser-OMe (105 mg, 0.393 mmol) to provide 73 mg (28%) of the desired compound after column chromatography (2% MeOH-$CHCl_3$). $^1H$ NMR 0.80-1.8 (several br m), 3.35 (s,3H), 4.20-4.45 (several br m,2H), 5.05 (t,1H), 8.51 (br m,2H). Mass spectrum: $(M+H)^+$ = 657.

## Example 310

### N-(Boc-Leu-Asn Amide of 5(R)-(3-(tert-Butyloxycarbonyl)-2,2-dimethyl-4(S)-cyclohexylmethyl-5(R)-oxazolidinyl)-2-thioxazolidinon-4(R)-carbonyl)-Ile-Ser Methyl Ester.

The resultant compound from Example 309 (307 mg, 0.467 mmol) was dissolved in 5 ml methylene chloride, cooled to 0° under a nitrogen atmosphere, and 5 ml trifluoroacetic acid was added. The solution was stirred for 2 h at 0°, then the solvent was evaporated under reduced pressure at room temperature and the residue placed under vacuum for 0.5 h. The residue was then dissolved in 3.5 ml THF and 1.5 ml water and stirred at room temperature for 2.5 h. The solution was concentrated under reduced pressure, the resulting aqueous mixture was basified with 1 M $Na_2CO_3$, and then extracted with methylene chloride several times. The combined organic phases were washed with brine, dried over $Na_2SO_4$, and concentrated under reduced pressure to afford 190 mg (79%) of the crude aminoalcohol.

Using the procedure of Example 66, in which the above crude amino alcohol replaced the resultant compound from Example 196, the desired compound (30mg, 40%) was obtained after column chromatography on silica gel (7% $MeOH-CH_2Cl_2$). $^1H$ NMR 0.80-1.85 (several br m), 3.55-3.85(several br m), 4.0 (br m,2H), 4.36 (br m,2H), 4.5 (d,2H), 4.92 (d,1H), 7.36 (d,1H), 7.73 (d,1H), 7.89 (d,1H), 8.05 (d,1H). Mass spectrum: $(M+H)^+$ = 844.
Anal. Calcd. for $C_{38}H_{65}N_7O_{12}S$: C, 54.08; H, 7.76; N, 11.62.
Found: C, 53.55; H, 7.74; N, 11.59.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxy-ethanesulfonate, lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, tosylate, mesylate and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl., and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Other salts include salts with alkali metals or alkaline earth metals, such as sodium, potassium, calcium or magnesium or with organic bases.

The compounds of the present invention can also be used in the form of esters. Examples of such esters include a hydroxyl-substituted compound of formula I which has been acylated with a blocked or unblocked amino acid residue, a phosphate function, or a hemisuccinate residue. The amino acid esters of particular interest are glycine and lysine; however, other amino acid residues can also be used. These esters serve as pro-drugs of the compounds of the present invention and serve to increase the solubility of these substances in the gastrointestinal tract. These pro-drugs are metabolized in vivo to provide the hydroxyl-substituted compound of formula I. The preparation of the pro-drug esters is carried out by reacting a hydroxyl-substituted compound of formula I with an activated amino acyl, phosphoryl or hemisuccinyl derivative. The resulting product is then deprotected to provide the desired pro-drug ester.

The novel compounds of the present invention are inhibitors of retroviral proteases and in particular are inhibitors of HIV-1 protease and HIV-2 protease. Compounds of the invention inhibit HIV-1 protease in the range of 22-91% at concentrations of .04-1.0 mM. The novel compounds of the present invention are useful for the treatment or prophylaxis of diseases caused by retroviruses, especially acquired immune deficiency syndrome or an HIV infection.

Total daily dose administered to a host in single or divided doses may be in amounts, for example, from 0.001 to 10 mg/kg body weight daily and more usually 0.01 to 1 mg. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, by inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. The term parenteral as used herein includes subcutaneous injections, intravenous, intramuscular, intrasternal injection, or infusion techniques.

Injectable preparations, for example, sterile injectable aqueous or oleagenous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-butanediol. Among the acceptable vehicles and solvents that may be employed are water, Ringer's solution, and isotonic sodium chloride solution. In addition, sterile, fixed oils are conventionally employed as a solvent or suspending

medium. For this purpose any bland fixed oil may be employed including synthetic mono- or diglycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

While the compounds of the invention can be administered as the sole active pharmaceutical agent, they can also be used in combination with one or more immunomodulators, antiviral agents or other antiinfective agents. When administered as a combination, the therapeutic agents can be formulated as separate compositions which are given at the same time or different times, or the therapeutic agents can be given as a single composition.

The foregoing is merely illustrative of the invention and is not intended to limit the invention to the disclosed compounds. Variations and changes which are obvious to one skilled in the art are intended to be within the scope and nature of the invention which are defined in the appended claims.

## Claims

1. A compound of the formula:

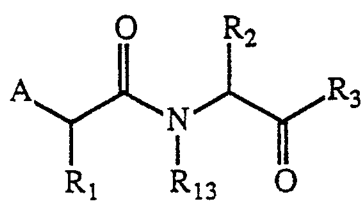

or a pharmaceutically acceptable salt, prodrug or ester thereof;
wherein:
A is
(1) hydrogen,
(2) loweralkyl,
(3) functionalized alkyl,
(4) amino,
(5) functionalized amino,
(6) functionalized carbonyl,
(7) functionalized sulfonyl or
(8) $-OR_{14}$ or $-SR_{14}$ wherein $R_{14}$ is hydrogen, loweralkyl or aminoalkyl;
$R_1$ is
(1) hydrogen,
(2) loweralkyl,
(3) hydroxyalkyl,
(4) cycloalkylalkyl,
(5) $-(CH_2)_yC(O)OH$ wherein y is 1 to 3,
(6) $-(CH_2)_yC(O)NH_2$ wherein y is 1 to 3,
(7) $-(CH_2)_yC(O)NHOH$ wherein y is 1 to 3,
(8) phenylalkyl,
(9) (substituted phenyl)alkyl wherein substituted phenyl is independently as defined above,

(10) naphthylalkyl or
(11) (substituted naphthyl)alkyl wherein substituted naphthyl is independently as defined above;
$R_2$ is
(1) loweralkyl,
(2) $-(CH_2)_zC(O)OH$ wherein z is 1 to 3,
(3) $-(CH_2)_zC(O)NH_2$ wherein z is 1 to 3,
(4) $-(CH_2)_zC(O)NHOH$ wherein z is 1 to 3,
(5) heterocyclic alkyl or
(6) (substituted heterocyclic)alkyl wherein substituted heterocyclic is independently as defined above;
$R_3$ is (substituted alkyl)amino; and
$R_{13}$ is hydrogen or loweralkyl.

2. The compound of Claim 1 wherein
$R_1$ is
(i)hydrogen,
(ii)methyl,
(iii) hydroxymethyl,
(iv) isopropyl,
(v) isobutyl,
(vi)-$(CH_2)_yCOOH$ wherein y is 1 to 3 or
(vii) $-(CH_2)_yCONH_2$ wherein y is 1 to 3; $R_2$ is
(i) methyl,
(ii) isopropyl,
(iii) isobutyl,
(iv) $-(CH_2)_zCOOH$ wherein z is 1 to 3 or
(v) $-(CH_2)_zCONH_2$ wherein z is 1 to 3;
$R_3$ is

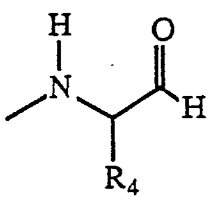

or

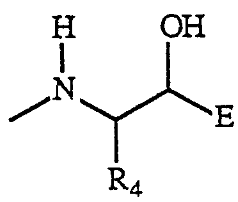

wherein $R_4$ is
(i) loweralkyl,
(ii) cycloalkyl,
(iii) cycloalkylalkyl,
(iv) (naphthyl)methyl,
(v) (substituted naphthyl)methyl wherein substituted naphthyl is independently as defined above,
(vi) benzyl or
(vii) substituted benzyl wherein the phenyl ring is substituted with a substituent selected from loweralkyl, alkoxy, thioalkoxy, hydroxy, halogen, mercapto, nitro, amino, loweralkylamino, dialkylamino, carboxaldehyde, carboxy, alkoxycarbonyl, and carboxamide; and
E is

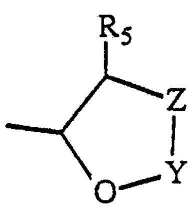

or

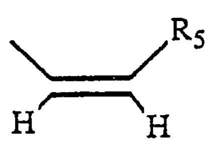

wherein $R_5$ is
(i) hydrogen,
(ii) loweralkyl,

(iii) $-C(O)R_6$ or (iv) $-C(O)-R_{75}-R_6$ wherein $R_6$ is hydroxy, alkoxy or $-NR_{15}R_{16}$ wherein $R_{15}$ and $R_{16}$ are independently as defined above and $R_{75}$ is $-NHCH(R'')C(O)-$, $-NHCH(R'')C(O)NHCH(R''')C(O)-$, $-NHCH(R'')C(O)NHCH(R''')C(O)-R_{220}-$ or $-NHCH(R'')C(O)NHCH(R''')C(O)-R_{220}-R_{221}-$wherein R″ is hydrogen, loweralkyl, cycloalkylalkyl, (phenyl)alkyl, (substituted phenyl)alkyl wherein substituted phenyl is independently as defined above, (naphthyl)alkyl or (substituted naphthyl)alkyl wherein substituted naphthyl is independently as defined above, R‴ is hydrogen, loweralkyl, hydroxyalkyl, aminoalkyl, carboxyalkyl, carboxamidoalkyl, N-hydroxycarboxamidoalkyl or guanidinoalkyl, and R220 and R221 are amino acyl residues independently selected from the twenty naturally occuring alpha amino acids;

Y is -C(O)-, -C(S)-, $-C(=CH_2)-$ or $-C(R_{11})(R_{70})-$wherein $R_{11}$ and $R_{70}$ are independently selected from hydrogen and loweralkyl; and

Z is O, $-C(=CH_2)-$, $-C(R_{11})(R_{70})-$ or $-N(R_{12})-$wherein $R_{11}$ and $R_{70}$ are independently selected from hydrogen and loweralkyl and $R_{12}$ is hydrogen, loweralkyl, alkoxy, hydroxyalkyl or alkylamino.

3. A compound of the formula:

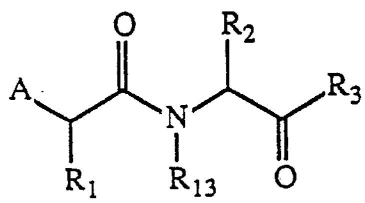

or a pharmaceutically acceptable salt, prodrug or ester thereof;
wherein:
A is
(1) hydrogen,
(2) loweralkyl,
(3) functionalized alkyl,
(4) amino,
(5) functionalized amino,
(6) functionalized carbonyl,
(7) functionalized sulfonyl or
(8) $-OR_{14}$ or $-SR_{14}$ wherein $R_{14}$ is hydrogen, loweralkyl or aminoalkyl;
$R_1$ is
(1) hydrogen,
(2) loweralkyl,
(3) hydroxyalkyl,
(4) cycloalkylalkyl,
(5) $-(CH_2)_yC(O)OH$ wherein y is 1 to 3,
(6) $-(CH_2)_yC(O)NH_2$ wherein y is 1 to 3,
(7) $-(CH_2)_yC(O)NHOH$ wherein y is 1 to 3,
(8) phenylalkyl,
(9) (substituted phenyl)alkyl wherein substituted phenyl is independently as defined above,
(10) naphthylalkyl or
(11) (substituted naphthyl)alkyl wherein substituted naphthyl is independently as defined above;
$R_2$ is
(1) loweralkyl,
(2) $-(CH_2)_zC(O)OH$ wherein z is 1 to 3,
(3) $-(CH_2)_zC(O)NH_2$ wherein z is 1 to 3,
(4) $-(CH_2)_zC(O)NHOH$ wherein z is 1 to 3,
(5) heterocyclic alkyl or
(6) (substituted heterocyclic)alkyl wherein substituted heterocyclic is independently as defined above;
$R_3$ is

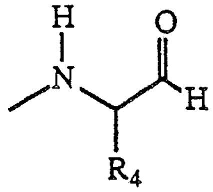

or

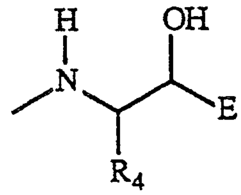

wherein $R_4$ is
(i) loweralkyl,
(ii) cycloalkyl,
(iii) cycloalkylalkyl,
(iv) (naphthyl)methyl,
(v) (substituted naphthyl)methyl wherein substituted naphthyl is independently as defined above,
(vi) benzyl or
(vii) substituted benzyl wherein the phenyl ring is substituted with a substituent selected from loweralkyl, alkoxy, thioalkoxy, hydroxy, halogen, mercapto, nitro, amino, loweralkylamino, dialkylamino, carboxaldehyde, carboxy, alkoxycarbonyl, and carboxamide; and
E is

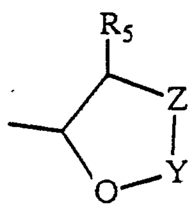

or

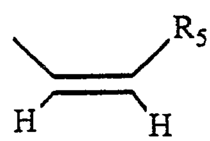

wherein $R_5$ is
(i) hydrogen,
(ii) loweralkyl,
(iii) $-C(O)R_6$ or
(iv) $-C(O)-R_{75}-R_6$ wherein $R_6$ is hydroxy, alkoxy or $-NR_{15}R_{16}$ wherein $R_{15}$ and $R_{16}$ are independently as defined above and $R_{75}$ is $-NHCH(R'')C(O)-$, $-NHCH(R'')C(O)NHCH(R''')C(O)-$, $-NHCH(R'')C(O)NHCH(R''')C(O)-R_{220}-$ or $-NHCH(R'')C(O)NHCH(R''')C(O)-R_{220}-R_{221}-$wherein $R''$ is hydrogen, loweralkyl, cycloalkylalkyl, (phenyl)alkyl, (substituted phenyl)alkyl wherein substituted phenyl is independently as defined above, (naphthyl)alkyl or (substituted naphthyl)alkyl wherein substituted naphthyl is independently as defined above, $R'''$ is hydrogen, loweralkyl, hydroxyalkyl, aminoalkyl, carboxyalkyl, carboxamidoalkyl, N-hydroxycarboxamidoalkyl or guanidinoalkyl, and R220 and R221 are amino acyl residues independently selected from the twenty naturally occuring alpha amino acids;
Y is $-C(O)-$, $-C(S)-$, $-C(=CH_2)-$ or $-C(R_{11})(R_{70})-$wherein $R_{11}$ and $R_{70}$ are independently selected from hydrogen and loweralkyl; and
Z is O, $-C(=CH_2)-$, $-C(R_{11})(R_{70})-$ or $-N(R_{12})-$wherein $R_{11}$ and $R_{70}$ are independently selected from hydrogen and loweralkyl and $R_{12}$ is hydrogen, loweralkyl, alkoxy, hydroxyalkyl or alkylamino; and
$R_{13}$ is hydrogen or loweralkyl.

4. The compound of Claim 3 wherein
$R_1$ is
(i) hydrogen,
(ii) methyl,
(iii) hydroxymethyl,
(iv) isopropyl,
(v) isobutyl,
(vi)$-(CH_2)_yCOOH$ wherein y is 1 to 3 or
(vii) $-(CH_2)_yCONH_2$ wherein y is 1 to 3; and
$R_2$ is
(i) methyl,
(ii) isopropyl,

(iii) isobutyl,
(iv) $-(CH_2)_zCOOH$ wherein z is 1 to 3 or
(v) $-(CH_2)_zCONH_2$ wherein z is 1 to 3.

5. A method for inhibiting a retroviral protease comprising administering to a host in need of such treatment a therapeutically effective amount of the compound of Claim 1.

6. The method of Claim 5 wherein the retroviral protease is HIV-1 protease or HIV-2 protease.

7. A method for treating a retroviral infection comprising administering to a host in need of such treatment a therapeutically effective amount of the compound of Claim 1.

8. The method of Claim 7 wherein the retroviral infection is caused by HIV-1 or HIV-2.

9. A pharmaceutical composition for inhibiting a retroviral protease, comprising a pharmaceutical carrier and a therapeutically effective amount of the compound Claim 1.

10. A pharmaceutical composition for treating a retroviral infection, comprising a pharmaceutical carrier and a therapeutically effective amount of the compound of Claim 1.

11. A process for the preparation of a compound of the formula:

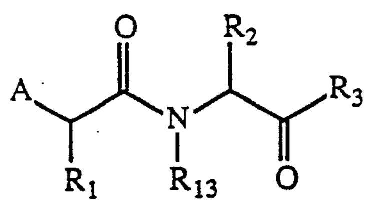

or a pharmaceutically acceptable salt, prodrug or ester thereof;
wherein:
A is
(1) hydrogen,
(2) loweralkyl,
(3) functionalized alkyl,
(4) amino,
(5) functionalized amino,
(6) functionalized carbonyl,
(7) functionalized sulfonyl or
(8) $-OR_{14}$ or $-SR_{14}$ wherein $R_{14}$ is hydrogen, loweralkyl or aminoalkyl;
$R_1$ is
(1) hydrogen,
(2) loweralkyl,
(3) hydroxyalkyl,
(4) cycloalkylalkyl,
(5) $-(CH_2)_yC(O)OH$ wherein y is 1 to 3,
(6) $-(CH_2)_yC(O)NH_2$ wherein y is 1 to 3,
(7) $-(CH_2)_yC(O)NHOH$ wherein y is 1 to 3,
(8) phenylalkyl,
(9) (substituted phenyl)alkyl wherein substituted phenyl is independently as defined above,
(10) naphthylalkyl or
(11) (substituted naphthyl)alkyl wherein substituted naphthyl is independently as defined above;
$R_2$ is
(1) loweralkyl,
(2) $-(CH_2)_zC(O)OH$ wherein z is 1 to 3,
(3) $-(CH_2)_zC(O)NH_2$ wherein z is 1 to 3,
(4) $-(CH_2)_zC(O)NHOH$ wherein z is 1 to 3,
(5) heterocyclic alkyl or
(6) (substituted heterocyclic)alkyl wherein substituted heterocyclic is independently as defined above;
$R_3$ is (substituted alkyl)amino; and
$R_{13}$ is hydrogen or loweralkyl,
comprising
(a) coupling a carboxylic acid of the formula

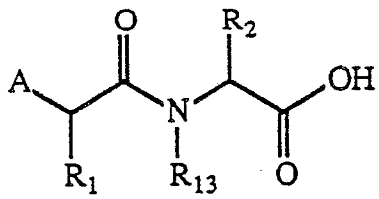

or an acid halide or activated ester derivative thereof wherein A, $R_1$, $R_2$ and $R_{13}$ are as defined herein with a compound of the formula

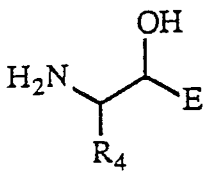 , 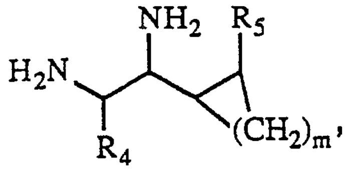 , 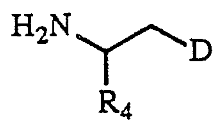 or 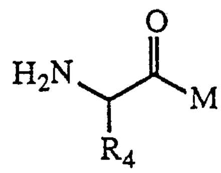

wherein m, $R_4$, $R_5$, D, E and M are as defined herein; or

(b) coupling an N-protected amino acid of the formula

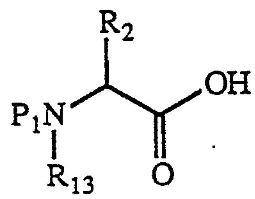

or an acid halide or activated ester derivative thereof wherein $P_1$ is an N-protecting group and $R_2$ and $R_{13}$ are as defined herein with a compound of the formula

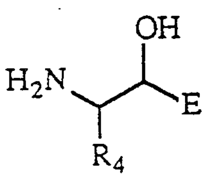 , 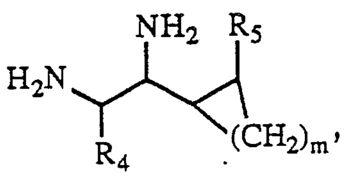 , 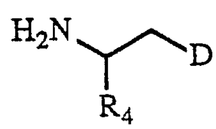 or 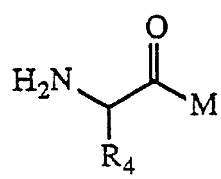

wherein m, $R_4$, $R_5$, D, E and M are as defined herein,

followed by removal of the N-protecting group and coupling with a carboxylic acid of the formula

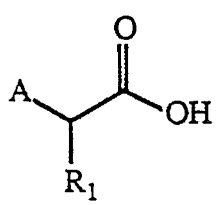

or an acid halide or activated ester derivative thereof wherein A and $R_1$ are as defined herein.